(19) Europäisches Patentamt
European Patent Office
Office européen des brevets

(11) Publication number : **0 530 994 A1**

# (12) EUROPEAN PATENT APPLICATION

(21) Application number : **92307398.5**

(22) Date of filing : **12.08.92**

(51) Int. Cl.$^5$ : **C07D 239/80,** C07D 239/82, C07D 487/04, C07D 498/04, C07D 405/04, A61K 31/505

(30) Priority : **16.08.91 US 746445**
**07.05.92 US 880121**

(43) Date of publication of application :
**10.03.93 Bulletin 93/10**

(84) Designated Contracting States :
**AT BE CH DE DK ES FR GB GR IE IT LI LU NL PT SE**

(71) Applicant : **MERCK & CO. INC.**
**126, East Lincoln Avenue P.O. Box 2000**
**Rahway New Jersey 07065-0900 (US)**

(72) Inventor : **Britcher, Susan F.**
**735 Port Indian Road**
**Norristown, PA 19403 (US)**
Inventor : **Lumma, William C., Jr.**
**25 Newman Road**
**Pennsburg, PA 18073 (US)**
Inventor : **Goldman, Mark E.**
**1510 Bridal Path Road**
**Lansdale, PA 19446 (US)**
Inventor : **Lyle, Terry A.**
**570 Camp WaWa Road**
**Lederach, PA 19450 (US)**
Inventor : **Huff, Joel R.**
**825 Surrey Drive**
**Gwynedd Valley, PA 19437 (US)**
Inventor : **Payne, Linda S.**
**1502 Henning Way**
**Lansdale, PA 19446 (US)**
Inventor : **Quesada, Martha L.**
**248 W. Dudley Avenue**
**Westfield, NJ 07090 (US)**
Inventor : **Young, Steven D.**
**1280 Mark Drive**
**Lansdale, PA 19446 (US)**
Inventor : **Sanders, William M.**
**1262 Skippack Pike**
**Center Square, PA 19422 (US)**
Inventor : **Sanderson, Philip E.**
**129 West Gravers Lane**
**Philadelphia, PA 19118 (US)**
Inventor : **Tucker, Thomas J.**
**114 Station Drive**
**North Wales, PA 19454 (US)**

(74) Representative : **Barrett-Major, Julie Diane et al**
**Merck & Co., Inc. European Patent Department Terlings Park Eastwick Road**
**Harlow Essex CM20 2QR (GB)**

## (54) Quinazoline derivatives as inhibitors of HIV reverse transcriptase.

(57) Compounds having a quinazolin-2-(thi)one nucleus are described. These compounds are useful in the inhibition of HIV reverse transcriptase, the prevention or treatment of infection by HIV and the treatment of AIDS, either as compounds, pharmaceutically acceptable salts, pharmaceutical composition ingredients, whether or not in combination with other antivirals, immunomodulators, antibiotics or vaccines. Methods of treating AIDS and methods of preventing or treating infection by HIV are also described.

EP 0 530 994 A1

## BACKGROUND OF THE INVENTION

A retrovirus designated human immunodeficiency virus (HIV) is the etiological agent of the complex disease that includes progressive destruction of the immune system (acquired immune deficiency syndrome; AIDS) and degeneration of the central and peripheral nervous system. This virus was previously known as LAV, HTLV-III, or ARV. A common feature of retrovirus replication is reverse transcription of the RNA genome by a virally encoded reverse transcriptase to generate DNA copies of HIV sequences, a required step in viral replication. It is known that some compounds are reverse transcriptase inhibitors and are effective agents in the treatment of AIDS and similar diseases, e.g., azidothymidine or AZT.

Nucleotide sequencing of HIV shows the presence of a pol gene in one open reading frame [Ratner, L. et al., Nature, 313, 277(1985)]. Amino acid sequence homology provides evidence that the pol sequence encodes reverse transcriptase, an endonuclease and an HIV protease [Toh, H. et al., EMBO J. 4, 1267 (1985); Power, M.D. et al., Science, 231, 1567 (1986); pearl, L.H. et al., Nature 329, 351(1987)].

Applicants demonstrate that the compounds of this invention are inhibitors of HIV reverse transcriptase.

## BRIEF DESCRIPTION OF THE INVENTION

Compounds of formula I or II, as herein defined, are disclosed. These compounds are useful in the inhibition of HIV reverse transcriptase, the prevention of infection by HIV, the treatment of infection by HIV and in the treatment of AIDS and/or ARC, either as compounds, pharmaceutically acceptable salts (when appropriate), pharmaceutical composition ingredients, whether or not in combination with other antivirals, anti-infectives, immunomodulators, antibiotics or vaccines. Methods of treating AIDS, methods of preventing infection by HIV, and methods of treating infection by HIV are also disclosed.

## DETAILED DESCRIPTION OF THE INVENTION AND PREFERRED EMBODIMENTS

This invention is concerned with compounds of formula I or II, combinations thereof, or pharmaceutically acceptable salts thereof, in the inhibition of HIV reverse transcriptase, the prevention or treatment of infection by HIV and in the treatment of the resulting acquired immune deficiency syndrome (AIDS). Compounds of formula I or II are defined as follows:

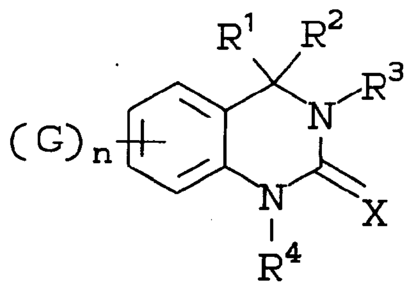

I

or

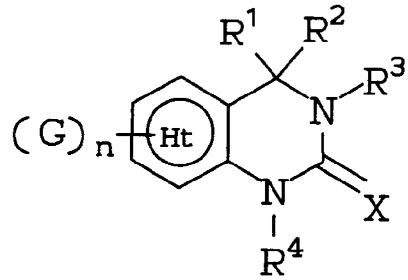

II

wherein:

X is O, S, or NR, and R is CN, H, $NH_2$, OH, $C_{1-3}$alkoxy, or $C_{1-3}$ alkyl;

G, when present, is 1-4 substituents independently selected from $C_{1-4}$ alkyl; halo; amino; nitro; cyano; carboxy; carbamoyl $C_{1-4}$ alkyl;

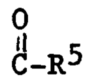

wherein $R^5$ is $CH_3$, H or $NH_2$; sulfamoyl; $C_{1-3}$ alkylsulfonamido; methanesulfonyl; $CF_3$; hydroxy; or $C_{1-4}$alkoxy;

n is 0-4;

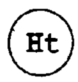 informula II denotes heterocycle of 5-6 members (with 1-3 heteroatoms selected from O, N or S) in place of benzo in formula I;

$R^1$ is H; $C_{1-3}$ alkyl; $C_{2-4}$ alkenyl; $C_{2-5}$ alkynyl; $C_{3-4}$ cycloalkyl; hydroxy; $C_{1-4}$ alkoxy; $C_{1-3}$ alkyl substituted one or more times with halo, carboxy, $C_{3-4}$ cycloalkyl, CN, hydroxy, methylsulfinyl, methanesulfonyl, $C_{1-4}$ alkoxy, $C_{2-4}$ alkenyl $C_{1-4}$alkoxy, $C_{2-4}$ alkynl-$C_{1-4}$ alkoxy, aryloxy, $C_{1-4}$ alkylcarbonyl, or nitro; or aryl unsubstituted or substituted with $(G)_n$;furanyl; thienyl; benzofuranyl; $R^1$ and $R^3$ may be joined to form a fused heterocycle of 5 or 6 members containing up to one additional heteroatom selected from 0, S or NR, provided that the heteroatoms are not directly linked to each other;

$R^2$ is $C_{1-8}$ alkyl; $C_{2-8}$ alkenyl; $C_{3-4}$ cycloalkyl; $C_{2-5}$ alkynyl; aryl unsubstituted or substituted at the meta position with halo, methoxy or cyano; heterocycle;

$R^3$ is H; $C_{1-8}$ alkyl unsubstituted or substituted with one or more of A, wherein A is halo, hydroxy, amino, $C_{3-8}$ cycloalkyl, $C_{1-4}$ alkoxy, di-($C_{1-4}$ alkyl)amino, $C_{1-4}$ alkylamino, or carbamoyl; $C_{2-3}$ alkenyl unsubstituted or substituted with one or more of A; $C_{2-8}$ alkynyl unsubstituted or substituted with one or more of A; $C_{3-8}$ cycloalkyl; aryl $C_{1-4}$ alkyl wherein aryl is optionally substituted with up to three substituents selected from methyl, halo, CN, or methoxy; heterocycle of 5 or 6 members containing up to 3 heteroatoms selected from O, S or NR; hydroxyl; CN; or carbamoyl;

$R^4$ is H; $C_{1-3}$ alkyl unsubstituted or substituted with one or more of A; $C_{2-3}$ alkenyl unsubstituted or substituted with one or more of A; $C_{2-8}$ alkynyl unsubstituted or substituted with one or more of A; $C_{1-3}$ alkanoyl; benzoyl unsubstituted or substituted with one to three of halo, methoxy, carboxy, or $C_{1-4}$ alkyl optionally substituted with carboxy, amino, $C_{1-4}$ alkylamino or di-$C_{1-4}$ alkylamino; carbamoyl; methylcarbamoyl; dimethylcarbamoyl; formyl; hydroxy; alpha-amino $C_{1-4}$ alkanoyl; or meta-cyanobenzyl;

or pharmaceutically acceptable salt or ester thereof.

In one embodiment, the compounds of formula I or II are further limited to those wherein X is NR and R is CN, H, $NH_2$, OH, $C_{1-3}$ alkoxy or $C_{1-3}$ alkyl.

In another embodiment, compounds are limited to formula III:

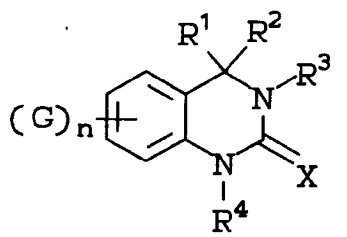 III

wherein:

X is O, S, or NR, and R is CN, $NH_2$, OH, $C_{1-3}$ alkoxy, H or $C_{1-3}$ alkyl;

G, when present, is 1-4 substituents independently selected from $C_{1-4}$ alkyl; halo; amino; nitro; cyano; carboxy; carbamoyl $C_{1-4}$ alkyl;

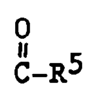

wherein $R^5$ is $CH_3$, H or $NH_2$; sulfamoyl; $C_{1-3}$ alkylsulfonamido; methanesulfonyl; $CF_3$; hydroxy; or $C_{1-4}$alkoxy;

n is 0-4;

$R^1$ is $C_{1-8}$ alkyl; $C_{2-4}$ alkenyl; $C_{2-5}$ alkynyl; $C_{3-4}$ cycloalkyl; $C_{1-3}$ alkyl substituted one or more times with halo, carboxy, $C_{3-4}$ cycloalkyl, CN, hydroxy, methylsulfinyl, methanesulfonyl, $C_{1-4}$ alkoxy, $C_{2-4}$ alkynyl-$C_{1-4}$ alkoxy, or nitro; or aryl unsubstituted or substituted with $(G)_n$; $R^1$ and $R^3$ may be joined by a carba bridge to form a ring of 5 to 6 members;

$R^2$ is $C_{1-8}$ alkyl; $C_{2-8}$ alkenyl; $C_{3-4}$ cycloalkyl; $C_{2-5}$ alkynyl; aryl unsubstituted or substituted at the meta position with halo, methoxy or cyano; heterocycle of 5 or 6 members containing up to 3 heteroatoms selected from O, S, or NR;

$R^3$ is H; $C_{1-8}$ alkyl unsubstituted or substituted with one or more of A, wherein A is halo, hydroxy, amino, $C_{3-8}$ cycloalkyl, $C_{1-3}$ alkoxy, di-($C_{1-4}$ alkyl)amino, $C_{1-4}$ alkylamino, or carbamoyl; $C_{2-8}$ alkenyl unsubstituted or substituted with one or more of A; $C_{2-8}$ alkynyl unsubstituted or substituted with one or more of A; $C_{3-8}$ cycloalkyl; aryl $C_{1-4}$ alkyl wherein aryl is optionally substituted with up to three substituents selected from methyl, halo, CN, or methoxy;

$R^4$ is H; $C_{1-8}$ alkyl unsubstituted or substituted with one or more of A; $C_{2-8}$ alkenyl unsubstituted or substituted with one or more of A; $C_{2-8}$ alkynyl unsubstituted or substituted with one or more of A; $C_{1-3}$ alkanoyl; benzoyl unsubstituted or substituted with one to three of halo, methoxy, carboxy, or $C_{1-4}$ alkyl optionally substituted with carboxy, amino, $C_{1-4}$ alkylamino or di-$C_{1-4}$ alkylamino; carbamoyl; methylcarbamoyl; dimethylcarbamoyl; formyl; hydroxy; alpha-amino $C_{1-4}$ alkanoyl; or meta-cyanobenzyl;

with the proviso that when $R^1$ is $C_{1-2}$ alkyl, then $R^2$ is not $C_{1-2}$ alkyl;

or pharmaceutically acceptable salt or ester thereof.

A third embodiment encompasses compounds of Formula IV having a more limited scope in $R^2$:

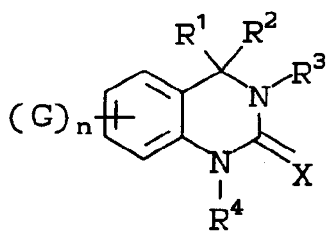

IV

wherein:

X is O, S, or NR, and R is CN, $NH_2$, OH, $C_{1-3}$ alkoxy, H or $C_{1-3}$ alkyl;

G, when present, is 1-4 substituents independently selected from $C_{1-4}$ alkyl; halo; amino; nitro; cyano; carboxy; carbamoyl $C_{1-4}$ alkyl;

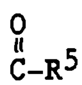

wherein $R^5$ is $CH_3$, H or $NH_2$; sulfamoyl; $C_{1-3}$ alkylsulfonamido; methanesulfonyl; $CF_3$; hydroxy; or $C_{1-4}$alkoxy;

n is 0-4;

$R^1$ is $C_{1-8}$ alkyl; $C_{2-4}$ alkenyl; $C_{2-5}$ alkynyl; $C_{3-4}$ cycloalkyl; $C_{1-3}$ alkyl substituted one or more times with halo, carboxy, $C_{3-4}$ cycloalkyl, CN, hydroxy, methylsulfinyl, methanesulfonyl, $C_{1-4}$ alkoxy, $C_{2-4}$ alkynyl-$C_{1-4}$ alkoxy, or nitro; or aryl unsubstituted or substituted with $(G)_n$; $R^1$ and $R^3$ may be joined by a carba bridge to form a ring of 5 to 6 members;

$R^2$ is $C_{1-8}$ alkyl; $C_{2-8}$ alkenyl; $C_{3-4}$ cycloalkyl; $C_{2-5}$ alkynyl; aryl unsubstituted or substituted at the meta position with halo, methoxy or cyano;

$R^3$ is H; $C_{1-8}$ alkyl unsubstituted or substituted with one or more of A, wherein A is halo, hydroxy, amino, $C_{3-8}$ cycloalkyl, $C_{1-3}$ alkoxy, di-($C_{1-4}$ alkyl)amino, $C_{1-4}$ alkylamino, or carbamoyl; $C_{2-8}$ alkenyl unsubstituted or substituted with one or more of A; $C_{2-8}$ alkynyl unsubstituted or substituted with one or more of A; $C_{3-8}$ cycloalkyl; aryl $C_{1-4}$ alkyl wherein aryl is optionally substituted with up to three substituents selected from methyl, halo, CN, or methoxy;

$R^4$ is H; $C_{1-8}$ alkyl unsubstituted or substituted with one or more of A; $C_{2-8}$ alkenyl unsubstituted or substituted with one or more of A; $C_{2-8}$ alkynyl unsubstituted or substituted with one or more of A; $C_{1-3}$ alkanoyl;

benzoyl unsubstituted or substituted with one to three of halo, methoxy, carboxy, or $C_{1-4}$ alkyl optionally substituted with carboxy, amino, $C_{1-4}$ alkylamino or di-$C_{1-4}$ alkylamino; carbamoyl; methylcarbamoyl; dimethylcarbamoyl; formyl; hydroxy; alpha-amino $C_{1-4}$ alkanoyl; or meta-cyanobenzyl;
with the proviso that when $R^1$ is $C_{1-2}$ alkyl, then $R^2$ is not $C_{1-2}$ alkyl;
or pharmaceutically acceptable salt or ester thereof.

In a fourth embodiment, compounds are further limited to formula V:

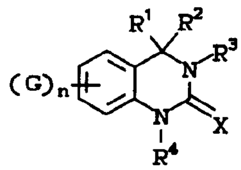

V

wherein

X is 0;

G, when present, is $C_{1-4}$alkyl; halo; amino; nitro; cyano; carboxy; carbamoyl$C_{1-4}$alkyl; or $C_{1-3}$alkylsulfonamido;

n is 0-4;

$R^1$ is $C_{3-4}$alkyl; $C_{3-4}$alkenyl; $C_{2-5}$ alkynyl; $C_{3-5}$ cycloalkyl; $C_{1-3}$ alkyl substituted with $C_{3-4}$ cycloalkyl, $C_{1-4}$ alkoxy, or $C_{2-4}$ alkynyl-$C_{1-4}$ alkoxy;

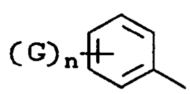 or 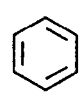 ;

$R^1$ and $R^3$ may be joined by a carba bridge to form a ring of 5 to 6 members;

$R^2$ is $C_{1-3}$alkyl $C_{2-3}$alkenyl, $C_{2-3}$alkynyl, $C_{3-4}$cycloalkyl, or aryl;

$R^3$ is H; $C_{1-4}$alkyl unsubstituted or substituted with $C_{3-3}$ cycloalkyl, $C_{2-4}$ alkenyl, or $C_{2-4}$ alkynyl; $C_{3-4}$cycloalkyl;

$R^4$ is H; $C_{1-3}$ alkyl unsubstituted or substituted with one or more of A; $C_{2-3}$ alkenyl unsubstituted or substituted with one or more of A; $C_{2-8}$ alkynyl unsubstituted or substituted with one or more of A; $C_{1-3}$ alkanoyl; benzoyl unsubstituted or substituted with one to three of halo, methoxy, carboxy, or $C_{1-4}$ alkyl optionally substituted with carboxy, amino $C_{1-4}$ alkylamino or di-$C_{1-4}$ alkylamino; carbamoyl; methylcarbamoyl; dimethylcarbamoyl; formyl; hydroxy; alpha-amino $C_{1-4}$ alkanoyl; or meta-cyanobenzyl;

with the proviso that $R^1$ and $R^2$ are not both straight-chain alkyl groups;
or pharmaceutically acceptable salts or esters thereof.

In another embodiment of Formula I, X is S, G is 6-chloro, $R^1$ is phenyl, $R^2$ is ethyl, $R^3$ is methyl, and $R^4$ is H.

In another embodiment of Formula I, X is S or O, G is 6-chloro, $R^1$ is phenyl, $R^2$ is ethynyl, $R^3$ is methyl, and $R^4$ is methyl, including each isomeric form.

In another embodiment of Formula I, X is S or O, G is 6-chloro, $R^1$ is phenyl, $R^2$ is ethynyl, $R^3$ is cyclopropyl, and $R^4$ is H.

In another embodiment of Formula I, X is S or O, G is 6-chloro, $R^1$ is phenyl, $R^2$ is cyclopropyl, $R^3$ is ethynyl, and $R^4$ is H.

In another embodiment of Formula I, X is O, G is 6-chloro, $R^1$ is propyl, $R^2$ is cyclopropyl, $R^3$ is cyclopropylmethyl, and $R^4$ is H.

In another embodiment of Formula I, X is O, G is 6-chloro, $R^1$ is phenyl, $R^2$ is ethynyl, $R^3$ is ethyl, and $R^4$ is H.

In another embodiment of Formula I, X is O, G is 6-chloro, $R^1$ is phenyl, $R^2$ is ethynyl, $R^3$ is isopropyl, and $R^4$ is H.

In another embodiment of Formula I, X is O, G is 6-chloro, $R^1$ is propyl, $R^2$ is cyclopropyl, $R^3$ is methyl and $R^4$ is H.

Compounds of this invention and their pharmaceutically acceptable salts include, but are not limited to, the list in the following Tables.

## TABLE I

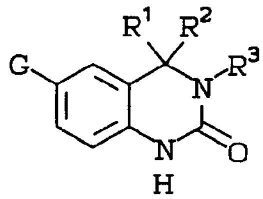

| Compound | G | $R^1$ | $R^2$ | $R^3$ | Ex | $IC_{50}$($\mu$M, rC-dG) |
|---|---|---|---|---|---|---|
| 1 | Cl | n-propyl | 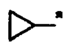 | $-CH_3$ | 13 | 0.0082 |
| 2 | Cl | n-propyl | 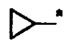 | 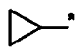 | | 0.015 |
| 3 | Cl | Ph | 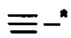 | $-CH_2CH_3$ | 12 | 0.032 |
| 4 | Cl | Ph | 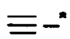 | $-CH_3$ | 10 | 0.034 |
| 5 | Cl | Ph | 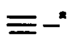 | $-CH(CH_3)_2$ | 11 | 0.061 |
| 6 | Cl | Ph | 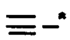 | 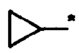 | | 0.060 |
| 7 | Cl | Ph | $-CH_2CH_3$ | $-CH(CH_3)_2$ | | 0.215 |

*designates point of attachment

## TABLE I. Cont'd.

| Compound | G | $R^1$ | $R^2$ | $R^3$ | Ex | $IC_{50}$(μM, rC-dG) |
|---|---|---|---|---|---|---|
| 8 | Cl | Ph | ≡-* | 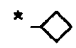 | | 0.068 |
| 9 | -CN | Ph | $-CH_2CH_3$ | $-CH_3$ | | 0.067 |
| 10 | Cl | $-CH_2CH_3$ | $-CH_2CH_3$ | $-CH_3$ | | 1.60 |
| 11 | Cl | ≡-* | $-CH_2OCH_2CH_3$ | H | 66 | 0.12 |
| 12 | Cl | ≡-* | $-(CH_2O)_2CH_2CH_2OCH_3$ | H | 67 | 3.5 |
| 13 | Cl | ≡-* | $-CH_2OH$ | H | 64 | 36.5 |
| 14 | Cl | ≡-* | $-CH(CH_3)_2$ | H | 62 | 0.26 |
| 15 | Cl | ≡-* | 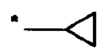 | H | 60 | 1.0 |
| 16 | Cl | ≡-* | n-propyl | H | 61 | 0.51 |
| 17 | Cl | ≡-* | 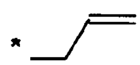 | $-CH_3$ | 59 | 0.28 |

## TABLE I Cont'd.

| Compound | G | $R^1$ | $R^2$ | $R^3$ | Ex | $IC_{50}$(μM, rC-dG) |
|---|---|---|---|---|---|---|
| 18 | Cl | 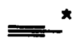 | 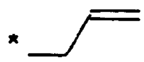 | H | 58 | 1.1 |
| 19 | " | 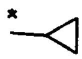 | 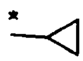 | $-CH_3$ | 27 | 0.74 |
| 20 | " | " | 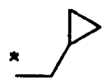 | -CN | 26 | 0.065 |
| 21 | " | " | $-CH(CH_3)_2$ | H | 34 | 9.1 |
| 22 | " | " | 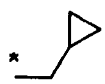 | 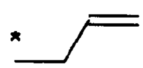 | 28 | 0.31 |
| 23 | " | " | 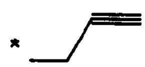 | H | 29 | 0.50 |
| 24 | " | " | 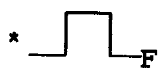 | H | 25 | 0.60 |

## TABLE I Cont'd.

| Compound | G | $R^1$ | $R^2$ | $R^3$ | Ex | $IC_{50}$(μM, rC-dG) |
|---|---|---|---|---|---|---|
| 25 | -Cl | 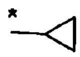 | 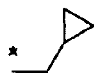 | 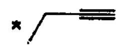 | 36 | 0.032<br>$CIC_{95}$=0.10μM |
| 26 | " | 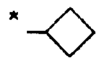 | 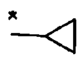 | H | 44 | 1.55 |
| 27 | " | " | " | $CH_3$ | 45 | 0.83 |
| 28 | " | 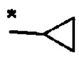 | n-propyl | ethyl | 37 | 0.014<br>$CIC_{95}$=0.025μM |
| 29 | " | " | 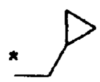 | H | 24 | 0.21<br>$CIC_{95}$=0.8 |
| 30 | " | " | $-CH_2OCH_2CH_3$ | ethyl | 51 | 0.035<br>$CIC_{95}$=0.009 |
| 31 | " | " | $-(CH_2)_2OCH_2CH_3$ | ethyl | 23 | 0.067 |

## TABLE I Cont'd.

| Compound | G | $R^1$ | $R^2$ | $R^3$ | Ex | $IC_{50}$(μM, rC-dG) |
|---|---|---|---|---|---|---|
| 32 | Cl | 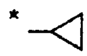 | $-CH_2OCH_2CH_3$ | H | 52 | 0.052 |
| 33 | " | " | $-CH_2OCH_3$ | $-CH_3$ | 53 | 0.072 |
| 34 | " | " | $-(CH_2)_2OCH_3$ | $-CH_3$ | 22 | 0.038<br>$CIC_{95}$=0.1 |
| 35 | " | " | $-(CH_2)_2OH$ | $-CH_3$ | 21 | 150 |
| 36 | " | " | $-(CH_2)_3OH$ | $-CH_3$ | 20 | 2.35 |
| 37 | " | " | -[(2S)-hydroxy)] propyl | " | 32 | 2.3 |
| 38 | " | " | -[(2R)-hydroxy)] propyl | " | 33 | 21.0 |

TABLE I Cont'd.

| Compound | G | $R^1$ | $R^2$ | $R^3$ | Ex | $IC_{50}$(μM, rC-dG) |
|---|---|---|---|---|---|---|
| 39 | -Cl | 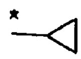 | t-butylmethyl | $CH_3$ | 19 | 3.3 |
| 40 | " | " | $-CH_2CH(CH_3)_2$ | " | 38 | 0.025 |
| 41 | " | " | $-CH_2C(O)CH_3$ | " | 31 | 11.5 |
| 42 | " | " | 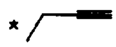 | " | 30 | 0.275 |
| 43 | " | " | n-butyl | H | 39 | 0.014<br>$CIC_{95}$=0.1 |
| 44 | " | " | 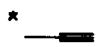 | 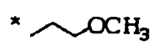 | 46 | 0.57 |
| 45 | " | " | " | 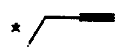 | 47 | 0.096 |

## TABLE I Cont'd.

| Compound | G | $R^1$ | $R^2$ | $R^3$ | Ex | $IC_{50}$(μM, rC-dG) |
|---|---|---|---|---|---|---|
| 46 | -Cl | 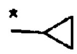 | 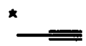 | $-CH_3$ | 48 | 0. 034<br>$CIC_{95}$=0. 1 |
| 47 | " | " | " | H | 49 | 0. 56 |
| 48 | " | i-propyl | 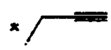 | H | 76 | 1. 0 |
| 49 | " | " | n-propyl | H | 77 | 0. 21 |
| 50 | H<br>(+)isomer | " | " | $-CH_3$ | 78 | 0. 30 |
| 51 | Cl | 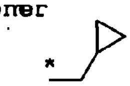 | 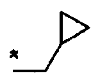 | H | 72 | 4. 9 |
| 52 | Cl | 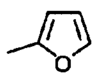 | 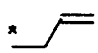 | $CH_3$ | 57 | 0. 29 |

## TABLE I Cont'd.

| Compound | G | $R^1$ | $R^2$ | $R^3$ | Ex | $IC_{50}$(μM, $rC_dG$) |
|---|---|---|---|---|---|---|
| 53 | -Cl | 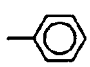 | 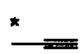 | $-CH_2OH$ | 93 | 0. 375 |
| 54 | " | 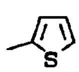 | 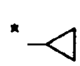 | $-CH_3$ | 18 | 0. 275 |
| 55 | " | 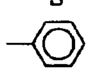 | 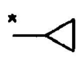 | $-CH_3$ | 100 | 0. 34 |
| 56 | F | " | 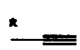 | $-CH_3$ | 96 | 0. 12 |
| 57 | Cl<br>(+)isomer | 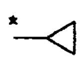 | n-propyl | $-CH_3$ | 40 | 0. 117 |
| 58 | Cl<br>(+/-) | " | 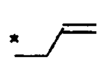 | " | 41 | 0. 125 |
| 59 | Cl<br>(+/-) | " | n-propyl | H | 42 | 0. 081 |

TABLE I Cont'd.

| Compound | G | $R^1$ | $R^2$ | $R^3$ | Ex | $IC_{50}$(μM, rC-dG) |
|---|---|---|---|---|---|---|
| 60 | Cl (+) | 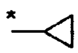 | n-propyl | H | 43 | 0.046 |
| 61 | Cl (+/-) | " | 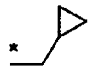 | $-CH_3$ | 15 | 0.013 |
| 62 | Cl (+/-) | " | 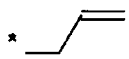 | " | 14 | 0.016 |
| 63 | H | 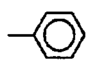 | 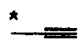 | " | 97 | 0.54 |
| 64 | Br | " | 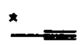 | $CH_3$ | 98 | 0.048 |
| 65 | Cl | 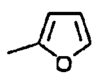 | 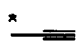 | " | 99 | 0.54 |
| 66 | " | 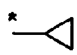 | 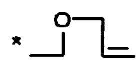 | " | 50 | 0.013 |

## TABLE I Cont'd.

| Compound | G | $R^1$ | $R^2$ | $R^3$ | Ex | $IC_{50}$($\mu$M, rC-dG) |
|---|---|---|---|---|---|---|
| 67 | Cl | 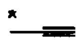 | 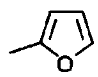 | H | 70 | 0.14 |
| 68 | " | " | t-butoxy-methyl | " | 69 | 0.725 |
| 69 | " | " | 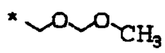 | " | 68 | 0.072 |
| 70 | " | 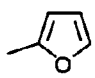 | 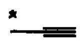 | $-CH_3$ | 71 | 0.059 |
| 71 | " | 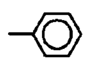 | " | H | 95 | 0.43 |
| 72 | " | 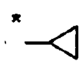 | 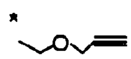 | 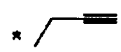 | 54 | 0.019 |
| 73 | " | " | 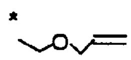 | 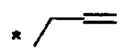 | 55 | 0.016 |

## TABLE I Cont'd.

| Compound | G | $R^1$ | $R^2$ | $R^3$ | Ex | $IC_{50}$($\mu$M, rC-dG) |
|---|---|---|---|---|---|---|
| 74 | Cl | 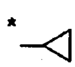 | 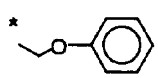 | H | 56 | 0.025 |
| 75 | " | " | H | 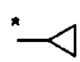 | 103 | 0.17 |

## TABLE II

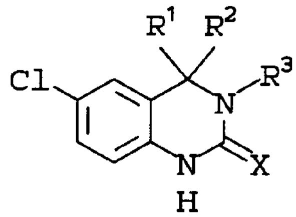

| Compound | $R^1$ | $R^2$ | $R^3$ | X | Ex | $IC_{50}$($\mu$M, rC-dG) |
|---|---|---|---|---|---|---|
| 76 | 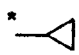 | 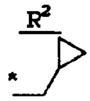 | $-CH_3$ | $NOCH_3$ | 88 | 0.027<br>$CIC_{95}$=0.05$\mu$M |
| 77 | · | 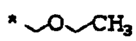 | $-CH_2CH_3$ | NCN | 79 | 0.25 |
| 78 | 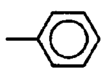 | 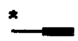 | $-CH_3$ | $NOCH_3$ | 82 | 0.104 |
| 79 | 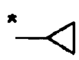 | 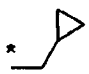 | $-CH_3$ | NCN | 89 | 1.15 |
| 80 | 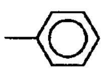 | 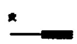 | · | NH | 83 | 7.2 |

## TABLE II Cont'd.

| Compound | $R^1$ | $R^2$ | $R^3$ | X | Ex | $Ic_{50}$($\mu$M, rC-dG) |
|---|---|---|---|---|---|---|
| 81 | 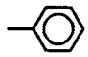 | 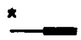 | $-CH_3$ | NCN | 84 | 0.033<br>$CIC_{95}$=0.1 |
| 82 | " | $-CH_2CH_3$ | " | $NCH_3$ | 85 | 1.4 |
| 83 | " | " | " | $NNH_2$•HCl | 86 | 0.54 |
| 84 | " | " | " | NH | 87 | 24. |
| 85 | 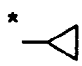 | 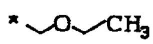 | $-CH_2CH_3$ | NOH | 81 | 0.040 |
| 86 | " | " | " | $NOHCH_3$•2HCl | 80 | 0.064 |

TABLE III

| Compound | structure | Ex | $IC_{50}$ |
|---|---|---|---|
| 87 | 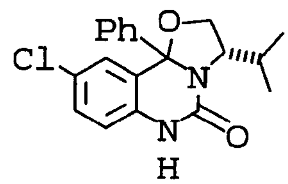 | | 1.35$\mu$M |
| 88 | 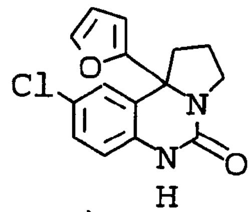 | 73 | 1.9 |
| 89 | 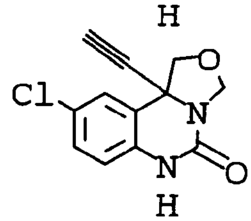 | 65 | 1.85 |
| 90 | 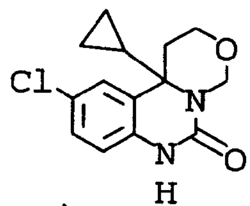 | 17 | 3.4 |
| 91 | 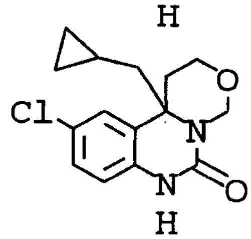 | 74 | 10.5 |

## TABLE III Cont'd.

| Compound | structure | Ex | $IC_{50}$ |
|---|---|---|---|
| 92 | 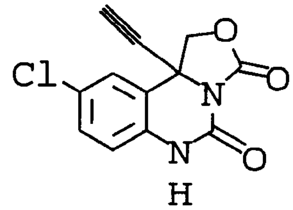 | 63 | 1.5 |
| 93 | 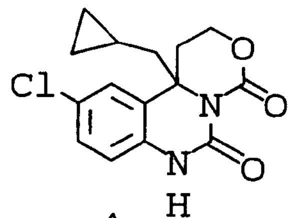 | 75 | >10 |
| 94 | 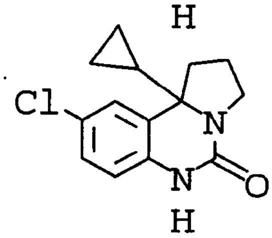 | 16 | 0.275 |
| 95 | 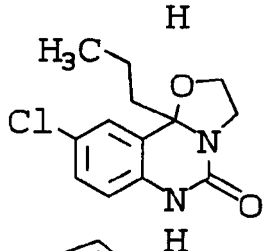 | 101 | 1.63 |
| 96 | 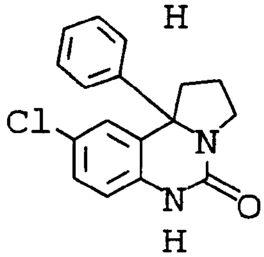 | 102 | 0.107 |

Most preferred compounds include the following:

A.

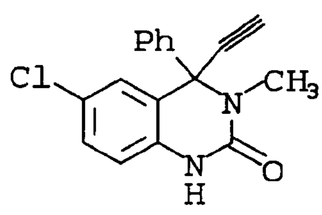

6-Chloro-3,4-dihydro-3-methyl-4-phenyl-4-ethynyl-2(1H)-quinazolinone; of which the (+) enantiomer is most

active;

B.

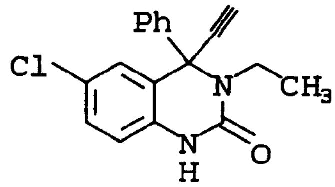

6-Chloro-3,4-dihydro-3-ethyl-4-phenyl-4-ethynyl-2(1H)-quinazolinone;

C.

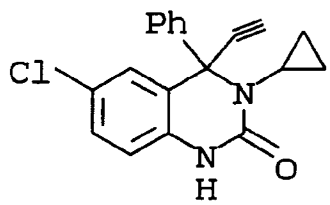

6-Chloro-3,4-dihydro-3-cyclopropyl-4-phenyl-4-ethynyl-2(1H)-quinazolinone; and

D.

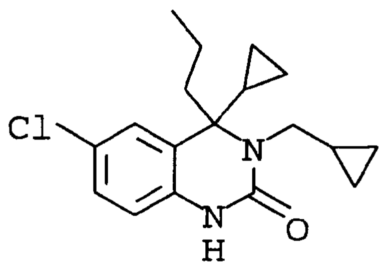

6-Chloro-3,4-dihydro-3-cyclopropylmethyl-4-cyclopropyl-4-propyl-2(1H)-quinazolinone.

E.

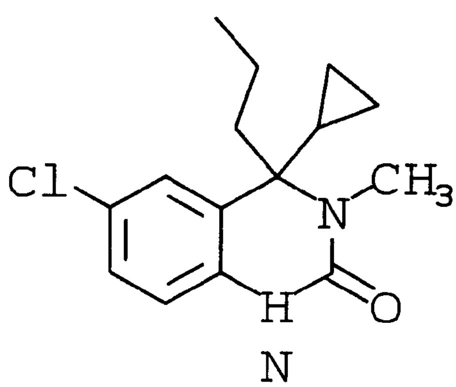

6-Chloro-3,4-dihydro-3-methyl-4-cyclopropyl-4-propyl-2(1H)-quinazolinone.

F.

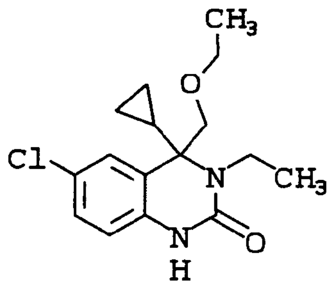

6-Chloro-3,4-dihydro-3-ethyl-4-cyclopropyl-4-(2-ethoxymethyl)-2(1H)-quinazolinone, (Example 51).

G.

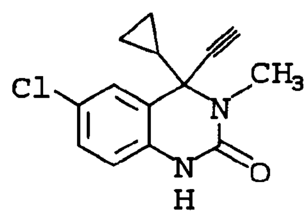

6-Chloro-3,4-dihydro-3-methyl-4-cyclopropyl-4-ethynl-2(1H)-quinazolinone, (Example 48).

H.

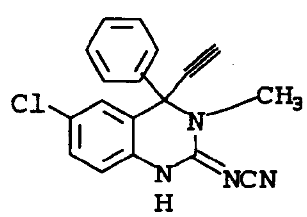

6-Chloro-3,4-dihydro-3-methyl-4-phenyl-4-ethynyl-2(1H)-cyanoiminoquinazolinone, (Example 84).

I.

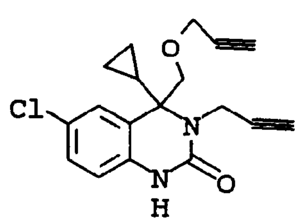

6-Chloro-3,4-dihydro-3-(2-propynyl)-4-cyclopropyl-4-(2-propynyloxymethyl)-2(1H)-quinazolinone, (Example 54),

J.

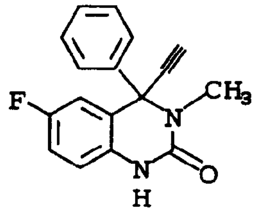

6-fluoro-3,4-dihydro-3-methyl-4-phenyl-4-ethynyl-2(1H)-quinazolinone, (Example 96).

K.

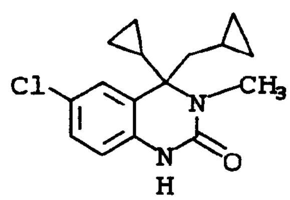

6-Chloro-3,4-dihydro-3-methyl-4-cyclopropyl-4-cyclopropylmethyl-2(1H)-quinazolinone (Example 15),

L.

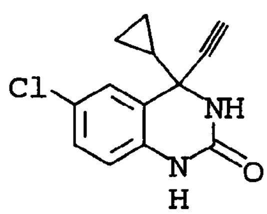

6-chloro-3,4-dihydro-4-cyclopropyl-4-ethynyl-2(1H)-quinazolinone (Example 49), or

M

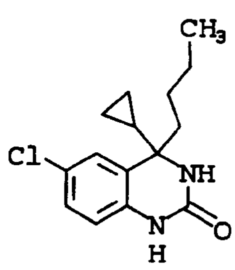

6-chloro-3,4-dihydro-4-cyclopropyl-4-(N-butyl)-2(1H)-quinazolinone (Example 39).

The compounds of the present invention may have asymmetric centers and occur as racemates, racemic mixtures and as individual diastereomers, or enantiomers, with all isomeric forms being included in the present invention.

When any variable (e.g., G, $R^1$, $R^2$, $R^3$, etc.) occurs more than one time in any constituent or in formula I, its definition on each occurrence is independent of its definition at every other occurrence. Also, combinations of substituents and/or variables are permissible only if such combinations result in stable compounds.

As used herein except where noted, "alkyl" is intended to include both branched- and straight-chain saturated aliphatic hydrocarbon groups having the specified number of carbon atoms; "alkenyl" is intended to cover

both branched- and straight chain alkyl groups with at least one carbon-carbon double bond; "alkynyl" is intended to cover both branched- and straight chain alkyl groups with at least one carbon-carbon triple bond. "Halogen" or "halo" as used herein, means fluoro, chloro, bromo and iodo.

As used herein, with exceptions as noted, "aryl" is intended to mean phenyl, naphthyl, tetrahydronaphthyl, biphenyl, phenanthryl, anthryl or acenaphthyl.

The term heterocycle or heterocyclic, as used herein except where noted, represents a stable 5- to 7-membered monocyclic or stable 8- to 11-membered bicyclic ring which is either saturated or unsaturated, and which consists of carbon atoms and from one to four heteroatoms selected from the group consisting of N, O and S, and wherein the nitrogen and sulfur heteroatoms may optionally be oxidized, and including any bicyclic group in which any of the above-defined heterocyclic rings is fused to a benzene ring. The heterocyclic ring may be attached at any heteroatom or carbon atom which results in the creation of a stable structure. Examples of such heterocyclic elements include piperidinyl, piperazinyl, 2-oxopiperazinyl, 2-oxopiperidinyl, 2-oxopyrrolidinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazoyl, benzopyranyl, benzothiazolyl, benzoxazolyl, furyl, tetrahydrofuryl, benzofuranyl, tetrahydropyranyl, thienyl, benzothienyl, thiamorpholinyl, thiamorpholinyl sulfoxide, thiamorpholinyl sulfone, and oxadiazolyl.

The compounds of the present invention can be synthesized by the following schemes.

SCHEME I

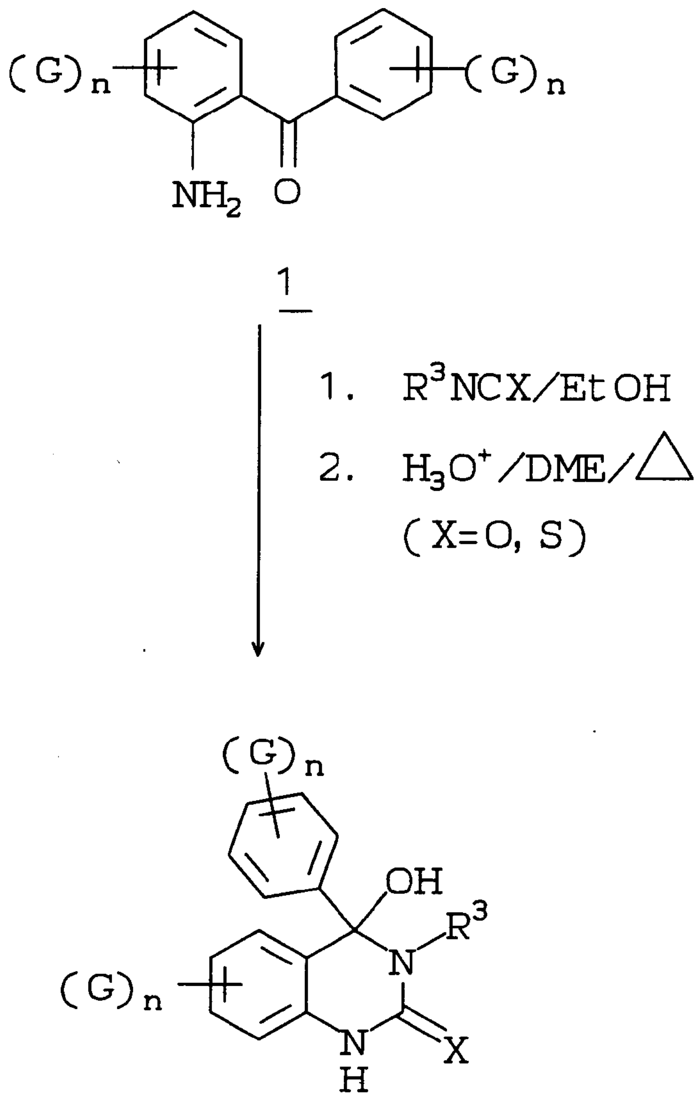


1

2

SCHEME I cont'd

1 2

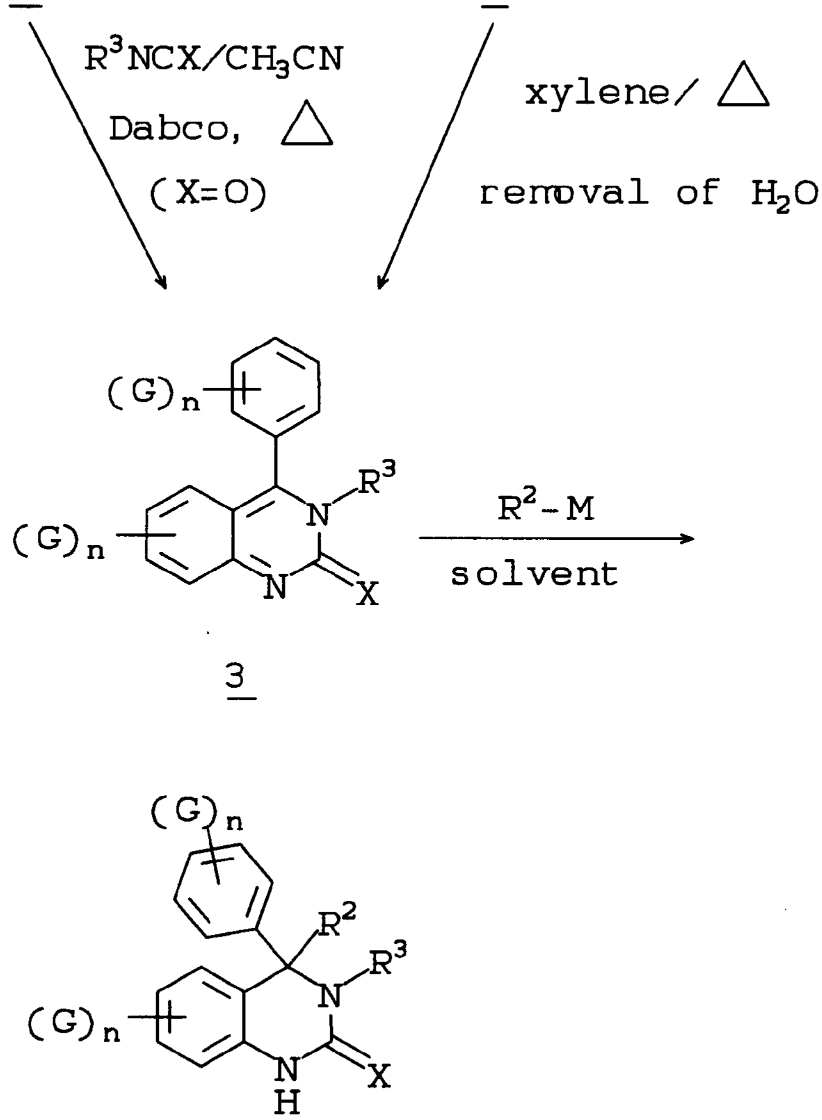


3

4

According to Sternbach, L. et al. J. Org. Chem. 31, 1007 (1966), the hydroxy intermediate 2 is prepared by heating 1 in the presence of absolute ethanol with an excess of e.g. alkylisothiocyanate, followed by hydrolysis with aqueous acid to give 2. Subsequent dehydration of 2 by refluxing in xylenes affords the tetraene 3, wherein aromaticity of the benzene ring is lost. Nucleophilic addition at the 4-position to form a second carbon-carbon bond can be accomplished in a variety of ways. In Scheme I, reaction with the appropriate Grignard reagent gives 4. Alternatively, 1 (X=O) may be directly converted to 3 by reaction with excess alkylisothiocyanate, acetonitrile, and triethylenediamine in the presence of heat.

Scheme I is subject to a variety of modifications or adaptations, including Scheme IA.

## SCHEME IA

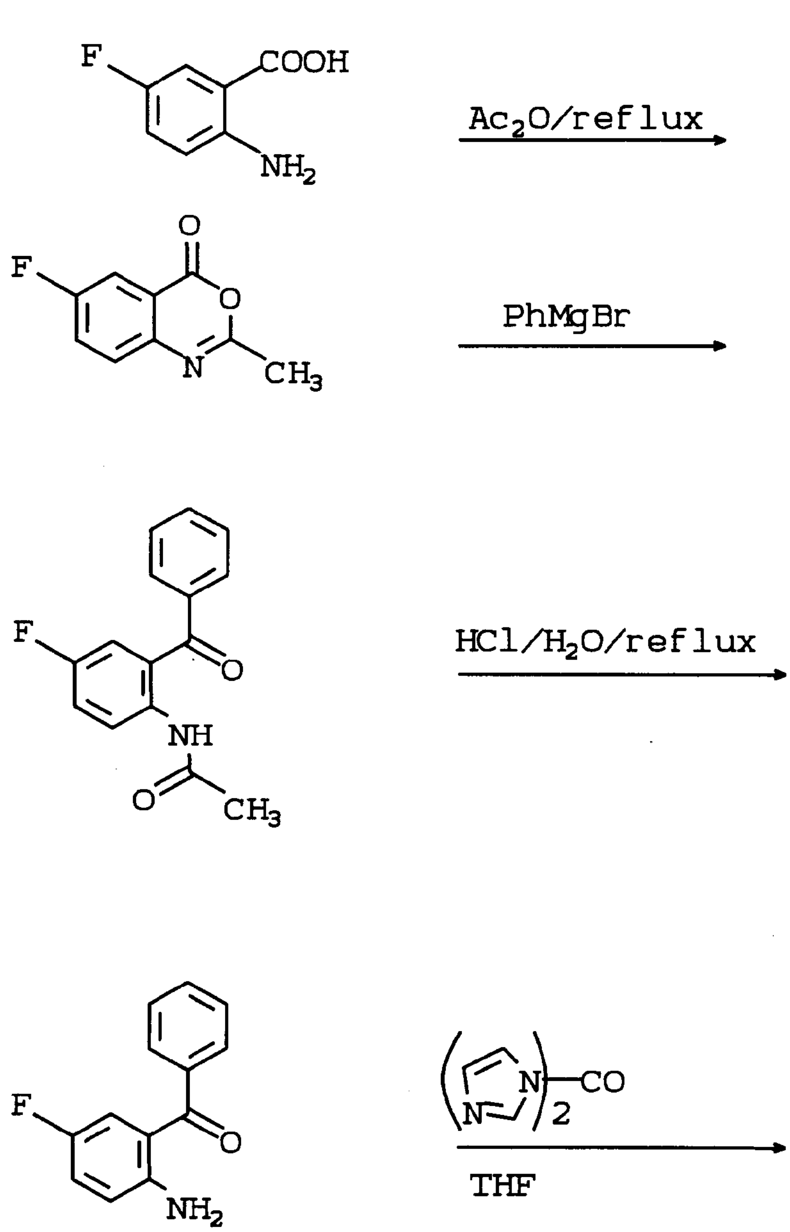

SCHEME IA, CON'T.

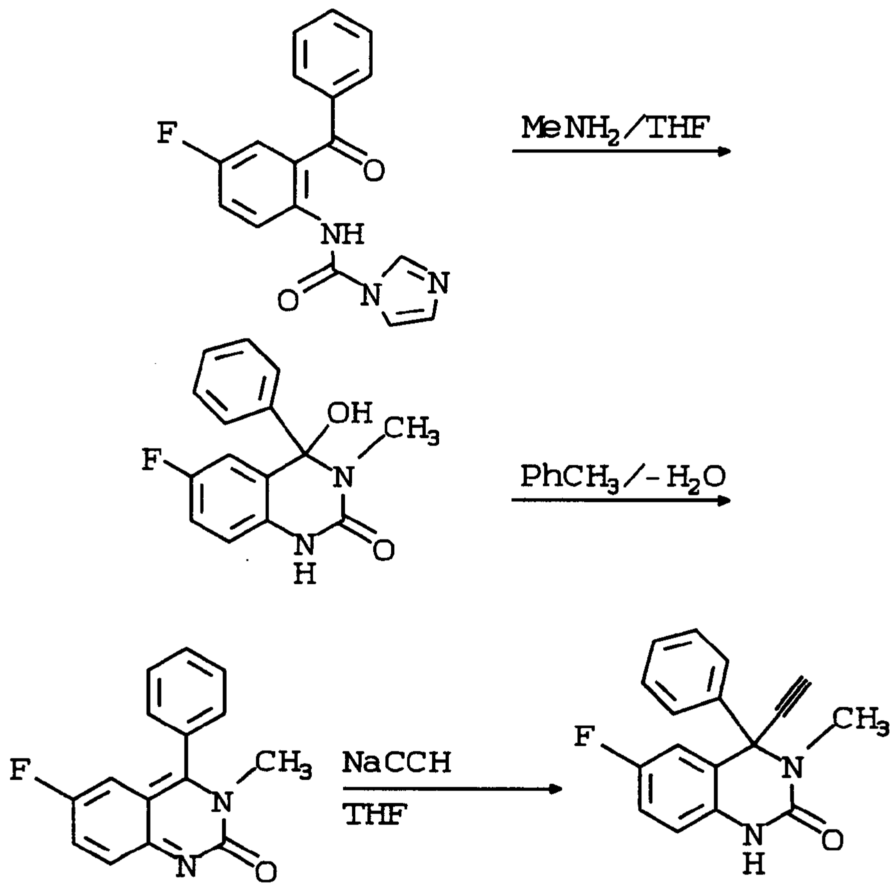


Schemes I and Ia are also specifically illustrated in Examples 93-100 among others.

Scheme II is another method of synthesizing the compounds of this invention, although not preferred.

## SCHEME II

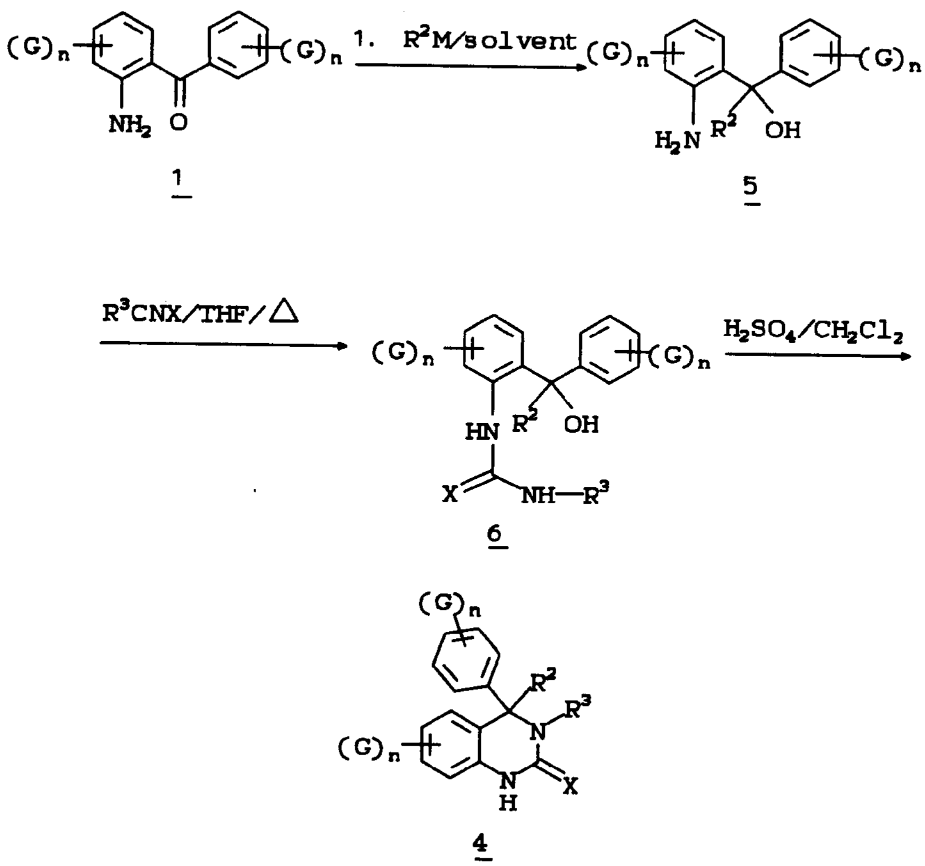


1 5 6 4

Reaction of the parent benzophenone 1 with the appropriate alkyl metal gives the carbinol intermediate 5. Subsequent treatment with e.g. alkylisothiocyanate, followed by acid gives 4.

Separate scheme III is preferred in the synthesis of 4,4-dialkyl quinazolin-2-ones.

SCHEME III

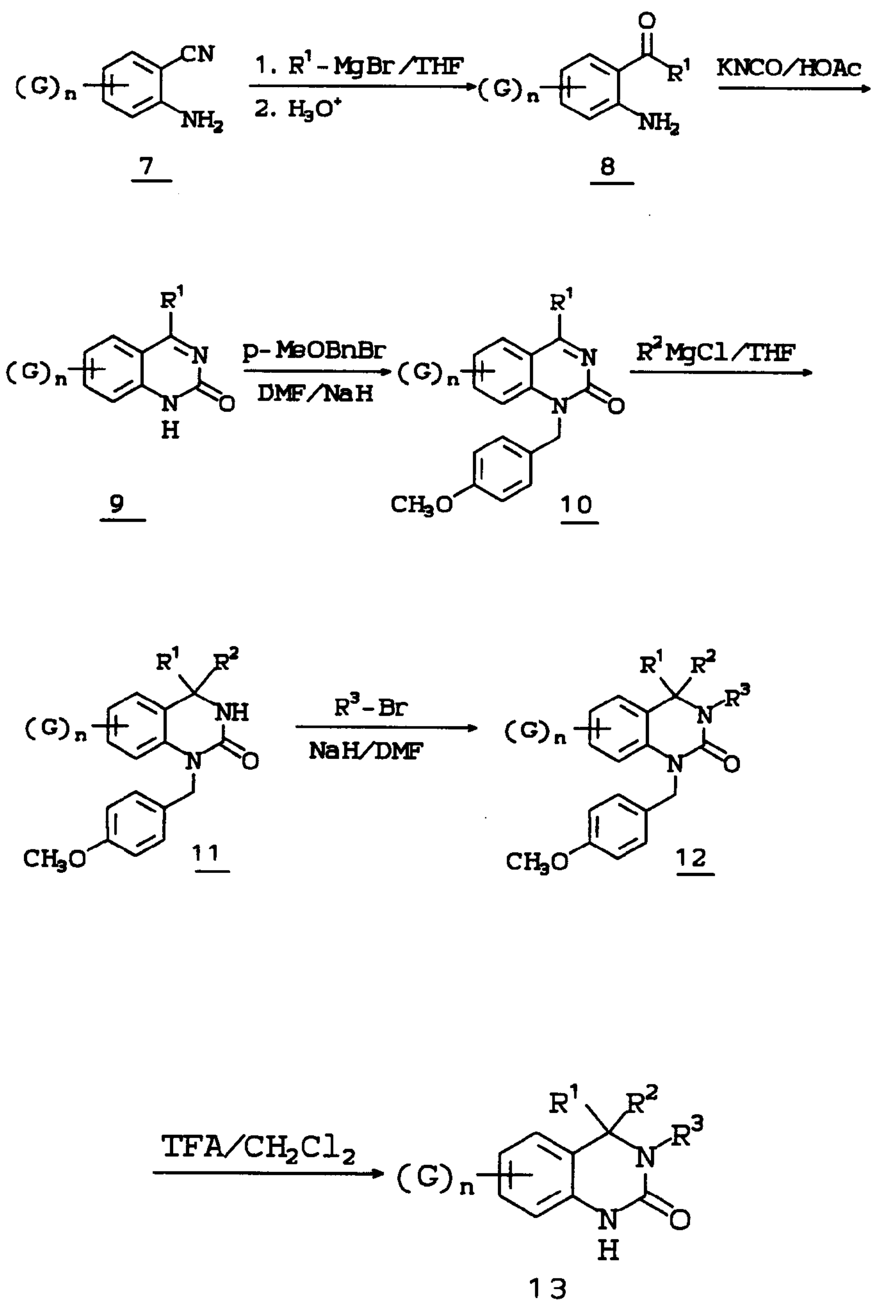
1. $R^1$-MgBr/THF
2. $H_3O^+$
KNCO/HOAc
7
8
p-MeOBnBr
DMF/NaH
$R^2MgCl$/THF
9
10
$R^3$-Br
NaH/DMF
11
12
$TFA/CH_2Cl_2$
13

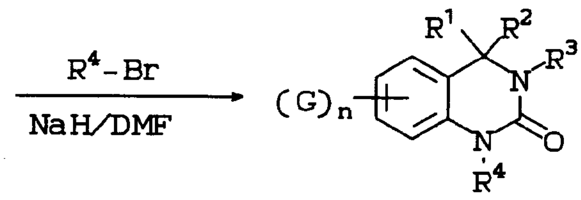


14

Scheme III is characterized by the Grignard addition to quinazoline 10. $R^1$ and $R^2$ lack an aromatic or heterocyclic ring. Appropriate protecting groups can be employed as needed. Preparation of 14, wherein $R^4$ is not H, is conveniently achieved by reaction of 13 with $R^4$-Br.

Scheme III is subject to a variety of modifications or adaptations, including Schemes IIIA and IIIB.

## SCHEME IIIA

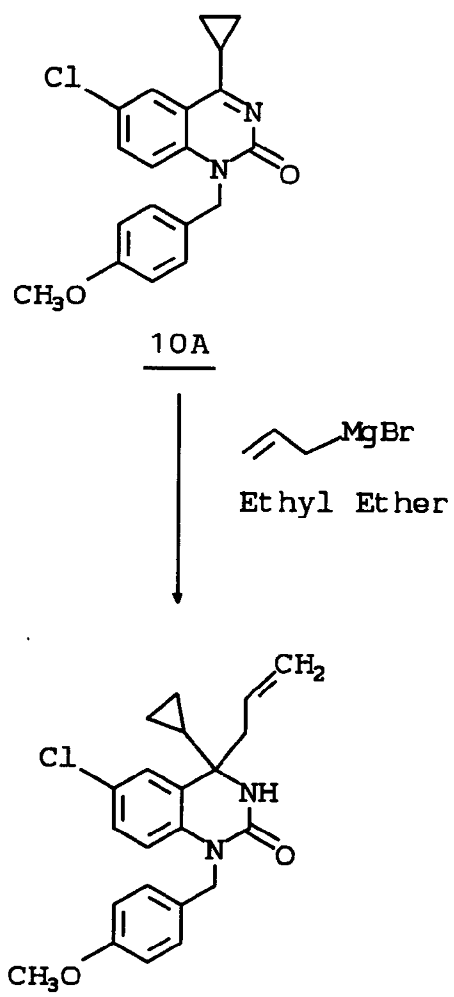

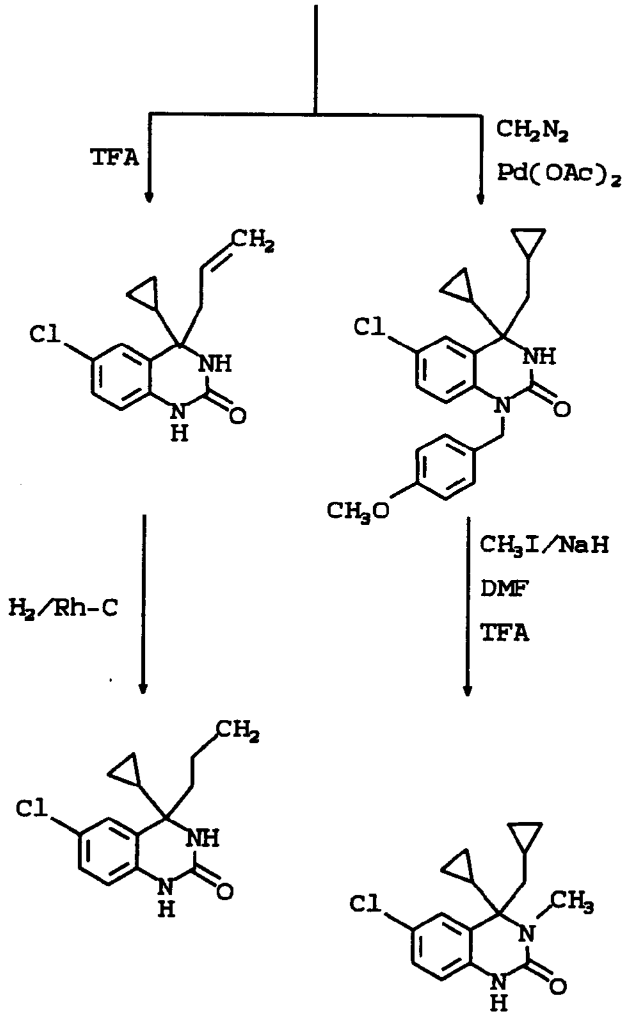
TFA
$CH_2N_2$
$Pd(OAc)_2$
$H_2$/Rh-C
$CH_3I$/NaH
DMF
TFA

## SCHEME IIIB

10A

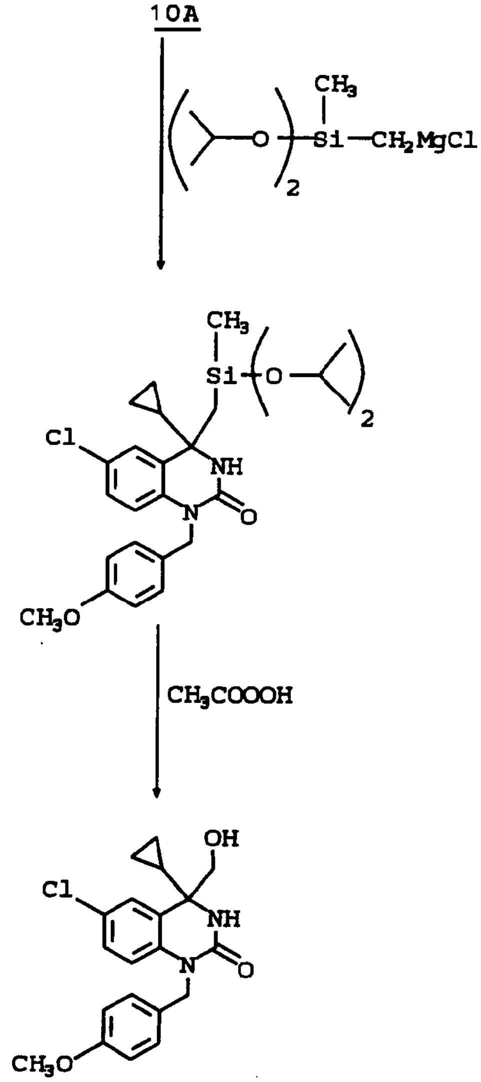

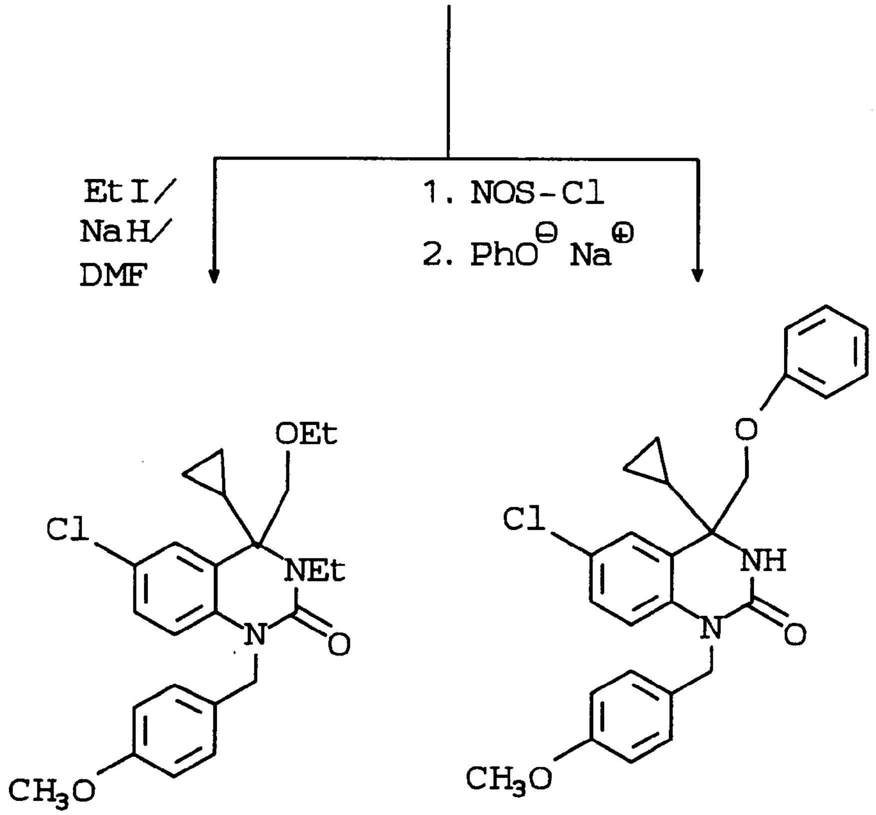


Treatment of either product with TFA removes the N-protecting PMB group. Schemes III and variants IIIA and IIIB are also illustrated by Examples 14-57.

Additional methods of synthesizing the compounds of formulas I or II include R-metal addition to tetraene, base catalyzed addition of solvent conjugate bases to 3-alkyl-4-aryl-quinazoline-2-ones or 3-alkyl-4-arylquinazoline-2-thiones, organosilane addition to tetraene, acid catalyzed cyclization, direct conversion of thiocarbonyl to oxocarbonyl, synthesis via o-aminophenyl ketone imidazoleurea intermediates, cyanoguanidine synthesis, and others.

Organosilane addition is illustrated by Schemes IV and IVA.

SCHEME IV

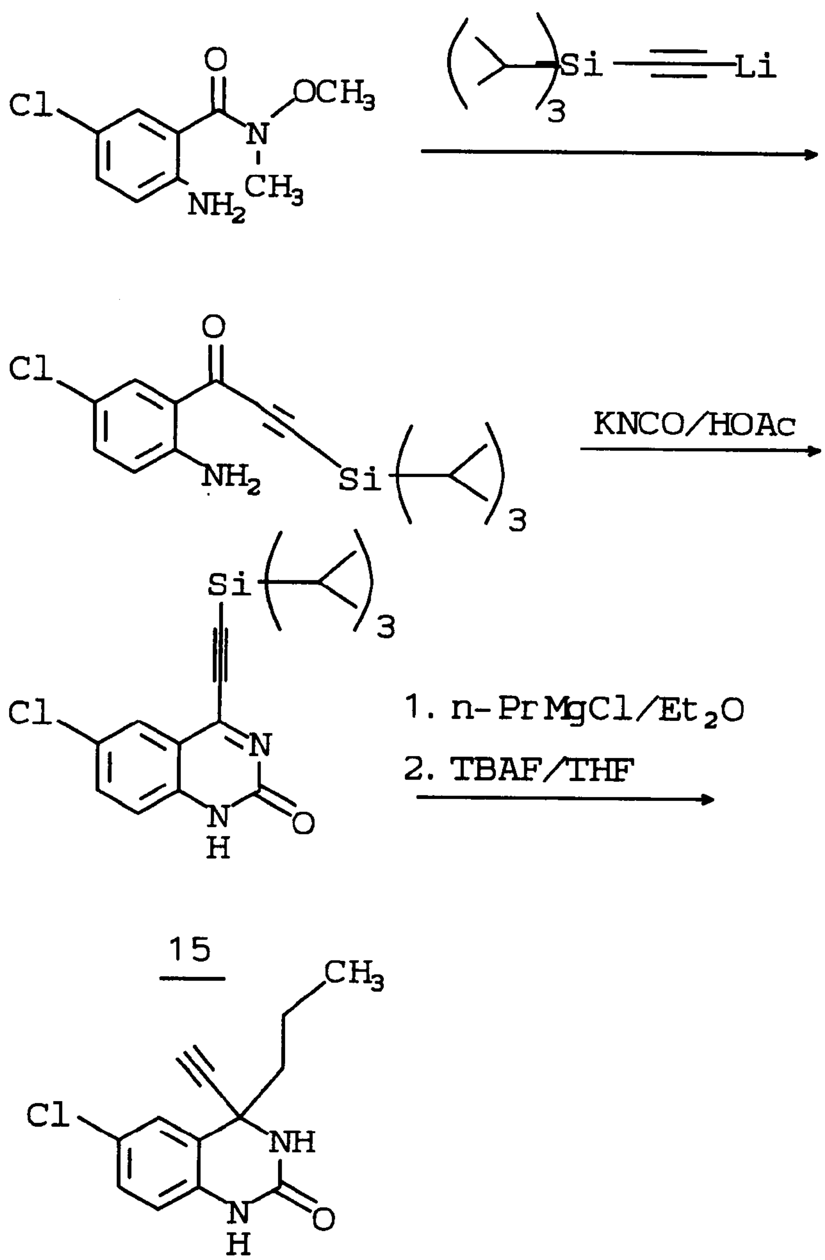


15

## SCHEME IVA

15

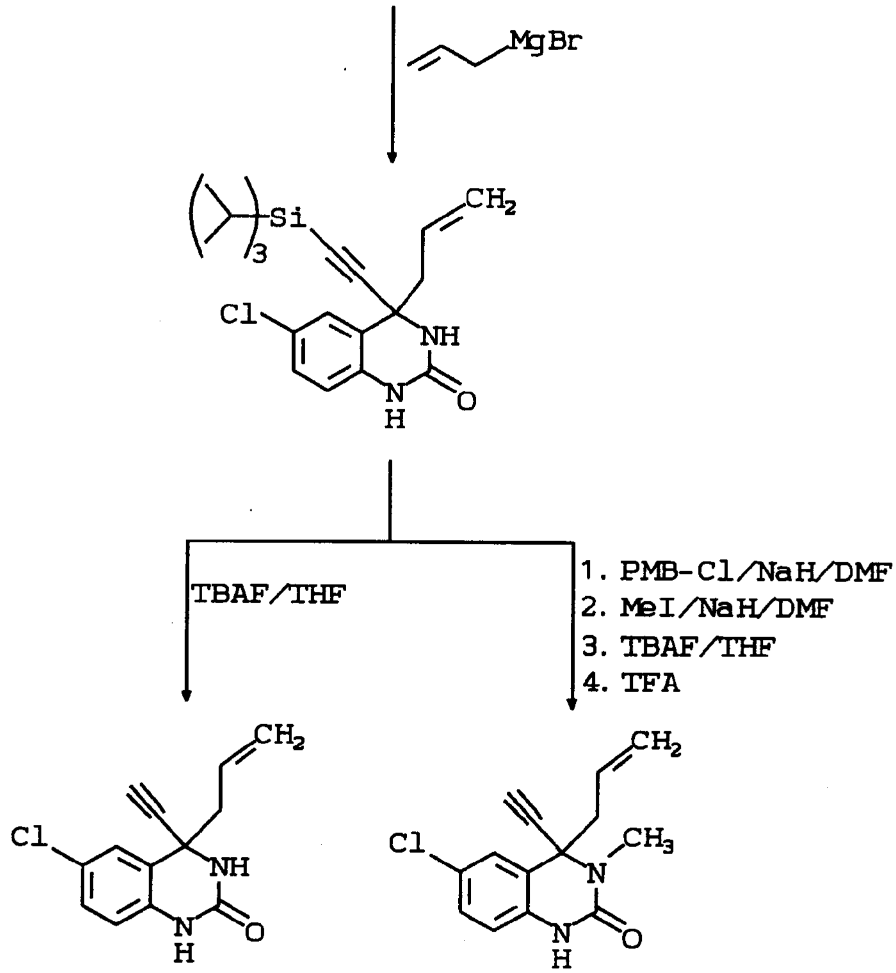


Organosilane processes are also illustrated by Examples 58-71.

Another variation of the Grignard synthesis involves hydrogenation as one of the final steps. See Scheme V.

## SCHEME V

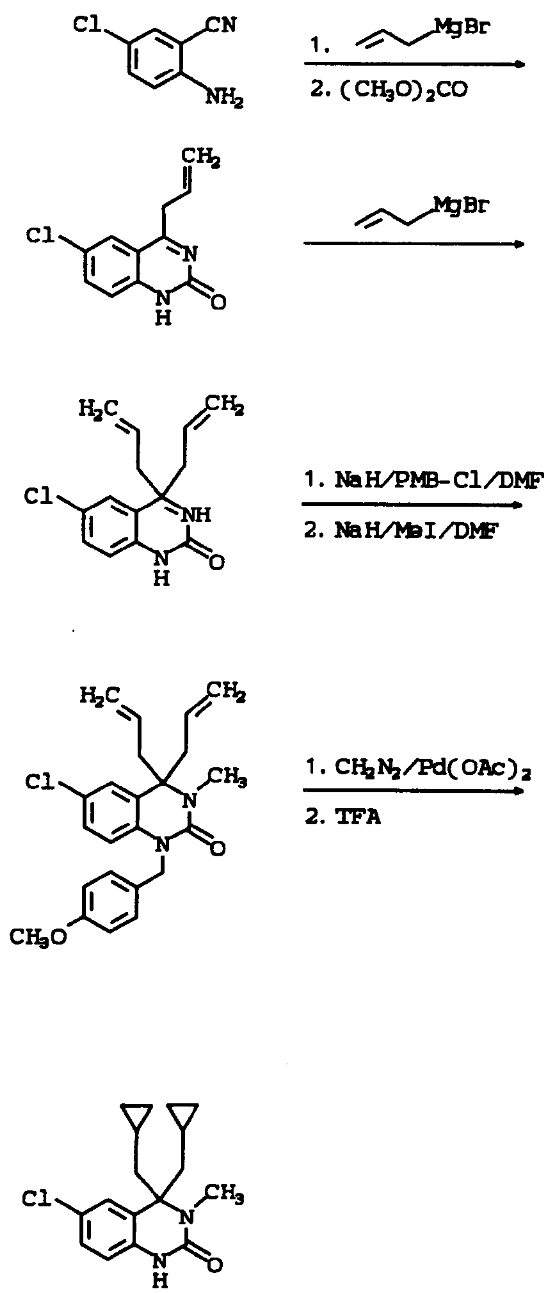


Scheme V is specifically illustrated by Examples 72, 74-78.

SCHEME VI

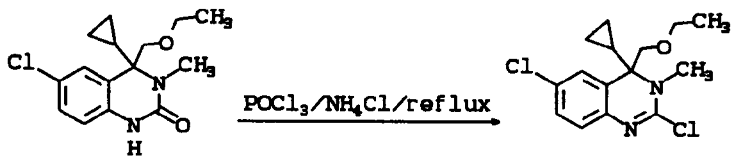


16

Example 52

16

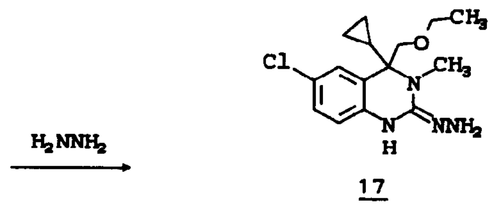


17

16

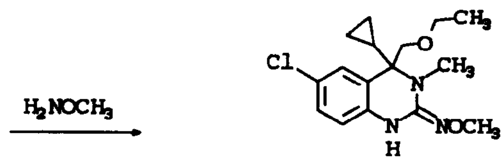


18

16

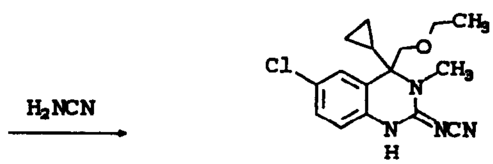


19

Scheme VI sets forth various ways of derivatizing the keto group at the 2-position. Reaction with phosphoryl chloride and the appropriate amine yields desired compounds 17, 18 and 19. Scheme VI is applicable to a wide variation in substituents $R^1$, $R^2$, $R^3$ and $R^4$, and is specifically illustrated by Examples 79-81, 86 and 89.

The compounds of the present inventions are useful in the inhibition of HIV reverse transcriptase, (including resistant varieties thereof) the prevention of treatment of infection by human immunodeficiency virus (HIV) and the treatment of consequent pathological conditions such as AIDS. Treating AIDS or preventing or treating infection by HIV is defined as including, but not limited to, treating a wide range of states of HIV infection: AIDS, ARC (AIDS related complex), both symptomatic and asymptomatic, and actual or potential exposure to HIV. For example, the compounds of this invention are useful in treating infection by HIV after suspected past exposure to HIV by e.g., blood transfusion, exchange of body fluids, bites, accidental needle stick, or exposure to patient blood during surgery.

For these purposes, the compounds of the present invention may be administered orally, parenterally (including subcutaneous injections, intravenous, intramuscular, intrasternal injection or infusion techniques), by inhalation spray, or rectally, in dosage unit formulations containing conventional non-toxic pharmaceutically-acceptable carriers, adjuvants and vehicles.

Thus, in accordance with the present invention there is further provided a method of treating and a pharmaceutical composition for treating HIV infection and AIDS. The treatment involves administering to a patient in need of such treatment a pharmaceutical composition comprising a pharmaceutical carrier and a therapeutically-effective amount of a compound of the present invention.

These pharmaceutical compositions may be in the form of orally-administrable suspensions or tablets; nasal sprays; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweeteners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquidify and/or dissolve in the rectal cavity to release the drug.

The compounds of this invention can be administered orally to humans in a dosage range of 1 to 100 mg/kg body weight in divided doses. One preferred dosage range is 1 to 10 mg/kg body weight orally in divided doses. Another preferred dosage range is 1 to 20 mg/kg body weight orally in divided doses. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The present invention is also directed to combinations of the HIV reverse transcriptase inhibitor compounds with one or more agents useful in the treatment of AIDS. For example, the compounds of this invention may be effectively administered, whether at periods of pre-exposure and/or post-exposure, in combination with effective amounts of the AIDS antivirals, immunomodulators, antiinfectives, or vaccines, such as those in the following table.

TABLE

ANTIVIRALS

| Drug Name | Manufacturer | Indication |
|---|---|---|
| AL-721 | Ethigen (Los Angeles, CA) | ARC, PGL HIV positive, AIDS |
| Recombinant Human Interferon Beta | Triton Biosciences (Almeda, CA) | AIDS, Kaposi's sarcoma, ARC |
| Acemannan | Carrington Labs (Irving, TX) | ARC (See also immunomodulators) |
| Cytovene Ganciclovir | Syntex (Palo Alto, CA) | sight threatening CMV peripheral CMV retinitis |
| d4T Didehydrodeoxy-thymidine | Bristol-Myers (New York, NY) | AIDS, ARC |
| ddI Dideoxyinosine | Bristol-Myers (New York, NY) | AIDS, ARC |
| EL10 | Elan Corp, PLC (Gainesville, GA) | HIV infection (See also immunomodulators) |

| Drug Name | Manufacturer | Indication |
|---|---|---|
| Trisodium Phosphonoformate | Astra Pharm. Products, Inc. (Westborough, MA) | CMV retinitis, HIV infection, other CMV infections |
| Dideoxycytidine; ddC | Hoffman-La Roche (Nutley, NJ) | AIDS, ARC |
| Novapren | Novaferon Labs, Inc. (Akron, OH) Diapren, Inc. (Roseville, MN, marketer) | HIV inhibitor |
| Peptide T Octapeptide Sequence | Peninsula Labs (Belmont, CA) | AIDS |
| Zidovudine; AZT | Burroughs Wellcome (Rsch. Triangle Park, NC) | AIDS, adv, ARC pediatric AIDS, Kaposi's sarcoma, asymptomatic HIV infection, less severe HIV disease, neurological involvement, in combination with other therapies. |

| Drug Name | Manufacturer | Indication |
|---|---|---|
| Ansamycin LM 427 | Adria Laboratories (Dublin, OH) Erbamont (Stamford, CT) | ARC |
| Dextran Sulfate | Ueno Fine Chem. Ind. Ltd. (Osaka, Japan) | AIDS, ARC, HIV positive asymptomatic |
| Virazole Ribavirin | Viratek/ICN (Costa Mesa, CA) | asymptomatic HIV positive, LAS, ARC |
| Alpha Interferon | Burroughs Wellcome (Rsch. Triangle Park, NC) | Kaposi's sarcoma, HIV in combination w/Retrovir |
| Acyclovir | Burroughs Wellcome | AIDS, ARC, asymptomatic HIV positive, in combination with AZT. |
| Antibody which neutralizes pH labile alpha aberrant Interferon in an immuno-adsorption column | Advanced Biotherapy Concepts (Rockville, MD) | AIDS, ARC |
| L-697,661 | Merck & Co. (Rahway, NJ) | AIDS, ARC, HIV positive asymptomatic; also in combination with AZT. |
| L-696,229 | " | " |

## IMMUNO-MODULATORS

| Drug Name | Manufacturer | Indication |
|---|---|---|
| AS-101 | Wyeth-Ayerst Labs. (Philadelphia, PA) | AIDS |
| Bropirimine | Upjohn (Kalamazoo, MI) | advanced AIDS |
| Acemannan | Carrington Labs, Inc. (Irving, TX) | AIDS, ARC (See also anti-virals) |
| CL246,738 | American Cyanamid (Pearl River, NY) Lederle Labs (Wayne, NJ) | AIDS, Kaposi's sarcoma |
| EL10 | Elan Corp, PLC (Gainesville, GA) | HIV infection (See also anti-virals) |
| Gamma Interferon | Genentech (S. San Francisco, CA) | ARC, in combination w/TNF (tumor necrosis factor) |
| Granulocyte Macrophage Colony Stimulating Factor | Genetics Institute (Cambridge, MA) Sandoz (East Hanover, NJ) | AIDS |

| Drug Name | Manufacturer | Indication |
|---|---|---|
| Granulocyte Macrophage Colony Stimulating Factor | Hoeschst-Roussel (Somerville, NJ) Immunex (Seattle, WA) | AIDS |
| Granulocyte Macrophage Colony Stimulating Factor | Schering-Plough (Madison, NJ) | AIDS<br>AIDS, in combination w/AZT |
| HIV Core Particle Immunostimulant | Rorer (Ft. Washington, PA) | seropositive HIV |
| IL-2 Interleukin-2 | Cetus (Emeryville, CA) | AIDS, in combination w/AZT |
| IL-2 Interleukin-2 | Hoffman-La Roche (Nutley, NJ) Immunex | AIDS, ARC, HIV, in combination w/AZT |
| Immune Globulin Intravenous (human) | Cutter Biological (Berkeley, CA) | pediatric AIDS, in combination w/AZT |
| IMREG-1 | Imreg (New Orleans, LA) | AIDS, Kaposi's sarcoma, ARC, PGL |
| IMREG-2 | Imreg (New Orleans, LA) | AIDS, Kaposi's sarcoma, ARC, PGL |

| Drug Name | Manufacturer | Indication |
|---|---|---|
| Imuthiol Diethyl Dithio Carbamate | Merieux Institute (Miami, FL) | AIDS, ARC |
| Alpha-2 Interferon | Schering Plough (Madison, NJ) | Kaposi's sarcoma w/AZT: AIDS |
| Methionine-Enkephalin | TNI Pharmaceutical (Chicago, IL) | AIDS, ARC |
| MTP-PE Muramyl-Tripeptide | Ciba-Geigy Corp. (Summit, NJ) | Kaposi's sarcoma |
| Granulocyte Colony Stimulating Factor | Amgen (Thousand Oaks, CA) | AIDS, in combination w/AZT |
| rCD4 Recombinant Soluble Human CD4 | Genentech (S. San Francisco, CA) | AIDS, ARC |
| rCD4-IgG hybrids | | AIDS, ARC |
| Recombinant Soluble Human CD4 | Biogen (Cambridge, MA) | AIDS, ARC |
| Interferon Alfa 2a | Hoffman-La Roche (Nutley, NJ) | Kaposi's sarcoma AIDS, ARC, in combination w/AZT |

| Drug Name | Manufacturer | Indication |
|---|---|---|
| SK&F106528 Soluble T4 | Smith, Kline & French Laboratories (Philadelphia, PA) | HIV infection |
| Thymopentin | Immunobiology Research Institute (Annandale, NJ) | HIV infection |
| Tumor Necrosis Factor; TNF | Genentech (S. San Francisco, CA) | ARC, in combination w/gamma Interferon |

ANTI-INFECTIVES

| Drug Name | Manufacturer | Indication |
|---|---|---|
| Clindamycin with Primaquine | Upjohn (Kalamazoo, MI) | PCP |
| Fluconazole | Pfizer (New York, NY) | cryptococcal meningitis, candidiasis |
| Pastille Nystatin Pastille | Squibb Corp. (Princeton, NJ) | prevention of oral candidiasis |
| Ornidyl Eflornithine | Merrell Dow (Cincinnati, OH) | PCP |
| Pentamidine Isethionate (IM & IV) | LyphoMed (Rosemont, IL) | PCP treatment |

| Drug Name | Manufacturer | Indication |
|---|---|---|
| Piritrexim | Burroughs Wellcome (Rsch. Triangle Park, NC) | PCP treatment |
| Pentamidine isethionate for inhalation | Fisons Corporation (Bedford, MA) | PCP prophylaxis |
| Spiramycin | Rhone-Poulenc Pharmaceuticals (Princeton, NJ) | cryptosporidial diarrhea |
| Intraconazole-R51211 | Janssen Pharm. (Piscataway, NJ) | histoplasmosis; cryptococcal meningitis |
| Trimetrexate | Warner-Lambert | PCP |

OTHER

| Drug Name | Manufacturer | Indication |
|---|---|---|
| Recombinant Human Erythropoietin | Ortho Pharm. Corp. (Raritan, NJ) | severe anemia assoc. with AZT therapy |
| Megestrol Acetate | Bristol-Myers (New York, NY) | treatment of anorexia assoc. w/AIDS |
| Total Enteral Nutrition | Norwich Eaton Pharmaceuticals (Norwich, NY) | diarrhea and malabsorption related to AIDS |

It will be understood that the scope of combinations of the compounds of this invention with AIDS antivirals, immunomodulators, anti-infectives or vaccines is not limited to the list in the above Table, but includes in principle any combination with any pharmaceutical composition useful for the treatment of AIDS.

EXAMPLE 1

R-Metal Addition to Tetraene

A. 6-Chloro-3,4-dihydro-3,4-dimethyl-4-phenyl-2(1H)-quinazolinthione

A 200 mL 3-neck round bottomed flask fitted with a stirring bar and argon inlet was charged with 6-chloro-3-methyl-4-phenyl-2(3H)-quinazolinthione (1.00 g, 3.49 mmol, see Sternbach et al., J. Org. Chem. 31, 1007 (1966)) and 70 mL of dry ether. Methylmagnesium bromide (2.91 M in ether, 3.6 mL) was added to this solution over 1-2 min. at 25°C. After 1 h the mixture was poured into 400 mL of ice-cold saturated aqueous $NH_4Cl$ solution and extracted with $CH_2Cl_2$. The combined organic layers were washed with saturated aqueous $NaHCO_3$ solution, brine, and dried ($MgSO_4$). Removal of the drying agent and solvent left a foam which, upon trituration with ether/hexane, deposited 849 mg of the title compound as a crystalline white solid, mp 167-168°C. An analytical sample was recrystallized from EtOAc/hexane:
$^1$H-NMR ($CDCl_3$): δ 1.94 (s, 3H), 3.09 (s, 3H), 6.46 (d, J=2Hz, 1H), 6.68 (d, J=8.5Hz, 1H), 7.07 (dd, J=2, 8.5Hz, 1H), 7.30-7.48 (m, 5H), 8.58 (s, 1H).

B. 6-Chloro-3,4-dihydro-3-methyl-4-phenyl-4-vinyl-2(1H)-quinazolinthione

In the manner outlined in part A of this Example, a THF solution of vinylmagnesium bromide (threefold excess) was added to a THF solution of 6-chloro-3-methyl-4-phenyl-2(3H)-quinazolinthione to give the title compound, mp 185-185.5°C ($CH_3CN$).
$^1$H-NMR ($CDCl_3$): δ 3.16 (s, 3H), 5.02 (d, J=17Hz, 1H), 5.50 (d, J=10.5Hz, 1H), 6.22 (dd, J=10.5, 17Hz, 1H), 6.49 (d, J=2Hz 1H), 6.77 (br d, J=8.5Hz, 1H), 7.15 (dd, J=8.5, 2Hz, 1H), 7.30-7.50 (m, 5H), 8.60-9.00 (br s, 1H).

C. 6-Chloro-3,4-dihydro-3-methyl-4-ethyl-4-phenyl- 2(1H)-quinazolinthione

In the manner outlined in part A of this Example, a solution of ethylmagnesium bromide (3M in ether, threefold excess) was added to an ether solution of 6-chloro-3-methyl-4-phenyl-2(3H)-quinazolinthione to give the title compound, mp 175-176°C.
$^1$H-NMR ($CDCl_3$): δ 0.91 (t, J=7Hz, 3H), 2.22 (m, 1H), 2.50 (m, 1H), 3.03 (s, 3H), 6.50 (d, J=2Hz 1H), 6.65 (d, J=8.5Hz, 1H), 7.07 (dd, J=2, 8.5Hz, 1H), 7.28-7.48 (m, 5H), 8.48 (s, 1H).

D. 6-Bromo-3,4-dihydro-4-ethyl-3-methyl-4-phenyl- 2(1H)-quinazolinthione

In the manner outlined in Part A of this Example, and starting with 2-amino-4-bromobenzophenone, 6-Bromo-3,4-dihydro-4-ethyl-3-methyl-4-phenyl-2(1H)quinazolinthione was prepared. MP: 180-183°C.
$^1$H-NMR ($CDCl_3$): δ 0.91 (t, J=7Hz, 3H), 2.19-2.29 (m, 1H), 2.48-2.55 (m, 1H), 3.02 (s, 3H), 6.58 (d, J=8.5Hz 1H), 6.63 (d, J=2Hz, 1H), 7.21 (dd, J=2, 8.5Hz, 1H), 7.28-7.41 (m, 5H), 8.41 (br s, 1H).

E. 6-Chloro-3,4-dihydro-4-ethynyl-3-methyl-4-phenyl-2(1H)quinazolinthione

To a 100 mL round bottomed three-neck flask fitted with a stirring bar and an argon inlet was added 6-chloro-3-methyl-4-phenyl-2(3H)quinazolinthione (0.573 g, 2.0 mmol) and dry THF (30 mL). Sodium acetylide (2.35 g, 8.8 mmol, 18% slurry in xylene) was added via syringe at room temperature. The reaction was stirred at room temperature for 18 h. The mixture was poured into ethyl acetate, washed with water, brine, dried ($Na_2SO_4$), filtered and concentrated in vacuo. The crude product was chromatographed on silica gel using 20% EtOAc in hexanes as eluant, and product cuts were recrystallized from boiling EtOAc-hexanes to afford the title compound, 160 mg white solid after drying at 40°C, 0.05 torr. MP: 228-229°C.
$^1$H-NMR ($CDCl_3$): δ 3.07 (s, 1H), 3.34 (s, 3H), 6.71 (d, J=8.6Hz, 1H), 6.98 (d, J=2.2Hz, 1H), 7.16 (dd, J=1.8, 8.6Hz, 1H), 7.41-7.58 (m, 5H), 8.62 (brs, 1H).

F.3,8-Dimethyl-4-phenyl-4-ethyl-6-chloro-3,4-dihydro-2(1H)-quinazolinone

In a manner according to Part A of this Example, the title compound was prepared from 3,8-dimethyl-4-phenyl-6-chloro-2(3H)quinazolinone and ethyl magnesium bromide in 70% yield. MP: 245-247°C.
$^1$H-NMR ($CDCl_3$): δ 0.89 (t, J=6, 3H), 2.20 (s, 3H), 2.16-2.30 (m, 1H), 2.40-2.51 (m, 1H), 2.64 (s, 3H), 6.41 (d,

J=2, 1H), 6.69 (broad s, NH, 1H), 6.91 (d, J=2, 1H), 7.25-7.31 (m, 2H), 7.34-7.44 (m, 3H).

G. 3,8-Dimethyl-4-phenyl-4-ethyl-6-chloro-3,4-dihydro-2(1H)quinazolinthione

In a manner according to Part A of this Example, the title compound was prepared from 3,8-dimethyl-4-phenyl-6-chloro-2(3H)quinazolinthione and ethyl magnesium bromide in 75% yield. MP: 182-183°C (decomp). $^1$H-NMR ($CDCl_3$): δ 0.91 (t, J=6, 3H), 2.18-2.29 (m, 1H), 2.27 (s, 3H), 2.46-2.56 (m, 1H), 3.03 (s, 3H), 6.38 (d, J=2, 1H), 6.97 (d, J=2, 1H), 7.29-7.35 (m, 2H), 7.35-7.43 (m, 3H), 7.9-8.1 (very broad s, 1H).

H. 6-Chloro-3-cyclopropyl-3,4-dihydro-4-ethynyl-4-phenyl-2-(1H)-quinazolinone

6-Chloro-4-phenyl-3-cyclopropyl-2(H)-quinazolinone (2.1g, 7.1 mmol, see Example 5, Part A, below) was dissolved in 30 mL dry tetrahydrofuran in a three-necked flask equipped with magnetic stirrer and $N_2$ inlet. The resulting yellow solution was treated with 10 mL of an 18% suspension of sodium acetylide (ca. 5 equiv.) in xylene/mineral oil at room temperature. The resulting mixture was stirred 1.5 hr under $N_2$ during which the yellow color discharged. The mixture was decanted under $N_2$ into a mixture of 50 mL of 10% aqueous citric acid and 50 mL ethyl acetate. The layers were separated and the aqueous layer extracted with two more portions of ethyl acetate. The combined ethyl acetate extracts were washed with water and saturated aqueous NaCl and dried over $Na_2SO_4$. Concentrations of the filtered ethyl acetate extract in vacuo gave a gummy solid which was stirred with hexane to give 1.71g of crude title compound. Chromatography of the hexane washes on silica gel gave, on elution with ethyl acetate:
hexane (3:1) fractions containing pure title compound which was recrystallized from n-butylchloride to give a while solid.
MP: 163-165°C
mass spectrum M+$H^+$ 323 (FAB/thioglycerol),
NMR ($CDCl_3$): δ 0.5 (1H, m), 0.68 (2H, m), 1.09 spectrum M+$H^+$ 323(FAB/thioglycerol),
NMR ($CDCl_3$): δ 0.5 (m, 1H), 0.68 (m, 2H), 1.09 (m, 1H), 247 (m, 1H), 2.998 (s, 1H), 6.58 (dd, J=8.73, ≦1Hz, 1H), 7.078 (d, J=2.26 Hz, 1H), 7.123 (dd, J=8.73, 2.26 Hz, 1H), 7.34 (m, 3H), 7.6 (broad, 1H).

I. 6-Chloro-3-cyclobutyl-3,4-dihydro-4-ethynyl-4-phenyl-2(1H)-quinazolinone

In the manner outlined for the 3-cyclopropyl analogue in Part H of this example, 6-chloro-3-cyclobutyl-2(3H)quinazolinone was reacted with excess sodium acetylide in THF. Chromatography of the crude product on silica gel gave, on elution with 10% ethyl acetate/n-butyl chloride, the title compound, MP 193-195°C. NMR (δ, $CDCl_3$): 1.49 (m, 1H), 1.61 (m, 1H), 1.77 (m, 1H), 2.11 (m, 1H), 2.99 (pentuplet, 1H), 3.14 (pentuplet, 1H), 3.91 (pentuplet, 1H), 6.70 (d, J=8.54 Hz, 1H), 6.90 (d, J=2.2 Hz, 1H), 1.076 (q, J=2.2, 8.54, 1H), 7.39 (m, 3H), 7.62 (d, J=7.8 Hz, 2H).

J. 6-Chloro-3,4-dihydro-3-methyl-4-cyclopropyl-4-phenyl-2(1H)quinazolinthione

In the method of Part A of this example, a solution of cyclopropylmagnesium bromide (freshly prepared, 1M in THF) was added to a THF solution of 6-chloro-3-methyl-4-phenyl-2(3H)quinazolinthione. The crude product was flash chromatographed on silica gel (15% EtOAc in hexane) and an analytical sample of the title compound was obtained by recrystallization from cyclohexane; MP 204-206°C dec.
$^1$H-NMR ($CDCl_3$) δ 0.115-0.132 (m, 2H), 0.65 (m, 1H), 0.80 (m, 1H), 1.56 (m, 1H), 6.27 (d, J=2 Hz, 1H), 6.63 (m, 1H), 7.12 (dd, J=2, 8.5 Hz, 1H), 7.36-7.54 (m, 5H), 8.28 (br s, 1H).

K. 6-Chloro-3,4-dihydro-3,4-diethyl-4-phenyl-2(1H)-quinazolinthione

Ethylmagnesium bromide (3M in ether, 3.3 mL) was added to a solution of 6-chloro-3-ethyl-4-phenyl-2(3H)quinazolinthione (1.0 g, 3.3 mmol) in 20 mL of THF. After 3h the reaction was worked up as described above. Silica gel chromatography and recrystallization from EtOAc-hexane afforded the pure title compound, MP 212-213°C.
$^1$H NMR ($CDCl_3$) δ 0.90 (t, J=7 Hz, 3H), 1.06 (t, J=7 Hz, 3H), 2.20 (m, 1H), 2.55 (m, 1H), 3.40-3.68 (m, 2H), 6.43 (d, J=2 Hz, 1H), 6.66 (d, J=8 Hz, 1H), 7.07 (dd, J=2, 8 Hz, 1H), 7.22-7.50 (m, 5H), 8.56 (br s, 1H).

L. 6-Chloro-3,4-dihydro-4-ethynyl-3-isopropyl-4-phenyl-2(1H)quinazolinthione

Step I: 5-chloro-2-(1-imidazolecarbonylamino)-benzophenone

5-Chloro-2-aminobenzophenone (23.2 g, 0.10 mmol) and carbonyldiimidazole (17.6 g, 0.11 mmol) were combined in 100 mL $CH_2Cl_2$ and the mixture refluxed under argon for 4 hours. The initial yellow solution deposited a thick, white precipitate. The mixture was cooled and the solid, 5-chloro-2-(1-imidazolecarbonylamino)benzophenone, which was collected and washed with $CH_2Cl_2$ by suction, afforded 23.2 g (71%), MP: 185-187°C.
NMR ($CDCl_3$ + 1-2 drops DMSO-$d_6$): δ 2.87 (1H, broad s), 6.71 (1H, d, J=2.44 Hz), 7.01 (1H d, J=1.47 Hz), 7.05 (1H d, J=1.3 Hz), 7.1 (1H dd, J=2.58, 8.49 Hz), 7.19 (2H, complex), 7.44 (5H, complex).

Step II: 6-chloro-4-phenyl-4-hydroxy-3-isopropyl-3,4-dihydroquinazoline-2(1H)one

To a suspension of the imidazolylcarbonyl intermediate (5 g, 15.4 mmol) in 125 ml tetrahydrofuran was added excess (25 mL) isopropylamine and the reaction mixture heated to 40°C and stirred under argon. The resulting clear solution was evaporated in vacuo, dissolved in ethyl acetate, washed with 10% aqueous citric acid, water, brine, dried over $MgSO_4$, filtered, concentrated, and chromatographed using EtOAc-Hexane (1:1) to afford a yellow foam 6-chloro-4-phenyl-4-hydroxy-3-isopropyl-3,4-dihydroquinazoline-2(1H)one (4.24 g, 86%).

Step III : 6-chloro-3-isopropyl-4-phenyl-2(3H)quinazolinthione

The hydroxy intermediate (4.86 g, 15.1 mmol) was dissolved in toluene (250 mL), and the mixture was refluxed for 17 hours under argon to remove the water using a Dean Stark trap. The resulting yellow solution was concentrated under vacuum to afford crystalline tetraene which was triturated with hexane, filtered and dried in vacuo (60°C, 0.05 torr) to afford the bright yellow title compound (3.75 g, 83%) FAB MS: M+H=299 m/e.
NMR (DMSO): δ 1.53 (6H, d; J=7 Hz), 4.28 (1H, m), 6.64 (1H, d; J=2.3 Hz), 7.33 (2H, m), 7.70 (5H; complex).

Step IV: 6-Chloro-3,4-dihydro-4-ethynyl-3-isopropyl-4-phenyl-2(1H)quinazolinthione

6-chloro-3-isopropyl-4-phenyl-2(3H)-quinazolinthione (1.50 g, 5.02 mmol) suspended in dry THF to which (30 mL) sodium acetylide (4.02 g, 15.1 mmol, 18% slurry in xylene) was added via syringe at -25°C. Upon warming to room temperature over 3 hours, the reaction mixture was poured into 10% aqueous citric acid and ethyl acetate, washed with water, brine, dried ($Na_2SO_4$), filtered and concentrated in vacuo.
The crude product was recrystallized from boiling EtOAc-hexanes to afford the title compound, a white solid 545 mg after drying at 40°C, 0.05 torr. MP: 229-230°C.
$^1$H-NMR ($CDCl_33$): δ 1.14 (d, J=6.8 Hz, 3H), 1.60 (d, J=6.4 Hz, 3H), 3.01 (s, 1H), 3.56 (q, J=6.6 Hz, 1H), 6.75 (d, J=8.6 Hz, 1H), 6.85 (d, J=0.01 Hz, 1H), 7.08 (dd, J=0.7, 2.3 Hz, 1H), 7.35-7.41 (m, 3H), 7.68 (dd, 0.95, 2.25 Hz, 2H), 9.56 (s, 1H).

M. 6-Chloro-3,4-dihydro-4-ethyl-3-isopropyl-4-phenyl-2(1H)quinazolinthione

In the exact manner as outlined above for the preparation (Steps I, II, III) and reacton (Step IV) of tetraene 6-chloro-3-isopropyl-4-phenyl-2(3H)-quinazolinthione, a solution of ethylmagnesium bromide (3M in ether, threefold excess) was added via syringe to a THF suspension of the tetraene 6-chloro-3-isopropyl-4-phenyl-2(3H)-quinazolinthione at -35°C and stirred for 1 hour. The reaction was poured into 2N HCl and ethyl acetate, the organic layer was washed with water, brine, dried ($Na_2SO_4$), filtered and concentrated in vacuo. The crude product was chromatographed on silica gel using 5% i-PrOH-$CHCl_3$ and recrystallized from boiling EtOAc-hexanes to afford the title compound, a white solid 183 mg after drying at 40°C, 0.05 torr.
MP:-235-237°C.
$^1$H-NMR ($CDCl_3$): δ 0.94 (t, J=7.3 Hz, 3H), 1.09 (d, J=6.7 Hz, 3H), 1.48 (d, J=6.6, 3H), 2.19 (m, 1H), 3.05 (q, J=6.8, 1H), 6.35 (d, J=2.4 Hz, 1H), 6.62 (d, J=8.2 Hz, 1H), 7.03 (dd, J=2.3, 8.5 Hz, 1H), 7.28-7.48 (m, 5H), 8.16 (s, 1H).

EXAMPLE 2

Base Catalyzed Addition of Solvent Conjugate Bases to 3-Alkyl-4-arylquinazoline-2-ones (thiones)

A. 6-chloro-3,4-dihydro-4-nitromethyl-4-Phenyl-2(1H)-quinazolinone

A solution of 6-chloro-4-phenyl-3-methyl-2(3H)-quinazolinone (1.0 g, 3.7 mmol) in nitromethane (5 ml) was treated with a catalytic amount of N,N,N',N'-tetramethylethane-1,2-diamine and refluxed 12 hours under nitrogen. Cooling the mixture provided 4-phenyl-4-nitromethyl-6-chloro-3,4-dihydro-2(1H)-quinazolinone as a white solid. MP: 240-dec; 299-300°C.
$^1$H-NMR ($CDCl_3$+DMSO-$d_6$): δ 2.79 (s, 3H), 5.17 (d, J=10, 1H), 5.33 (d, J=10, 1H), 6.58 (d, J=1.5, 1H), 6.73 (d, J=7, 1H), 7.11 (dd, J=1.5, 7, 1H), 7.28 (m, 2H), 7.42 (m, 3H), 8.6 (br s, 1H).

B. 6-Chloro-3,4-dihydro-4-phenyl-4-methylsulfinyl methyl-3-methyl-2(1H)quinazolinone

In a manner according to Part A of this Example, 6-chloro-4-phenyl-4-methylsulfinylmethyl-3,4-dihydromethyl-2(1H)quinazolinone was prepared from 6-chloro-4-phenyl-3-methyl-2-(3H)-quinazolinone (1.0 g, 3.7 mmol) by addition to a solution of sodium hydride (0.200 g of a 50% suspension in mineral oil) in dry dimethylsulfoxide (5.0 mL) at room temperature. The mixture was quenched on ice and acetic acid, and the semi-solid chloro-4-phenyl-4-methylsulfinylmethyl-3, 4-dihydromethyl-2(1H)quinazolinone was extracted with EtOAc. The crude product was chromatographed on alumina and recrystallized from 2-propanol to afford 6-chloro-3,4-dihydromethyl-4-phenyl-4-methylsulfinylmethyl-2(1H)-quinazolinone.
MP: 299-300°C (dec).
$^1$H-NMR ($CDCl_3$+DMSO-$d_6$): δ 2.47 (s, 3H), 2.72 (s, 3H), 3.73 (d, J=12, 1H), 3.90 (d, J=12, 1H), 6.48 (d, J=1.5, 1H), 6.73 (d, J=7, 1H), 7.06 (dd, J=1.5, 7, 1H), 7.4 (m, 5H), 9.31 (br s, 1H).

C. 6-Chloro-3,4-dihydro-3-methyl-4-nitromethyl-4-phenyl-2(1H)-quinazolinthione

A solution of 6-chloro-3-methyl-4-phenyl-2(3H)-quinazolinthione (0.8 g, 2.79 mmol) in nitromethane (20 ml) was treated with a catalytic amount of N,N,N',N'-tetramethylethane-1,2-diamine and heated at reflux for 18 hours under argon. Evaporation of the solvent and recrystallization of the solid residue from chloroform-hexanes afforded the title compound, a white solid after drying at 40°C, 0.05 torr. MP: 230-231°C.
$^1$H-NMR ($CDCl_3$): δ 3.23 (s, 3H), 5.29 (dd, J=11.5, 53.5Hz, 2H), 6.62 (d, J=2.2Hz, 1H), 6.75 (d, J=8.5, 1H), 7.20 (dd, J=2.2, 8.6Hz, 1H), 7.26-7.52 (m, 5H), 8.72 (br s, 1H).

D. 6-Chloro-3,4-dihydro-4-t-butoxycarbonylmethyl-3-methyl-4-phenyl-2(1H)quinazolinthione

To a 500 mL three-neck round bottomed flask fitted with a stirring bar, thermometer and an argon inlet was added diisopropylamine (1.12 g, 11.03 mmol) and 30 ml dry THF. The solution was chilled to -10°C before adding n-BuLi (4.41 ml, 11.03 mmol of a 2.5 M solution in hexane) via syringe over 5 minutes keeping the temperature ≦ 0°C. The reaction was chilled to -74°C in a dry ice-acetone bath and freshly distilled t-butyl acetate (1.28 g, 1.49 ml, 11.03 mmol) was added via syringe keeping the temperature ≦-70°C then stirred at -74°C for 1 hour before adding the tetraene. 6-Chloro-3-methyl-4-phenyl-2(3H)-quinazolinthione (3.00 g, 10.5 mmol) dissolved in THF (total volume=300 ml due to limited solubility) was added over 30 minutes via syringe keeping the temperature at ≦-65°C, the ice bath was removed, and the reaction was warmed to room temperature over 3 hours. The reaction mixture was diluted with EtOAc, washed with 10% citric acid, water, brine, dried ($Na_2SO_4$), filtered and concentrated in vacuo to afford 5.04 g of an expanded foam. Chromatography of this crude product on silica gel using 20% ethyl acetate as eluant, recrystallization from boiling $Et_2O$-hexanes afforded 2.6 g of the title compound after drying at 40°C, 0.05 torr. MP: 103-105°C.
$^1$H-NMR ($CDCl_3$): δ 1.38 (br s, 3H), 3.15 (s, 1H), 3.29 (dd, J=13.8, 57.2Hz, 2H), 6.62 (d, J=2.1Hz, 1H), 6.70 (d, J=8.6Hz, 1H), 7.11 (dd, J=2.3, 8.6Hz, 1H), 7.29-7.44 (m, 5H), 8.73 (br s, 1H).

E. 6-Chloro-3,4-dihydro-4-aminomethyl-3-methyl-4-phenyl-2(1H)quinazolinthione

To a 100 mL round bottomed flask fitted with a stirring bar and an argon inlet was added 6-chloro-3,4-dihydro-3-methyl-4-nitromethyl-4-phenyl-2(1H)-quinazolinthione (1.50 g, 4.78 mmol) and 25 ml THF. The reaction mixture was chilled to 0°C, 1.0 M $LiAlH_4$ in THF (4.78 ml, 4.78 mmol) was added over 5 minutes via syringe and stirred at 0°C for 1 hour. The reaction mixture was quenched into aqueous saturated Na K tartrate,

extracted with EtOAc, washed with water, brine, dried ($Na_2SO_4$), filtered and concentrated in vacuo to affored 1.6 g of an expanded foam. MPLC chromatography of this crude product on silica gel using ethyl acetate and eluant, followed by recrystallization from boiling EtOAc-hexanes, afforded 435 mg of the title compound after drying at 60°C, 0.05 torr.
MP: 184-185°C.
$^1$H-NMR ($CDCl_3$): δ 1.19 (br s, 2H), 3.13 (s, 3H), 3.59 (dd, J=13.7, 61.4Hz, 2H), 6.57 (d, J=2.14Hz, 1H), 6.74 (d, J=0.85Hz, 1H), 7.12-7.16 (m, 1H), 7.35-7.46 (m, 5H), 8.72 (br s, 1H).

F. 6-Chloro-3,4-dihydro-4-formamidomethyl-3-methyl-4-phenyl-2(1H)quinazolinthione

To a 50 mL round bottomed flask fitted with a stirring bar, reflux condenser and argon inlet was added 6-chloro-3,4-dihydro-4-aminomethyl-3-methyl-4-phenyl-2(1H)quinazolinthione (0.092 g, 0.29 mmol) and 10 ml ethyl formate. The reaction mixture was heated to reflux for 18 hours. The reaction mixture was stripped to dryness, the residue chromatographed on silica gel using 30% hexanes in ethyl acetate as eluant, and the pure fractions were recrystallized from boiling EtOAc-hexanes to afford 45 mg of the title compound after drying at 60°C, 0.05 torr. MP: 183-185°C.
$^1$H-NMR ($CDCl_3$): δ 3.16 (d, J=7.3 Hz, 3H), 3.94 (dd, 9.1, 15.5Hz, 0.5H), 4.08-4.24 (m, 1H), 4.55 (dd, 7.3, 13.9Hz, 0.5H), 6.51 (dd, J=2.2, 38.1Hz, 1H), 6.90 (d, J=8.5Hz, 1H), 7.13-7.21 (m, 1H), 7.39-7.52 (m, 5H), 8.21 (s, 0.5H), 8.94 (br s, 0.5H), 9.32 (br s, 0.5H).

G. 6-Chloro-3,4-dihydro-4-methanesulfonamidomethyl-3-methyl-4-phenyl-2(1H)quinazolinthione

To a 25 mL round bottomed flask with a stirring bar and argon inlet was added 6-chloro-3,4-dihydro-4-aminomethyl-3-methyl-4-phenyl-2(1H)quinazolinthione (0.070 g, 0.22 mmol), 5 ml methylene chloride, methanesulfonic anhydride (0.042 mg, 0.242 mmol) and finally diisopropylethylamine (31 mg, 0.242 mmol). The reaction mixture was stirred at room temperature for 18 hours. The reaction mixture was stripped to dryness, the residue chromatographed on silica gel using 50% ethyl acetate in hexanes as eluant, and the pure fractions were evaporated to a glass, dried at 60°C, 0.05 torr to afford 32 mg of the title compound. MP: glass.
$^1$H-NMR ($CDCl_3$: δ 2.89 (s, 3H), 3.14 (s, 3H), 4.04 (dd, J=2.0, 7.8Hz, 1H), 4.15 (dd, J=5.5, 10.4Hz, 1H), 6.57 (d, J=2.2, 1H), 6.84 (br d, 2H), 7.34-7.48 (m, 5H), 9.62 (br s, 1H).

H. 6-Chloro-3,4-dihydro-4-(N,N-dimethyl)aminomethyl-3-methyl-4-phenyl-2(1H)quinazolinthione

To a 100 mL round bottomed flask with a stirring bar and an argon inlet was added 6-chloro-3,4-dihydro-4-aminomethyl-3-methyl-4-phenyl-2(1H)-quinazolinthione (0.318 g, 1.0 mmol) and 25 ml methanol. Small pieces of aluminum foil (0.539 g, 20 mmol) and mercuric chloride (27.2 mg, 0.1 mmol) were added to the solution followed by aqeuous formalin solution (0.150 g, 0.139 ml, 5 mmol). The reaction mixture was stirred at room temperature for 18 hours and was quenched into aqueous saturated Na K tartrate, extracted with EtOAc, washed with water, brine, dried ($Na_2SO_4$), filtered and concentrated in vacuo to give 120 mg of an oil. Chromatography of the crude product on silica gel using 15% ethyl acetate in hexanes as eluant, recrystallization from boiling $CHCl_3$-hexanes afforded 70 mg of the title compound after drying at 60°C, 0.05 torr.
MP: 222-223°C.
$^1$H-NMR ($CDCl_3$): δ 2.17 (s, 6H), 3.08 (s, 3H), 3.23 (dd, J=13.9, 66.2Hz, 2H), 6.47 (d, J=2.14Hz, 1H), 6.66 (d, J=8.14Hz, 1H), 7.09 (dd, 2.2, 8.5, 1H), 7.29-7.44 (m, 5H), 8.47 (br s, 1H).

I. 6-Chloro-3,4-dihydro-4-(2-hydroxyethyl)-3-methyl-4-phenyl-2(1H)quinazolinthione

To a 100 mL round bottomed flask with a stirring bar and an argon inlet was added 6-chloro-3,4-dihydro-4-t-butyoxycarbonylmethyl-3-methyl-4-phenyl-2(1H)quinazolinthione (0.403 g, 1.0 mmol) and 15 ml dry THF. The reaction mixture was chilled to 0°C and $LiAlH_4$ (1 ml, 1 mmol; 1.0 M LAH in THF) was added. The reaction mixture was stirred at room temperature for 18 hours and was quenched into aqueous saturated Na K tartrate, extracted with EtOAc, washed with water, brine, dried ($Na_2SO_4$), filtered and concentrated in vacuo to give 500 mg of an oil. Chromatography of the crude products on silica gel using 40% ethyl acetate in hexanes as eluant, recrystallization from boiling EtOAc-hexanes afforded the title compound, 275 mg after drying at 60°C, 0.05 torr.
MP: 195-196°C.
$^1$H-NMR ($CDCl_3$): δ 1.60 (br s, 1H), 2.47-2.57 (m, 1H), 2.81-2.85 (m, 1H), 3.09 (s, 3H), 3.68-3.81 (m, 2H), 6.50 (d, J=2.2Hz, 1H), 6.70 (d, J=8.5Hz, 1H), 7.11 (dd, J=2.2, 8.5Hz, 1H), 7.35-7.44 (m, 5H), 8.63 (br s, 1H).

J. 6-Chloro-3,4-dihydro-4-carboxymethyl-3-methyl-4-phenyl-2(1H)quinazolinthione

To a 50 mL round bottomed flask with a stirring bar and an argon inlet was added 6-chloro-3,4-dihydro-4-t-butyoxycarbonylmethyl-3-methyl-4-phenyl-2(1H)quinazolinthione (0.200 g, 0.50 mmol) and 10 ml methylene chloride. Trifluoroacetic acid (10 ml) was added and the reaction mixture was stirred at room temperature for 1 hour then stripped to dryness to give an orange oil. Chromatography of the crude product on silica gel using 1/2% acetic acid-ethyl acetate as eluant, recrystallization from boiling EtOAc-hexanes afforded the title compound, 97 mg, after drying at 60°C, 0.05 torr.
MP: 262-263°C.
$^1$H-NMR ($CDCl_3$): δ 3.09 (s, 3H), 3.38 (dd, J=10.6, 25.6Hz, 2H), 6.61 (s, 1H), 6.94-7.03 (m, 2H), 7.33-7.45 (m, 5H), 10.6 (br s, 1H).

EXAMPLE 3

Organosilane Addition to Tetraene

A. 6-Chloro-3,4-dihydro-4-allyl-3-methyl-4-phenyl-2(1H)quinazolinthione

To a 50 mL round bottomed flask with a stirring bar and an argon inlet was added 6-chloro-3-methyl-4-phenyl-2(3H)quinazolinthione (0.500 g, 1.74 mmol) and dry THF (10 mL). To this stirred solution allyl trimethylsilane (3.99 g, 5.54 ml, 34.9 mmol) was added at room temperature. The reaction mixture was chilled to 0°C, boron trifluoride etherate (0.741 g, 5.22 mmol) was added via syringe, bath removed and stirred at room temperature for 60 hours. The mixture was poured into ethyl acetate and saturated aqueous sodium bicarbonate. The EtOAc extract was washed with brine, dried ($Na_2SO_4$), filtered and concentrated in vacuo. The crude product was chromatographed on E. Merck 60 silica gel using 20% EtOAc in hexanes as eluant. The pure fractions were combined and recrystallized from boiling EtOAc-hexanes to afford the title compound, 155 mg of a white solid after drying at 40°C, 0.05 torr. MP: 143-144°C.
$^1$H-NMR ($CDCl_3$): δ 2.95 (dd, J=7.9, 13.9Hz, 1H), 3.08 (s, 3H), 3.23.(dd, J=7.9, 13.9Hz, 1H), 5.11-5.18 (m, 2H), 5.62-5.78 (m, 1H), 6.53 (d, J=2.06Hz, 1H), 6.66 (d, J=8.49Hz, 1H), 7.09 (dd, J=2.5, 11.0Hz, 1H), 7.36-7.44 (m, 5H), 8.45 (br s, 1H).

B. 6-Chloro-3,4-dihydro-4-cyano-3-methyl-4-phenyl-2(1H)-quinazolinthione

To a 50 mL round bottomed flask with a stirring bar and an argon inlet was added 6-chloro-3-methyl-4-phenyl-2(3H)quinazolinthione (0.666 g, 2.0 mmol) and dry methylene chloride (15 mL). Trimethylsilyl cyanide (0.750 g, 7.5 mmol) was added at room temperature, the reaction mixture was chilled to 0°C, boron trifluoride (492 μl, 4.0 mmol) was added with a syringe, the bath was removed and the mixture was stirred at room temperature for 18 hours. The mixture was poured into ethyl acetate and saturated aqueous sodium bicarbonate. The EtOAc extract was washed with water, brine, dried ($Na_2SO_4$), filtered and concentrated in vacuo. The crude product was chromatographed on silica gel using 10% EtOAc in hexanes as eluant. The product was recrystallized from boiling EtOAc-hexanes to afford the title compound, 275 mg of a white solid after drying at 60°C, 0.05 torr. MP: 220-221°C.
$^1$H-NMR ($CDCl_3$): δ 3.37 (s, 3H), 6.90 (d, J=8.7Hz, 1H), 6.93 (d, J=2.2Hz, 1H), 7.26 (dd, J=2.2, 8.7Hz, 1H), 7.47-7.56 (m, 5H), 9.456 (br s, 1H).

C. 6-Chloro-3,4-dihydro-4-carboxamido-3-methyl-4-phenyl-2(1H)quinazolinthione

To a 50 mL round bottomed flask with a stirring bar, cold concentrated $H_2SO_4$ containing 20% $H_2O$ (5.33 mL, 100 mmol $H_2SO_4$ + 1.07 ml $H_2O$) was added to 6-chloro-3,4-dihydro-4-cyano-3-methyl-4-phenyl-2(1H)quinazolinthione (314 mg, 1 mmol) in an ice bath at 0°C. After 1 hour the mixture was quenched into vigorously stirring ice water and extracted with EtOAc. The combined extracts were washed with water, brine and dried ($Na_2SO_4$), filtered, solvent removed in vacuo to afford 300 mg residue. Chromatography of the crude product on silica gel using 20% EtOAc in hexanes as eluant was followed by elution with 10% 2-propanol in chloroform to solubilize the product which had crystallized on the column. The combined cuts were recrystallized from boiling EtOAc-hexanes to afford the title compound, 71 mg of a white solid after drying at 60°C, 0.05 torr. MP: 274-275°C.
$^1$H-NMR ($CDCl_3$): δ 3.26 (s, 3H), 5.88 (br s, 1H), 5.96 (br s, 1H), 6.76 (d, J=8.9Hz, 1H), 6.87 (d, J=2.2Hz, 1H), 7.26 (dd, J=2.4, 8.7Hz, 1H), 7.45-7.46 (m, 5H), 8.70 (br s, 1H).

EXAMPLE 4

Acid Catalyzed Cyclization

6-chloro-3,4-dihydro-4-butyl-3-methyl-4-phenyl-2(1H)-quinazolinthione

A. 1-(2-amino-4-chlorophenyl)-1-phenylpentanol

To a three necked 100 mL round bottomed flask with a stirring bar and an argon inlet was added 2-amino-4-chlorobenzophenone (10 g, 43.16 mmol) and dry THF (250 mL). This stirred solution was cooled to -78°c and n-butyllithium (34.55 ml, 86.32 mmol) was added with a syringe over 5 minutes. The cooling bath was removed and the mixture was warmed to 0°C. The mixture was poured into water and the resulting mixture was extracted.with EtOAc. The EtOAc extract was washed with brine, dried ($Na_2SO_4$), filtered and concentrated in vacuo. The crude product was recrystallized from boiling EtOAc-hexanes and dried at 20°C, 0.05 torr. MP: 184-185°C.
$^1$H-NMR ($CDCl_3$): δ 0.875 (t, J=7.2, 3H), 1.00-1.15 (m, 1H), 1.22-1.26 (m, 2H), 2.00-2.30 (m, 2H), 3.75 (br s, 3H), 6.51 (d, J=8.4, 1H), 7.05 (dd, J=2.5, 11.0, 1H), 7.20-7.40 (m, 6H).

B. 1-(Methylaminothiocarbonylamino-4-chloro-phenyl)-1-phenylpentanol

To a 50 mL round bottomed flask with a stirring bar, argon inlet, and a reflux condenser was added 1-(2-amino-4-chlorphenyl)-1-phenylpentanol (1.00 g, 3.4 mmol), dry THF (20 mL), and methyl isothiocyante (1.18 mL, 17.25 mmol). This mixture was heated at reflux for 48 hours. The solvent and excess methyl isothiocyante were removed in vacuo and the residue was chromatographed on 100 g of silica gel using 5% 2-propanol in hexanes as eluant. There was obtained 1.00 g of 1-(methylaminothiocarbonylamino-4-chlorophenyl)-1-phenylpentanol as a white solid.
$^1$H-NMR ($CDCl_3$): δ 0.878 (t, J=7.1Hz, 3H), 1.05-1.50 (m, 4H), 2.11-2.40 (m, 2H), 2.73 (d, J=4.61Hz, 3H), 3.18 (br s, 1H), 5.30 (br s, 1H), 7.20-7.40 (m, 7H), 7.68 (s, 1H), 7.92 (s, 1H).

C. 6-Chloro-3,4-dihydro-4-butyl-3-methyl-4-phenyl-2(1H)quinazolinthione

To a 50 mL round bottomed flask with a stirring bar was added 1-(methylaminothiocarbonylamino-4-chlorophenyl)-1-phenylpentanol (0.50 g, 1.378 mmol) and dry $CH_2Cl_2$ (15.0 mL). This solution was cooled in an ice bath to 0°C and concentrated $H_2SO_4$ (0.81 mL, 1.50 mmol) was added. After 15 minutes, the mixture was warmed to 20°C and an additional 0.1 mL of concentrated $H_2SO_4$ was added. This mixture was stirred for 1.75 hours and the solution was diluted with 20 mL of saturated $NaHCO_3$ solution. The mixture was extracted with $CHCl_3$ and the combined extracts were washed with brine and dried ($MgSO_4$). Filtration, removal of the solvent in vacuo, and chromatography of the residue on 100 g of silica gel using 3.75% 2-propanol in hexanes as eluant gave 51 mg of 6-chloro-3,4-dihydro-4-propanol-3-methyl-4-phenyl-2(1H)quinazolinthione as a white solid. An analytical sample was prepared by recrystallization from EtOAc-hexanes. MP: 189-191°C.
$^1$H-NMR ($CDCl_3$): δ 0.89 (t, J=7.3, 3H), 1.15 (m, 1H), 1.20-1.45 (m, 3H), 2.18 (m, 1H), 2.47 (m, 1H), 3.04 (s, 3H), 6.50 (d, J=2.2Hz, 1H), 6.79 (d, J=8.5Hz, 1H), 7.05 (dd, J=2.2, 9.5Hz, 1H), 7.25-7.40 (m, 5H), 9.40 (s, 1H).

6-Chloro-3,4-dihydro-4-propyl-3-methyl-4-phenyl-2(1H)quinazolinthione

Following the procedure of this Example but substituting n-propyl magnesium bromide for n-butyllithium in Step A, 6-chloro-3,4-dihydro-3-methyl-4-phenyl-4-propyl-2(1H)quinazolinthione was obtained. MP: 175-176°C.
$^1$H-NMR ($CDCl_3$): δ 0.97 (t, J=6.1Hz, 3H), 1.21 (m, 1H), 1.40 (m, 1H), 2.18 (m, 1H), 2.45 (m, 1H), 3.05 (s, 3H), 6.51 (d, J=2.2Hz, 1H), 6.70 (d, J=8.5Hz, 1H), 7.07 (dd, J=2.2, 8.5Hz,1H), 7.30-7.50 (m, 5H), 8.76 (br s, 1H).

EXAMPLE 5

Synthesis of 3, 4-di-substituted 2(3H)-quinazolinones via o-aminophanyl ketone imidazoleureas

A. 6-Chloro-3-cyclopropyl-4-phenyl-2(3H)quinazolinone

5-Chloro-2-aminobenzophenone (23.2 g, 0.10 mmol) and carbonyldiimidazole (17.6 g, 0.11 mmol) were

combined in 100 mL $CH_2Cl_2$ and the mixture refluxed under $N_2$ for 4h. The initial yellow solution deposited a thick, white precipitate. The mixture was cooled and the solid, 5-chloro-2-(1-imidazolecarbonylamino) benzophenone, which was collected and washed with $CH_2Cl_2$ by suction, yield 23.2 g (71%), MP: 185-187°C.
$^1$H-NMR ($CDCl_3$ + 1-2 drops DMSO-$d_6$): δ 2.87 (broad s, 1H), 6.71 (d, J=2.44Hz, 1H), 7.01 (d, J=1.47Hz, 1H), 7.05 (d, J=1.3Hz, 1H), 7.1 (dd, J=2.58, 8.49Hz, 1H), 7.19 (complex, 2H), 7.44 (complex, 5H).

To a solution of the imidazolylcarbonyl intermediate (8 g crude, ca. 25 mmol) in $CH_2Cl_2$ was added excess (2.8 mL) cyclopropylamine. The resulting clear solution was stirred overnight at room temperature under $N_2$. The beige solid which had separated was collected and washed with methylene chloride to give 6-chloro-4-phenyl-4-hydroxy-3-cyclopropyl-3,4-dihydroquinazoline-2(1H)one (4.06 g, ca. 50%), MP: 262-263° (dec). The hydroxy intermediate (3.15 g, 10.0 mmol) was suspended in toluene (25 mL) and the mixture stirred and refluxed with water azeotrope for 4h under $N_2$. The resulting yellow suspension was cooled and filtered to give the bright yellow title compound (2.5 g, ca. 90%), MP: 286-287°C (dec),
$^1$H-NMR ($CDCl_3$): δ 0.63 (m, 2H), 0.97 (m, 2H), 3.21 (m, 1H), 7.12 (d, J≦1Hz, 1H), 7.5 (complex, broad, 4H), 7.67 (complex, broad, 3H).

B. <u>6-Chloro-3-cyclobutyl-4-phenyl-2(3H)quinazolinone</u>

According to the method of part A of this Example, cyclobutylamine (0.6 mL, 7 mmol) was added to a solution of 5-chloro-2-(1-imidazolecarbonylamino) benzophenone (1.95 g, 6 mmol) in 10 mL of dry THF under $N_2$. The mixture was heated and stirred 24h at 50°C and then 40h at 25°. The mixture was conc. in vacuo and the residue stirred with n-butylchloride (35 mL) and 10% aqueous citric acid (15 mL) to give a white solid identified as 6-chloro-4-hydroxy-4-phenyl-3-cyclobutyl-3,4-dihydro-2(1H)quinazolinone, MP 152-154°C (dec.). Heating the above intermediate in toluene (20 mL) at vigorous reflux with water separation in a Dean-Stark trap for four hours gave a bright yellow solution from which the tetraene title compound crystallized on cooling, 1.0 g, MP 228-230°C (dec.).

C. <u>9-Chloro-10b-cyclopentyl-2,3,4,10b-tetrahydro-6H-oxazolo(3,2-c)quinazolin-5-one</u>

The title compound was prepared by a method according to Part A of this Example, to give a colorless solid: mp 191-193°C,
$^1$H-NMR ($CDCl_3$): δ 1.27-1.35 (m, 1H), 1.38-1.70 (m, 7H), 2.20-2.30 (m, 1H), 3.59-3.65 (m, 1H), 3.72-3.78 (m, 1H), 4.07-4.12 (m, 1H), 4.18-4.22 (m, 1H), 6.75 (d, J=8 Hz, 1H), 7.20 (dd, J=8, 2 Hz, 1H), 7.33 (d, J=2 Hz, 1H), 7.22 (br s, 1H).

D. <u>9-Chloro-10b-phenyl-3(S)-(propan-2-yl)-2,3,6,10b-tetrahydro-5H-oxazolo-(3,2-C)-quinazolin-5-one</u>

In a method according to Part A of this Example, 326.00 mg (1.00 mmol) of 5-chloro-2,1-imidazolecarbonylaminobenzophenone and 103.00 mg (1.00 mmol) of (S)-valinol were reacted, with subsequent heating in anhydrous DMSO at 120°C for 72 hours, to give 150 mg (42%) title compound as a white solid, MP 148-150°C.
$^1$H-NMR ($CDCl_3$): δ 0.60 (d, J=4 Hz, 3H), 0.96 (d, J=4 Hz, 3H), 1.15-1.30 (m, 1H), 3.79 (m, 1H), 4.12-4.22 (m 2H) 6.74 (d, J=7 Hz, 1H), 7.12 (dd, 1H), 7.20 (s, 1H), 7.26-7.39 (m, 3H), 7.52 (d, J=5 Hz, 2H), 8.19 (broad s, 1H).

E. <u>9-Chloro-10b-phenyl-3(R)-(propan-2-yl)-2,3,6,10b-tetrahydro-5H-oxazolo-(3,2-C)-quinazolin-5-one</u>

In a method according to Part A of this Example, 326.00 mg (1.00 mmol) of 5-chloro-2,1-imidazolecarbonylaminobenzophenone and 103.00 mg (1.00 mmol) (R)-valinol were reacted, with subsequent heating in anhydrous DMSO at 120°C for 72h, to give 210.00 mg (60%) of title compound as a white solid, MP 146-147°C.
$^1$H-NMR ($CDCl_3$): δ 0.70 (d, J=4 Hz, 3H), 0.97 (d, J=4 Hz, 3H), 1.12-1.13 (m, 1H), 3.80 (m, 1H), 4.12-4.27 (m, 2H), 6.76 (d, J=7 Hz, 1H), 7.12 (dd, 1H), 7.19 (s, 1H), 7.25-7.40 (m, 3H), 7.51 (d, J=5 Hz, 1H), 8.39 (broad s, 1H).

F. <u>9-Chloro-10b-cyclopropyl-2,3,6,10b-tetrahydro-5H-oxazolo-(3,2-C)-quinazolin-5-one</u>

In a method according to Part A of this Example, 300.00 mg (1.04 mmol) (5-chloro-2-imidazolecarbonylamino)phenylcyclopropyl ketone and 63.52 mg (1.04 mmol) ethanolamine were reacted, with subsequent heating at 100°C for 18h in anhydrous DMSO, to give 180.00 mg (65%) of title compound as a white solid, MP 193-194°C.
$^1$H-NMR (DMSO-$d_6$): δ 0.20-0.50 (m, 4H), 1.23 (m, 1H), 3.50 (q, 1H), 3.79 (q, 1H), 3.89 (m, 1H), 4.12 (m, 1H), 6.90 (d, J=8 Hz, 1H), 7.27 (d, 2H), 9.69 (s, 1H).

EXAMPLE 6

Thiocarbonyl to Oxocarbonyl Conversion

A. 6-Chloro-3,4-dihydro-4-propyl-3-methyl-4-phenyl-2(1H)quinazolinone

To a 50 mL round bottomed flask with a stirring bar, reflux condenser, and an argon inlet was added 6-chloro-3,4-dihydro-4-propyl-3-methyl-4-phenyl-2(1H)quinazolinthione (165 mg, 0.50 mmol), dimethoxyethane (20 mL), water (5.0 mL), LiOH (119 mg, 5.00 mmol), and hydrogen peroxide (0.20 mL, of 30% aqueous solution). This mixture was heated at reflux for 30 minutes. The mixture was diluted with EtoAc and the solution was washed with water and brine. Drying ($Na_2SO_4$), filtration and removal of the solvent in vacuo left an oil. Chromatography of this material on 50 g of silica gel using 3.5% 2-propanol in chloroform as eluant, followed by trituration with hexanes gave 6-chloro-3,4-dihydro-4-propyl-3-methyl-4-phenyl-2(1H) quinazolinone as a crystalline solid. MP: 223-225°C.
$^1$H-NMR ($CDCl_3$): δ 0.92 (t, J=8.6Hz, 3H), 1.20 (m, 1H), 1.45 (m, 1H), 2.16 (m, 1H), 2.39 (m, 1H), 2.63 (s, 3H), 6.50 (d, J=2.1Hz, 1H), 6.72 (d, J=8.4Hz, 1H), 7.05 (dd, J=2.1, 8.4Hz, 1H), 7.25-7.50 (m, 5H), 9.18 (br s, 1H).

6-Chloro-3,4-dihydro-4-ethyl-3-methyl-4-phenyl-2(1H)quinazolinone

Following the procedure of Part A of this Example, but substituting 6-chloro-3,4-dihydro-4-ethyl-3-methyl-4-phenyl-2(1H)quinazolinthione for 6-chloro-3,4-dihydro-4-propyl-3-methyl-4-phenyl-2(1H)quinazolinthione, there was obtained 6-chloro-3,4-dihydro-4-ethyl-3-methyl-4-phenyl-2(1H)quinazolinone.
MP: 200-202°C.
$^1$H-NMR ($CDCl_3$): δ 0.91 (t, J=7.0 Hz, 3H), 2.25 (m, 1H), 2.48 (m, 1H), 2.66 (s, 3H), 6.53 (d, J=2.2 Hz, 1H), 6.72 (d, J=8.5 Hz, 1H), 7.04 (dd, J=2.2 Hz, 8.5, 1H), 7.20-7.50 (m, 5H), 9.32 (br s, 1H).

EXAMPLE 7

Cyanoguanidine Synthesis

6-Chloro-2-cyanoimino-3,4-dihydro-4-ethyl-3-methyl-4-phenyl-2(1H)quinazoline

A. 6-Chloro-3,4-dihydro-4-ethyl-3-methyl-2-methylthio-4-phenyl-3(1H)quinazoline

To a 10 mL pressure tube with a stirring bar was added 6-chloro-3,4-dihydro-4-ethyl-3-methyl-4-phenyl-2(1H)quinazoline (463 mg, 1.46 mmol), chloroform (2 mL), and iodomethane (2.0 mL, 32.13 mmol). The tube was sealed and the mixture was stirred at 20°C for 3 hours. The mixture was diluted with EtOAc and the solution was washed with aqueous $NaHCO_3$, and brine. Drying ($MgSO_4$), filtration and removal of the solvent in vacuo gave 481 mg of 6-chloro-3,4-dihydro-4-ethyl-3-methyl-2-methylthio-4-phenyl-3(1H)-quinazoline as a colorless foam. This material was used in Step B without further purification.

B. 6-Chloro-2-cyanoimino-3,4-dihydro-4-ethyl-3-methyl-4-phenyl-2(1H)quinazoline

To a 50 mL round bottom flask with a stirring bar and an argon inlet was added 6-chloro-3,4-dihydro-4-ethyl-3-methyl-2-methylthio-4-phenyl-3(1H)quinazoline (248 mg, 0.75 mmol), lead cyanamide (370 mg, 1.50 mmol), cyanamide (400 mg, 9.51 mmol) and dry DMF (5.00 mL). This well stirred mixture was heated at 100°C for 5 days. The cooled reaction mixture was filtered and the filtrate was diluted with EtOAc. This solution was washed with water and brine. Drying ($Na_2SO_4$), filtration, chromatography on 20 g of silica gel using 33% EtOAc in hexanes as eluant, and recrystallization from boiling EtOAc-hexanes gave 6-chloro-2-cyanoimino-3,4-dihydro-4-ethyl-3-methyl-4-phenyl-2(1H)quinazoline as a white solid (51 mg), MP: 248-249°C.
$^1$H-NMR ($CDCl_3$): δ 0.88 (t, J=7.1 Hz, 3H), 2.25 (m, 1H), 2.45 (m, 1H), 2.67 (s, 3H), 6.60 (d, J=2.0 Hz, 1H), 7.08 (d, J=2.0 Hz, 1H), 7.10 (s, 1H), 7.38 (m, 5H), 9.2 (br s, 1H).

EXAMPLE 8

Grignard Additions to Quinazolines

6 Chloro-4-cyclopropyl-3-cyclopropylmethyl-4-ethyl-3,4-dihydroquinazolin-2(1H)-one

A. (2-Amino-4-chlorophenyl)cyclopropyl ketone

A solution of cyclopropylmagnesium bromide, prepared from 2.4 g (0.099 g atom) of magnesium turnings and 13.0 g (0.107 mol) of cyclopropyl bromide in 100 mL of THF, was stirred at 38°C as a solution of 5-chloroanthranilonitrile (3.65 g, 0.0239 mol) in 40 mL THF was added over 20 min. Stirring was continued at 40°C for 2h, following which the reaction mixture was cooled in an ice bath and 50 mL of saturated $NH_4Cl$ was added, followed by 100 mL of 2N HCl. The cooling bath was removed and stirring was continued at room temperature for 2h. The mixture was then brought to pH 9 by addition of 20% NaOH and was extracted 3 times with ether. The combined organic phases were washed with brine and dried over $MgSO_4$. Following removal of the solvents the oily residue was flash chromatographed, eluting with 15% EtOAc in hexane, to provide 3.0 g (64%) of the title compound as a yellow solid, MP 66-68°C.
$^1$H NMR ($CDCl_3$): δ 1.02 (m, 2H), 1.18 (m, 2H), 2.57 (m, 1H), 6.17 (s, 1H), 6.61 (d, J=8.5 Hz, 1H), 7.22 (dd, J=2, 8.5 Hz, 1H), 7.92 (d, J=2 Hz, 1H).

B. 6-chloro-4-cyclopropylquinazolin-2(1H)-one.

To a stirred suspension of 150 mg (0.767 mmol) of the product from Step A in 3 mL of glacial acetic acid at 0° was added a solution of 75 mg (0.922 mmol) of potassium cyanate in 0.3 mL of $H_5O$ in one portion. After stirring for 1 hour at 0°-5°, the reaction mixture was allowed to warm to room temperature over a 1 hour period. The reaction mixture was partitioned between EtOAc and $H_2O$, the organic layer washed with $H_2O$, filtered, washed with brine, dried over $Na_2SO_4$, and concentrated to afford 120 mg of a light yellow solid. This material was chromatographed on silica gel to give 122 mg of the title compound as a solid:
$^1$H-NMR ($CDCl_3$): δ 1.27 (m, 2H), 1.57 (m, 2H), 2.55 (m, 1H), 7.48 (d, J=8 Hz, 1H), 7.61 (dd, J=8, 2 Hz, 1H), 8.10 (d, J=2 Hz, 1H). FAB MS M+H=221, mp=215-217°C.

C. 6-chloro-4-cyclopropyl-1-(4-methoxybenzyl) quinazolin-2(1H)-one

To a stirred solution of 75 mg (0.338 mmol) of the product from Step B in 6 mL of dry DMF was added 17 mg (0.423 mmol) of sodium hydride (60% in mineral oil) in one portion. After 20 minutes when gas evolution ceased, 50 μL (0.372 mmol) of 4-methoxybenzylchloride was added in one portion. The reaction solution was stirred at room temperature for 2.5 hours, then heated to 80° under Ar for 4 hours, and allowed to stir at room temperature for 2.5 days. The reaction mixture was concentrated at reduced pressure and the residue partitioned between EtOAc and $H_2O$. The organic layer was washed with water, brine, dried over $Na_2SO_4$, and concentrated to give a residue which was chromatographed on silica gel using 1:1 EtOAc-hexane to give 73 mg of the title compound. An analytical sample was obtained by crystallization from EtOAc-hexane:
$^1$H-NMR ($CDCl_3$): δ 1.25 (m, 2H), 1.56 (m, 2H), 2.52 (m, 1H), 3.76 (s, 3H), 5.40 (s, 2H), 6.83 (d, J=8.7 Hz, 2H), 7.18 (d, J=8.7 Hz, 2H), 7.21 (d, J=9.2 Hz, 1H), 7.53 (dd, J=2.3, 9.2 Hz, 1H), 8.1 (d,J=2.3Hz, 1H). FAB MS M+H=341, mp 211-213°C.

D. 6-chloro-4-cyclopropyl-3,4-dihydro-1-(4-methoxybenzyl)-4-propylquinazolin-2(1H)-one

To a 50°C solution of 400 mg (1.17 mmol) of the product from step C in 20 mL of dry THF under $N_2$ was added 2.35 mL (4.69 mmol) of n-propylmagnesium chloride (2.0 M in ether) dropwise over a 5 minute period. After stirring for 3 hours at 50°, the reaction mixture was heated to reflux for 1 hour, cooled to room temperature and 0.15 mL (1.17 mmol) of trimethylsilylchloride added. The reaction mixture was reheated to reflux for 30 minutes and allowed to stand at room temperature. The reaction mixture diluted with water, extracted with EtOAc and the organic layer washed with brine, dried over $Na_2SO_4$, and the solvents stripped under reduced pressure to give 800 mg of a residue which was chromatographed on silica gel using 1:2 EtOAc-hexanes to give 147 mg of the title compound as a colorless solid:
$^1$H-NMR ($CDCl_3$): δ 0.15-0.6 (m, 4H), 0.89 (t, J=7 Hz, 3H), 1.1-1.22 (m, 1H), 1.4-1.65 (m, 2H), 1.80-1.92 (m, 1H), 3.77 (s, 3H), 4.95-5.12 (m, 2H), 5.32 (s, 1H), 6.69 (d, J=9 Hz, 1H), 6.83 (d, J=8 Hz, 2H), 7.05 (dd, J=9,2 Hz, 1H), 7.14 (d, J=2 Hz, 1H), 7.17 (d, J=8 Hz, 2H).

FAB/MS M+H=385.

E. 6-chloro-4-cyclopropyl-3-cyclopropylmethyl-3,4-dihydro-1-(4-methoxybenzyl)-4-propylquinazolin-2-(1H)-one

To a stirred solution of 147 mg (0.382 mmol) of the product from step D in 5 mL of dry DMF under $N_2$ was added 20 mg (0.496 mmol) of sodium hydride (60% in mineral oil) in one portion. After stirring for 0.5 hour, the solution was treated with 56 μL (0.573 mmol) of cyclopropylmethylbromide over a 4 minute period and allowed to stir at room temperature for 54 hours. The mixture was warmed to 50° for 3 hours, and an additional 10 mg NaH and 56 μL of cyclopropylmethylbromide were added. After 2 hours, an additional 10 mg of NaH and 111 μL of cyclopropylmethylbromide were added and the mixture stirred overnight at 50°. After cooling, the reaction mixture was partitioned between EtOAc and $H_2O$. The organic layer was washed with brine, dried over $Na_2SO_4$, and the solvents stripped under reduced pressure to give 250 mg of a residue which was chromatographed on silica gel using 1:4 EtOAc-hexane. The clean fractions were combined to afford 84 mg (50%) of the title compound as a colorless solid:
$^1$H-NMR ($CDCl_3$): δ 0.2-0.65 (m, 8H), 0.87 (t, J=7 Hz, 3H), 0.97-1.45 (m, 5H), 1.60-1.90 (m, 3H), 3.43-3.57 (m, 2H), 3.77 (s, 3H), 5.06 (br s, 2H), 6.65 (d, J=9 Hz, 1H), 6.83 (d, J=8 Hz, 2H), 7.03 (dd, J=9, 2 Hz, 1H), 7.17 (d, J=8 Hz, 2H), 7.19 (d, J=2 Hz, 1H).

F. 6-chloro-4-cyclopropyl-3-cyclopropylmethyl-3,4-dihydro-4-propylquinazolin-2(1H)-one

To a stirred solution of 77 mg (0.175 mmol) of the product from Step E in 0.75 mL of $CH_2Cl_2$ at 0° was added 375 μL of trifluoroacetic acid. After 1 hour at 0°, the reaction mixture was concentrated under reduced pressure and the residue partitioned between water and ethyl acetate. The organic layer was washed with sat. $NaHCO_3$, dried over $Na_2SO_4$ and the solvents stripped under reduced pressure to give 70 mg of a residue which was combined with 6 mg of crude product from a previous reaction. The material was chromatographed on $SiO_2$ using 1:2 EtOAc-hexane to give 50 mg of a solid which was triturated with hexane and dried at reduced pressure to afford the title compound as a colorless solid: mp 197-198°.
$^1$H-NMR ($CDCl_3$): δ 0.3-0.65 (m, 8H), 0.865 (t, J=6.8 Hz, 3H), 1.0-1.5 (m, 5H), 1.50-1.90 (m, 5H), 3.4-3.85 (m, 4H), 6.57 (br d, J=8 Hz, 1H), 7.12 (br d, J=8 Hz, 1h), 7.15 (d, J=1.2 Hz, 1H), 7.55 (br s, 1H).
FAB/MS M+H=319.

| | |
|---|---|
| Anal. Calcd for $C_{18}H_{23}ClN_2O$ | C, 67.80; H, 7.27; N, 8.79 |
| Found | C, 67.79; H, 7.21; N, 8.86 |

6-chloro-4,4-diethyl-3,4-dihydro-3-methylquinazolin-2(1H)-one

In the manner outlined above the 4,4-diethyl compound was prepared:
$^1$H-NMR ($CDCl_3$): δ 0.74 (t, J=7.3 Hz, 6H), 1.62-1.76 (m, 2H), 1.95-2.10 (m, 2H), 2.93 (s, 3H), 6.57 (d, J=8.5 Hz, 1H), 6.99 (d, J=2.2 Hz, 1H), 7.09 (dd, J=2.2, 8.5 Hz, 1H), 7.65 (br s, 1H).
FAB/MS M+H=253, mp 196-198°C.

| | |
|---|---|
| Anal. Calcd for $C_{13}H_{17}ClN_2O$ | C, 61.78; H, 6.78; N, 11.08 |
| Found | C, 61.84; H, 6.47; N, 10.94 |

EXAMPLE 9

Friedel-Crafts Reactions with Quinazolines

6-Acetyl-4-ethyl-3,4-dihydro-3-methyl-4-phenylquinazolin-2(1H)-one

To a stirred suspension of 200 mg (0.75 mmol) of 4-ethyl-3,4-dihydro-3-methyl-4-phenylquinazolin-1H-2-one (obtained using methods of Example 1) in 3 mL of methylene chloride at 0° under Ar was added 156 μL (2.2 mmol) of acetyl chloride, followed by 293 mg (2.2 mmol) of aluminum chloride. After stirring at 0° for 1h, the reaction mixture was stirred at room temperature for 3.5 h and then poured onto crushed ice. Once the ice had melted, the mixture was extracted with two portions of ethyl acetate. The combined organic layers were washed with water, 10% $Na_2CO_3$, brine, dried over $Na_2SO_4$ and the solvents removed to give a residue which contained 1:1 starting quinazolinone and the title compound by NMR analysis. An analytical sample was obtained by chromatography on silica gel using 98:2 chloroform-methanol followed by crystallization from ethyl acetate-hexanes.

NMR ($CDCl_3$): δ 0.79 (t, J=7 Hz, 3H), 2.28-2.38 (m, 1H), 2.39 (s, 3H), 2.45-2.55 (m, 1H), 2.68 (s, 3H), 6.71 (d, J=8 Hz, 1H), 7.25-7.30 (m, 2H), 7.38 (t, J=8 Hz, 2H), 7.43 (d, J=8 Hz, 2H), 7.69 (dd, J=8,2 Hz, 1H), 7.69 (br s, 1H), FAB MS M+H 309.

EXAMPLE 10

6-Chloro-3,4-dihydro-4-ethynyl-3-methyl-4-phenyl-2(1H)quinazolinone

Step A: 5-chloro-2-(1-imidazolecarbonylamino)benzophenone

5-Chloro-2-aminobenzophenone (23.2 g, 0.10 mmol) and carbonyldiimidazole (17.6 g, 0.11 mmol) were combined in 100 mL $CH_2Cl_2$ and the mixture refluxed under argon for 4 hours. The initial yellow solution deposited a thick, white precipitate. The mixture was cooled and the solid, 5-chloro-2-(1-imidazolecarbonylamino)benzophenone, which was collected and washed with $CH_2Cl_2$ by suction, afforded 23.2 g (71%), MP: 185-187°C. NMR ($CDCl_3$ + 1-2 drops DMSO-$d_6$): δ 2.87 (broad s, 1H), 6.71 (d, J=2.44 Hz, 1H), 7.01 (d, J=1.47 Hz, 1H), 7.05 (d, J=1.3 Hz, 1H), 7.1 (dd, J=2.58, 8.49 Hz, 1H), 7.19 (complex, 2H), 7.44 (complex, 5H).

Step B: 6-chloro-4-phenyl-4-hydroxy-3-methyl-3,4-dihydroquinazoline-2(1H)one

To a suspension of the imidazolylcarbonyl intermediate (17.1 g, 52.5 mmol) in 200 ml tetrahydrofuran, excess methyl amine was added over 5 hours via gas sparge tube. The reaction mixture was stirred overnight under argon. The resulting clear solution was evaporated in vacuo, dissolved in ethyl acetate, washed with 10% aqueous citric acid, water, brine, dried over $MgSO_4$, filtered, concentrated, and was recrystallized from EtOAc-Hexane (1:5) to afford the title compound as a white solid. (9.90 g, 69%).

Step C: 6-chloro-3-methyl-4-phenyl-2(3H)-quinazoline

The hydroxy intermediate (9.90 g, 36.3 mmol) was suspended in toluene (350 mL), and the mixture was refluxed for 17 hours under argon to remove the water using a Dean Stark trap. The resulting yellow slurry was cooled to 0°C, filtered to afford crystalline tetraene which was dried in vacuo (60°C, 0.05 torr) to vie the title compound, a bright yellow solid. (6.60 g, 67%). FAB/MS; M+H=271 m/e.

Step D: 6-Chloro-3,4-dihydro-4-ethynyl-3-methyl-4-phenyl-2(1H)-quinazolinone

6-chloro-3-methyl-4-phenyl-2(3H)-quinazolinone (6.0 g, 22.2 mmol) was suspended in dry THF (200 ml) to which sodium acetylide (17.74 g, 66.5 mmol, 18% slurry in xylene) was added via syringe at -25°C. Upon warming to room temperature over 3 hours the yellow color had discharged. The reaction mixture was poured into 10% aqueous citric acid and extracted with ethyl acetate, washed with water, brine, dried ($MgSO_4$), filtered and concentrated in vacuo. The crude product was chromatographed on silica gel using 20% EtOAc-hexanes and the pure fractions were combined and recrystallized from boiling EtOAC-hexanes (1:8) to afford the title compound, a white solid (2.24 g, 40%) after drying at 40°C, 0.05 torr. An additional 400 mg in mother liquors were recovered.
MP: 206-207°C, $^1$H-NMR ($CDCl_3$): δ 2.91 (s, 3H), 2.99 (s, 1H), 6.78 (d, J=8.54 Hz, 1H), 6.91 (d, J=2.2 Hz, 1H), 7.135 (dd, J=1.95 Hz, 2.44 Hz, 1H), 7.42 (m, 3H), 7.63 (m, 2H), 8.93 (broad s, 1H). FAB/MS; M+H=297 m/e.

EXAMPLE 11

6-Chloro-3,4-dihydro-4-ethynyl-3-isopropyl-4-phenyl-2(1H)quinazolinone

Step A:

5-chloro-2-(1-imidazolecarbonylamino)benzophenone was prepared in the exact manner as outlined in Example 10, Step A, from 5-chloro-2-aminobenzophenone and carbonyldiimidazole in $CH_2Cl_2$.

Step B: 6-chloro-4-phenyl-4-hydroxy-3-isopropyl-3,4-dihydroquinazoline-2(1H)one

To a suspension of the imidazolylcarbonyl intermediate (5 g, 15.4 mmol) in 125 ml tetrahydrofuran was

added excess (25 mL) isopropylamine and the reaction mixture was heated to 40°C and stirred under argon. The resulting clear solution was evaporated in vacuo, dissolved in ethyl acetate, washed with 10% aqueous citric acid, water, brine, dried over $MgSO_4$, filtered, concentrated, and chromatographed using EtOAc-Hexane (1:1) to afford the title compound as a yellow foam (4.24 g, 86%).

Step C: 6-chloro-3-isopropyl-4-phenyl-2 (3H)-quinazolinone

The hydroxy intermediate (4.86 g, 15.1 mmol) was dissolved in toluene (250 mL), and the mixture was refluxed for 17 hours under argon to remove the water using a Dean Stark trap. The resulting yellow solution was concentrated under vacuum to afford crystalline tetraene which was triturated with hexane, filtered and dried in vacuo (60°C, 0.05 toor) to affor the bright yellow title compound (3.75 g, 83%), FAB/MS; M+H=299 m/e.
NMR (DMSO): δ 1.53 (d; J=7 Hz, 6H), 4.28 (m, 1H), 6.64 (d; J=2.3 Hz, 1H), 7.33 (m, 2H), 7.70 (complex, 5H).

Step D: 6-chloro-3,4-dihydro-4-ethynyl-3-isopropyl-4-phenyl-2(1H)quinazolinone

6-chloro-3-isopropyl-4-phenyl-2(3H)-quinazolinone (1.50 g, 5.02 mmol) was suspended in dry THF to which (30 mL) sodium acetylide (4.02 g, 15.1 mmol, 18% slurry in xylene) was added via syringe at -25°C. After warming to room temperature over 3 hours, the reaction was poured into 10% aqueous citric acid and ethyl acetate, washed with water, brine, dried ($Na_2SO_4$), filtered and concentrated in vacuo. The crude product was recrystallized from boiling EtOAc-hexanes to afford the title compound, a white solid (545 mg) after drying at 40°C, 0.05 torr. MP: 229-230°C.
$^1$H-NMR ($CDCl_3$): δ 1.14 (d, J=6.8 Hz, 3H), 1.60 (d, J=6.4 Hz, 3H), 3.01 (s, 1H), 3.56 (q, J=6.6 Hz, 1H), 6.75 (d, J=8.6 Hz, 1H), 6.85 (d, J=0.01 Hz, 1H), 7.08 (dd, J=0.7, 2.3 Hz, 1H), 7.35-7.41 (m, 3H), 7.68 (dd, 0.95, 2.25 Hz, 2H), 9.56 (s, 1H).

6-Chloro-3,4-dihydro-4-ethyl-3-isopropyl-4-phenyl-2(1H)quinazolinone

In the exact manner as outlined above for the preparation (Steps A, B, C) and reaction (Step D) of the tetraene 6-chloro-3-isopropyl-4-phenyl-2(3H)-quinazolinone, a solution of ethylmagnesium bromide (3M in ether, threefold excess) was added via syringe to a THF suspension of the tetraene 6-chloro-3-isopropyl-4-phenyl-2(3H)-quinazolinone at -35°C and stirred for 1 hour. The reaction was poured into 2N HCl and ethyl acetate, washed with water, brine, dried ($Na_2SO_4$), filtered and concentrated in vacuo. The crude product was chromatographed on silica gel using 5%i-PrOH-$CHCl_3$ and recrystallized from boiling EtOAc-hexanes to afford the title compound, a white solid (183 mg) after drying at 40°C, 0.05 torr.
MP: 235-237°C. $^1$H-NMR ($CDCl_3$): δ 0.94 (t, J=7.3 Hz, 3H), 1.09 (d, J=6.7 Hz, 3H), 1.48 (d, J=6.6Hz, 1H), 2.19 (m, 1H), 3.05 (q, J=6.8Hz, 1H), 6.35 (d, J=2.4 Hz, 1H), 6.62 (d, J=8.2 Hz, 1H), 7.03 (dd, J=2.3, 8.5 Hz, 1H), 7.28-7.48 (m, 5H), 8.16 (s, 1H).

EXAMPLE 12

Preparation of 6-Chloro-3,4-dihydro-3-ethyl-4-ethynyl-4-phenyl-2(1H)quinazolinone

Step A: 6-Chloro-3-ethyl-4-phenyl-2(3H)quinazolinone

A mixture of 5.08 g (21.9 mmol) of 2-amino-5-chlorobenzophenone, 5.2 mL (65.7 mmol) of ethyl isocyanate, and 50 mg of Dabco in 100 mL of acetonitrile was stirred at reflux under argon for 18 hours, after which time the reaction mixture was charged with an additional 3 mL of ethyl isocyanate and 100 mg of Dabco. Reflux was continued for 7 hours. Concentration of the reaction mixture in vacuo left an oily solid which was triturated in a small volume of hot acetonitrile to afford 2.65 g of the title compound (3-ethyl tetraene) as a bright yellow solid:
$^1$H-NMR ($CDCl_3$): δ 1.30 (t, J=7 Hz, 3H), 4.10 (q, J=2, 7 Hz, 2H), 6.81 (d, J=2 Hz, 1H), 7.3-7.8 (m, 7H).

Step B: 6-Chloro-3,4-dihydro-3-ethyl-4-ethynyl-4-phenyl-2(1H)quinazolinone

In the manner described above for the reaction with 3-isopropyl tetraene (Step D, Example 11), sodium acetylide (1.2 g, 4.38 mmol, 18% slurry in xylene) was added to a solution of 6-chloro-3-ethyl-4-phenyl-2(3H)quinazolinone (0.50 g, 1.75 mmol) in 10 mL of THF. The crude product obtained from the ethyl acetate

extraction was chromatographed on silica gel, elutant 25-30% EtOAc in CHCl3, to afford 170 mg of the pure title compound as a white solid, mp 185-187°C.
$^1$H-NMR ($CDCl_3$): δ 1.12 (t, J=7 Hz, 3H), 2.96 (s, 1H), 3.33 (m, 1H), 3.48 (m, 1H), 6.70 (d, J=8.5 Hz, 1H), 7.00 (d, J=2 Hz, 1H), 7.10 (dd, J=2, 8.5 Hz, 1H), 7.29-7.45 (m, 3H), 7.59-7.61 (m, 2H), 8.40 (br s, 1H).

EXAMPLE 13

Preparation of 6-chloro-3,4-dihydro-3-methyl-4-cyclopropyl-4-propyl-2(1H)-quinazolinone

By the methods of Example 8, the title compound was obtained.
mp 197-200°C
$^1$H-NMR($CDCl_3$): δ 0.20-0.29 (m,1H), δ 0.30-0.38 (m, 1H), 0.46-0.61 (m,2H), 0.90 (t, J=7.1Hz, 3H), 1.02-1.30 (m, 4H), 1.68-1.98 (m, 2H), 3.10 (s, 3H), 6.58 (br d, J=8Hz, 1H), 7.08 (d, J=1.0Hz, 1H), 7.12 (br d J=8Hz, 1H), 7.43 (br s, 1H).

Anal. Calcd for $C_{15}H_{19}ClN_2O$:

| | C | H | N |
|---|---|---|---|
| Calcd | 64.62 | 6.87 | 10.05 |
| Found | 64.89 | 6.82 | 9.71 |

EXAMPLE 14

(+/-) 6-Chloro-4-cyclopropyl-3-methyl-4-(2-propenyl)-3,4 4-dihydroquinazolin-2(1H)-one

Step A: 6-chloro-4-cyclopropyl-3,4-dihydro-1-(4-methyoxybenzyl)-4-(2-propenyl)quinazolin-2(1H)-one

A solution of 2.0 g (5.87 mmol) of 6-chloro-4-cyclopropyl-1-(4-methyoxybenzyl)-quinazolin-2(1H)-one in 25 mL of dry THF under Ar was added to 10 mL of a solution of allylmagnesium bromide (1.0 M in ether) dropwise at 0°C. An additional 8 mL of allylmagnesium bromide solution was added to the reaction, and the solution stirred overnight at RT. The reaction was quenched by pouring into ice-cold 1M citric acid. The resulting mixture was extracted with two portions of $CHCl_3$ the organic layers washed with water, dried over $MgSO_4$, treated with activated carbon and solvents removed to give 1.8 g of a dark yellow oil which was chromatographed on 250 g of fine $SiO_2$ using 95:5 $CHCl_3$ - $CH_3CN$ to afford 730 mg of a yellow oil:
$^1$H NMR ($CDCl_3$): δ 0.23-0.65(m, 4H), 1.21-1.32(m, 1H), 2.47-2.66(m, 2H), 3.77(s, 3H), 4.84(s, 1H), 5.02(s, 2H), 5.05-5.20(m, 2H), 5.68-5.83(m, 1H), 6.69(d, J=8.8 Hz, 1H), 6.84 (d, J=8.6 Hz, 2H), 7.057(dd, J=8.8, 2.4 Hz, 1H), 7.165(d, J=8.6 Hz, 2H), 7.20(d, J=2.2 Hz, 1H).

Step B: 6-chloro-4-cyclopropyl-3-methyl-3,4-dihydro-1-(4-methyoxybenzyl)-4-(2-propenyl)quinazolin-2(1H)-one

To a 10 mL oven dried round botomed flask with a stirring bar and an argon inlet was added sodium hydride-oil suspension (12 mg, of a 60% suspension, 0.30 mmol). The oil was removed with two hexane washings and the oil free sodium hydride was suspended in dry DMF (0.5 mL). To this suspension was added the product from step A (0.07g, 0.182 mmol). This mixture was stirred until hydrogen evolution had ceased and methyl iodide (0.023 mL, 0.366 mmol) was added with a syringe. This mixture was stirred for 18h at room temperature. The mixture was diluted with EtOAc and this solution was washed with water, 10% aqueous citric acid and brine. Drying ($MgSO_4$), filtration and removal of the solvent in vacuo gave 71 mg of the methylation product which was used in step C without further purification.

Step C: (+/-) 6-chloro-4-cyclopropyl-3-methyl-4-(2-propenyl)-3, 4-dihydroquinazolin-2(1H)-one

The product from step B (0.071g, 0.179 mmol) was dissolved in 2 mL of a 1:1 solution of trifluoroacetic acid and methylene chloride. This mixture was stirred at room temperature for 2h. The solvents were removed in vacuo and the residue was dissolved in EtOAc. This solution was washed with saturated aqueous $NaHCO_3$ solution and brine. Drying ($MgSO_4$), filtration, removal of the solvent in vacuo, and chromatography on silica gel using 40% EtOAc in hexane as eluant gave 28 mg of the title compound as white crystals. An analytical sample was prepared by recrystallization from EtOAc-hexane.
$^1$H NMR ($CDCl_3$): δ 0.23-0.32 (m, 1 H); 0.32-0.43 (m, 1 H); 0.50-0.66 (m, 2 H); 1.23-1.36 (m, 1 H); 2.57 (dd,

J=5.9,15.6, 1 H); 2.79 (dd, J=7.5,15.6, 1 H); 3.12 (s, 3 H); 5.02-5.13 (m, 2 H); 5.47-5.62 (m, 1 H); 6.62 (d, J=9.3, 1 H); 7.11-7.14 (m, 2 H); 8.04 (br s, 1 H).
mp:182-184°C

EXAMPLE 15

(+/-) 6-Chloro-4-cyclopropyl-4-cyclopropylmethyl-3-methyl-3,4-dihydroquinazolin-2(1H)-one

Step A: 6-Chloro-4-cyclopropyl-4-cyclopropylmethyl-3,4-4-dihydro-1-(4-methoxybenzyl)quinazolin-2(1H)-one

6-Chloro-4-cyclopropyl-3,4-dihydro-1-(4-methoxybenzyl)-4-(2-propenyl)quinazolin-2(1H)-one (96 mg, 0.251 mmol) was dissolved in 5 ml ethyl ether and 2 ml ether solution of diazomethane was added at 0°C. Palladium acetate (1 mg) was added and stirred vigorously until gas evolution ceases. Diluted with 40 ml EtOAc and washed with water, brine, dried over $MgSO_4$ and solvent removed in vacuo to give an oil(100 mg, 99%).

Step B: 6-Chloro-4-cyclopropyl-4-cyclopropylmethyl-3,4-dihydro-1-(4-methoxybenzyl)-3-methylquinazolin-2(1H)-one

In a manner according to Example 14, step B, the title compound was prepared from 100 mg (0.251 mmol) of the product from step A to give 110 mg of an oil.

Step C: (+/-) 6-Chloro-4-cyclopropyl-4-cyclopropylmethyl-3-methyl-3,4-dihydroquinazolin-2(1H)-one

In a manner according to Example 14, step C, the title compound was prepared from 110 mg (0.251 mmol) of the product from step B to give 32 mg (44%) of a solid:
$^1$H NMR ($CDCl_3$): δ -0.24-(-)0.18 (m, 1H); 0.01-0.09 (m, 1H); 0.24-0.30 (m, 1H); 0.37-0.54 (m, 4H); 0.62-0.73 (m, 2H); 1.20 -1.29 (m, 1H); 1.49 (dd, J=6.2,14.6, 1H); 1.82 (dd, J=6.0,14.7, 1H); 3.12 (s, 3H); 6.58 (d, J=8.4, 1H); 7.13 (dd, J=2.2,8.4, 1H); 7.24 (d, J=2.2, 1H); 7.44 (br s, 1H).
mp:155°C (dec)

EXAMPLE 16

(+/-) 9-Chloro-10b-cyclopropyl-1,2,3,10b-tetrahydropyrrolo[1,2-c]quinazolin-5(6H)-one

Step A: 9-Chloro-10b-cyclopropyl-6-(4-methoxybenzyl)-1,2,3,10b-tetrahydropyrrolo[1,2-c]quinazolin-5(6H)-one

In a manner according to Example 25, step E, the title compound was prepared as the product from 24 mg (0.060 mmol) 6-chloro-4-cyclopropyl-4-(3-hydroxypropyl)-1-(4-methoxybenzyl)-3,4-dihydroquinazolin-2(1H)-one to give 20 mg of an oil.

Step B: 9-Chloro-10b-cyclopropyl-1,2,3,10b-tetrahydropyrrolo[1-2-c]quinazolin-5(6H)-one

In a manner according to Example 14, step C, the title compound was prepared from 20 mg (0.052 mmol) of the product from above to give 7 mg (51%) of a solid:
$^1$H NMR ($CDCl_3$): δ -0.08-0.02 (m, 2H); 0.28-0.36 (m, 1H); 0.40-0.48 (m, 1H); 1.03-1.11 (m, 1H); 2.02-2.09 (m,1H); 2.13-2.30 (m, 2H); 2.46-2.52 (m, 1H); 3.64-3.70 (m, 2H); 6.76 (d, J=8.5, 1H); 6.97 (d, J=2.3, 1H); 7.15 (dd, J=2.3,8.5, 1H).
mp: 271-272°C.

EXAMPLE 17

(+/-) 10-Chloro-11b-cyclopropyl-1,11b-dihydro-2H, 6H-[1,3]oxazino[4,3-c]quinazolin-6(7H)-one

Step A: 6-Chloro-4-cyclopropyl-4-(2-hydroxyethyl)-3,4-dihydroquinazolin-2(1H)-one

In a manner according to Example 22, step A, the title compound was prepared from 6-chloro-4-cyclopropyl-4-(2-propenyl)-3,4-dihydroquinazolin-2(1H)-one (130 mg, 0.495 mmol) to give 87 mg of a solid.

Step B: 10-Chloro-11b-cyclopropyl-1,11b-dihydro-2H, 6H-[1,3]oxazino[4,3-c]quinazolin-6(7H)-one

In a manner according to Example 74, step C, the title compound was prepared from 6-chloro-4-cyclopropyl-4-(2-hydroxyethyl)-3,4-dihydroquinazolin-2(1H)-one (87 mg, 0.326 mmol) to give 34 mg (37%) of a colorless solid.
$^1$H NMR ($CDCl_3$): δ 0.01-0.14 (m, 2H); 0.43-0.57 (m, 2H); 1.65-1.76 (m, 1H); 2.15 (dt, J=2.2,13.2, 1H); 2.32 (td, J=6.1,12, 1H); 4.04-4.22 (m, 2H); 4.79 (d, J=10, 1H); 5.75 (d, J=10, 1H); 6.66 (d, J=8.4, 1H); 6.93 (d, J=2.2, 1H); 7.16 (dd, J=2.2,8.4, 1H).
mp: 264°C (dec)

EXAMPLE 18

(+/-) 6-Chloro-4-cyclopropyl-3-methyl-4-(2-thiopheneyl-3,4-dihydroquinazolin-2(1H)-one

Step A: 6-Chloro-4-cyclopropyl-4-(2-thiopheneyl)-3,4-dihydroquinazolin-2(1H)-one

In a manner according to Example 14, step A, the title compound was prepared from 6-chloro-4-cyclopropyl-1-(4-methoxybenzyl)quinazolin-2(1H)-one (150 mg, 0.440 mmol) and 3-thiopheneylmagnesium bromide to give 187 mg of a solid.

Step B: 6-Chloro-4-cyclopropyl-1-(4-methoxybenzyl)-3-methyl-4-(2-thiopheneyl)-3,4-dihydroquinazolin-2(1H)-one

In a manner according to Example 14, step B, the title compound was prepared from 187 mg (0.440 mmol) of the product from step A to give 80 mg of an oil.

Step C: (+/-) 6-Chloro-4-cyclopropyl-3-methyl-4-(2-thiopheneyl)-3,4-dihydroquinazolin-2(1H)-one

Ceric ammonium nitrate (400 mg, 0.729 mmol) was dissolved in 1.6 ml water and added to the product from step B. (80 mg, 0.182 mmol) dissolved in 8 ml acetonitrile and stirred overnight. Solvent was removed, the residue taken up in EtOAc and washed with 5% $NaHCO_3$, water, brine, dried over $MgSO_4$ , filtered, solvent removed in vacuo and chromatographed on silica gel using 30% EtOAc in hexanes to give an oil which was triturated with ether/hexanes to give a solid(5 mg, 9%):
$^1$H NMR ($CDCl_3$): δ 0.08-0.19 (m,1H); 0.22-0.32 (m,1H); 0.50-0.62 (m,1H); 0.67-0.76 (m,1H); 1.68-1.78 (m,1H); 2.91 (s, 3H); 6.52 (d, J=2.2,1H); 6.65 (d, J=8.5,1H); 7.02 (dd, J=3.5, 5,1H); 7.12 (dd, J=2.2,8.5,1H); 7.28 (d, J=3.5,1H); 7.38 (d, J=5,1H); 7.81 (br s,1H).
mp: 216°C (dec)

EXAMPLE 19

(+/-) 6-Chloro-4-cyclopropyl-4-(2,2-dimethylpropyl)-3-methyl-3,4-dihydroquinazolin-2(1H)-one

Step A: 6-Chloro-4-cyclopropyl-4-(2,2-dimethylpropyl)-3,4-dihydro-1-(4-methoxybenzyl)quinazolin-2(1H)-one

In a manner according to Example 14, step A, the title compound was prepared from 6-chloro-4-cyclopropyl-1-(4-methoxybenzyl)quinazolin-2(1H)-one (200 mg, 0.587 mmol) and 2,2-dimethylpropylmagnesium bromide to give 66 mg of a solid.

Step B: 6-Chloro-4-cyclopropyl-4-(2,2-dimethylpropyl)-3, 4-dihydro-1-(4-methoxybenzyl)-3-methylquinazolin-2(1H)-one

In a manner according to Example 14, step B, the title compound was prepared from 66 mg (0.160 mmol) of the product from step A to give 85 mg of an oil.

Step C: (+/-) 6-Chloro-4-cyclopropyl-4-(2,2-dimethylpropyl)-3-methyl-3,4-dihydroquinazolin-2(1H)-one

In a manner according to Example 14, step C, the title compound was prepared from 85 mg (0.160 mmol) of the product from step B to give 12 mg (24%) of a colorless solid:
$^1$H NMR ($CDCl_3$): δ 0.33-0.42 (m,1H); 0.54-0.70 (m, 3H); 0.76 (s, 3H),.1.18-1.30 (m, 1H); 1.59 (d, J=15, 1H); 1.96 (d, J=15, 1H); 3.06 (s, 3H); 6.54 (d, J=8.5, 1H); 7.08 (dd, J=2.2,8.5, 1H); 7.13 (d, J=2.2, 1H); 7.75 (br s, 1H).
mp: 254°C

EXAMPLE 20

(+/-) 6-Chloro-4-cyclopropyl-4-(3-hydroxypropyl)-3-methyl-3,4-dihydroquinazolin-2(1H)-one

Borane/THF (0.479ml, 1.0M solution in THF) was added to (+/-) 6-Chloro-4-cyclopropyl-3-methyl-4(2-propenyl)-3,4-dihydroquinazolin-2(1H)-one (50 mg) dissolved in 2 ml dry THF at 0°C and stirred for 30 min. Ice bath was removed and reaction stirred overnight. Cooled to 0°C and 3 ml 2N NaOH, then 1 ml 30% hydrogen peroxide was added. The bath was removed and stirred for 1 h. Ether was added and was washed with water and brine. The organics were dried over $MgSO_4$, filtered, and solvent removed in vacuo to give a solid which was prepped on HPLC using C-18 and eluting with acetonitrile/0.1% TFA in water to give a colorless solid (10 mg, 18%):
$^1$H NMR ($CDCl_3$): δ 0.19-0.30 (m, 1H); 0.32-0.43 (m, 1H); 0.46-0.74 (m 2H); 1.22-1.48 (m, 3H); 1.77-1.90 (m, 1H); 2.04-2.18 (m, 1H); 3.07 (s, 3H); 3.51-3.62 (m, 2H); 6.65 (d, J=8, 1H); 7.10-7.14 (m, 2H).
mp: 219-220°C

EXAMPLE 21

(+/-) 6-Chloro-4-cyclopropyl-4-(2-hydroxyethyl)-3-methyl-3,4-dihydroquinazolin-2(1H)-one

In a manner according to Example 14, step C, the title compound was prepared from 60 mg(0.150 mmol) 6-chloro-4-cyclopropyl-4-(2-hydroxyethyl)-1-(4-methoxybe nzyl)-3-methyl-3,4-dihydroquinazolin-2(1H)-one to give 14 mg (34%) of a solid:
$^1$H NMR ($CDCl_3$): δ 0.22-0.33 (m, 1H); 0.36-0.49 (m, 1H); 0.49-0.77 (m, 2H); 1.23-1.35 (m, 1H); 1.97-2.09 (m, 1H); 2.25-2.38 (m, 1H); 3.12 (s, 3H); 3.42-3.58 (m, 2H); 6.67 (d, J=8, 1H); 7.12-7.17 (m, 2H).
mp: 203-205°C

EXAMPLE 22

(+/-) 6-Chloro-4-cyclopropyl-4-(2-methoxyethyl)-3-methyl-3,4-dihydroquinazolin-2(1H)-one

Step A: 6-Chloro-4-cyclopropyl-4-(2-hydroxyethyl)-3-methyl-3,4-dihydroquinazolin-2(1H)-one

6-Chloro-4-cyclopropyl-1-(4-methoxybenzyl)-3-methyl-4-(2-propenyl)-3,4-dihydroquinazolin-2(1H)-one (73 0 mg, 1.84 mmol) was dissolved in 45 ml 4:1 dichloromethane/methanol, cooled to -78°C and treated with ozone for 10 min. System was purged with argon, then 210 mg (5.52 mmol) of sodium borohydride was added as a solid. The reaction was warmed to room temperature and small portions of sodium borohydride were added until gas evolution ceased. The solvent was removed, 1M citric acid was added, and was extracted with EtOAc. The organic layer was washed with water, brine, dried over $MgSO_4$ , filtered, and the solvent removed in vacuo to give 627 mg of a foam.

Step B: 6-Chloro-4-cyclopropyl-1-(4-methoxybenzyl)-4-(2-methoxyethyl)-3-methyl-3,4-dihydroquinazolin-2(1H)-one

In a manner according to Example 14, step B, the title compound was prepared from 745 mg (1.18 mmol) of the product from step A to give 491 mg of an oil.

Step C: (+/-) 6-Chloro-4-cyclopropyl-4-(2-methoxyethyl)-3-methyl-3,4-dihydroquinazolin-2(1H)-one

In a manner according to Example 14, step C, the title compound was prepared from 491 mg(1.18 mmol)

of the product from step B to give 225 mg (65%) of a colorless solid:
$^1$H NMR ($CDCl_3$): δ 0.21-0.33 (m, 1H); 0.35-0.45 (m, 1H); 0.47-0.66 (m, 2H); 1.23-1.34 (m, 1H); 2.02-2.14 (m, 1H); 2.24-2.37 (m, 1H); 3.13 (s, 3H); 3.24 (s, 3H); 3.13-3:32 (m, 2H); 6.62 (d, J=9, 1H); 7.12-7.15 (m, 2H); 7.93 (br s, 1H).
mp: 159-160°C

EXAMPLE 23

(+/-) 6-Chloro-4-cyclopropyl-4-(2-ethoxyethyl)-3-ethyl-3,4-dihydroquinazolin-2(1H)-one:

Step A: 6-Chloro-4-cyclopropyl-4-(2-ethoxyethyl)-3-ethyl-1-(4-methoxybenzyl)-3,4-dihydroquinazolin-2(1H)-one

In a manner according to Example 14, step B, the title compound was prepared from 6-chloro-4-cyclopropyl-4-(2-hydroxyethyl)-1-(4-methoxybenzyl)-3, 4-dihydroquinazolin-2(1H)-one (248 mg, 0.522 mmol) and ethyl iodide (1 ml) to give 100 mg of an oil.

Step B: 6-Chloro-4-cyclopropyl-4-(2-ethoxyethyl)-3-ethyl-3,4-dihydroquinazolin-2(1H)-one

In a manner according to Example 14, step C, the title compound was prepared from 6-chloro-4-cyclopropyl-4-(2-ethoxyethyl)-3-ethyl-1-(4-methoxybenzyl)-3, 4-dihydroquinazolin-2(1H)-one (100 mg, 0.226 mmol) to give 25 mg (34%) of a solid:
$^1$H NMR ($CDCl_3$): δ 0.43-0.52 (m, 1H); 0.55-0.77 (m, 3H); 1.11 (t, J=7, 3H); 1.31 (t, J=7, 3H); 1.84-1.96 (m, 1H); 2.04-2.18 (m, 1H); 3.17-3.26 (m, 1H); 3.33 (q, J=7, 2H); 3.50 (q, J=7, 2H); 3.59-3.72 (m, 1H); 6.67 (d, J=8.6, 1H); 7.15 (dd, J=2.2,8.5, 1H); 7.25 (d, J=2.2, 1H); 8.86 (br s, 1H).
mp: 137-138°C

EXAMPLE 24

(+/-) 6-Chloro-4-cyclopropyl-4-cyclopropylmethyl-3,4-4-dihydroquinazolin-2(1H)-one

Step A: 6-Chloro-4-cyclopropyl-4-(2-propenyl)-3,4-dihydroquinazolin-2(1H)-one

In a manner according to Example 14, step A, the title compound was prepared from 6-chloro-4-cyclopropylquinazolin-2(1H)-one (2.0 g, 9.06 mmol) to give 2.2 g of a yellow solid.

Step B: 6-Chloro-4-cyclopropyl-4-cyclopropylmethyl-3,4-dihydroquinazolin-2(1H)-one

In a manner according to Example 15, step A, the title compound was prepared from 300 mg (1.14 mmol) of the product from above to give 217 mg (69%) of a foam:
$^1$H NMR ($CDCl_3$): δ -0.08-0.02 (m, 1H); 0.07-0.17 (m, 1H); 0.28-0.76 (m, 7H); 1.23-1.33 (m,1H); 1.63 (dd, J=6.7, 14.2, 1H); 1.77 (dd, J=6.7,14.2, 1H); 5.16 (br s, 1H); 6.67 (d, J=8.5, 1H); 7.11 (dd, J=2.3,8.5, 1H); 7.19 (d, J=2.3, 1H); 8.62 (br s, 1H).
(amorphous solid)

EXAMPLE 25

(+/-) 6-Chloro-4-cyclopropyl-4-(3-fluoropropyl)-3,4-4-dihydroquinazolin-2(1H)-one

Step A: 6-Chloro-4-cyclopropyl-4-(3-hydroxypropyl)-1-(4-methoxybenzyl)-3,4-dihydroquinazolin-2(1H)-one

6-Chloro-4-cyclopropyl-1-(4-methoxybenzyl)-4-(2-propenyl)-3,4-dihydroquinazolin-2(1H)-one (1.0 g, 2.61 mmol) was dissolved in 100 ml dry THF and 20.9 ml (0.5M solution in THF) of 9-BBN was added dropwise. The mixture was stirred for 45 min, then cooled in an ice bath and 1.5 ml 2N NaOH and 1.5 ml 30% hydrogen peroxide were added. The bath was removed and stirred for 2 h. Water (30 ml) was added and the mixture was extracted with EtOAc. The organic layers were washed with water, brine, dried over $MgSO_4$, filtered, solvent removed in vacuo, and chromatographed on silica gel using 5% methanol/chloroform to give 905 mg of an oil.

Step B: 6-Chloro-4-cyclopropyl-4-(3-t-butyldimethylsilyloxypropyl)-1-(4-methoxybenzyl)-3,4-di-hydroquinazolin-2(1H)-one

6-Chloro-4-cyclopropyl-4-(3-hydroxypropyl)-1-(4-methoxybenzyl)-3,4-dihydroquinazolin-2(1H)-one (880 mg, 2.20 mmol), imidazole (598 mg, 8.78 mmol), and t-butyldimethylsilyl chloride (662 mg, 4.39 mmol) were dissolved in 6 ml dry DMF and stirred under argon for 3 days. The solvent was removed under vacuum, the residue was dissolved in EtOAc, washed with 1M citric acid, water, brine, dried over $MgSO_4$ and solvent removed in vacuo to give 819 mg of a foam.

Step C: 6-Chloro-4-cyclopropyl-4-(3-t-butyldimethylsilyloxypropyl)-1,3-di(4-methoxybenzyl)-3,4-dihydroquinazolin-2(1H)-one

6-Chloro-4-cyclopropyl-4-(3-t-butyldimethyl-silyloxypropyl)-1-(4-methoxybenzyl)-3,4-dihydroquinazolin-2 (1H)-one (400 mg, 0.776 mmol) was dissolved in 3 ml dry DMF, NaH (67 mg, 1.68 mmol, 60% slurry in oil) was added in one portion and stirred vigorously for 30 min. 4-Methoxybenzyl chloride (0.105 ml, 0.776 mmol) was added and the mixture was stirred for 2 days. Water was added and the mixture was extracted with EtOAc. The organic layers were washed with water, brine, dried over $MgSO_4$ , filtered, and the solvent removed in vacuo to give 493 mg of an oil.

Step D: 6-Chloro-4-cyclopropyl-4-(3-hydroxypropyl)-1,3-di(4-methoxybenzyl)-3,4-dihydroquinazolin-2(1H)-one

6-Chloro-4-cyclopropyl-4-(3-t-butyldimethylsilyloxypropyl)-1,3-di(4-methoxybenzyl)-3,4-dihydroquinazol in-2(1H)-one (493 mg, 0.776 mmol) was dissolved in 6 ml 1M tetrabutylammonium fluoride in THF and stirred for 30 min. EtOAc was added , then washed with water, brine, dried over $MgSO_4$ , filtered, the solvent removed in vacuo, and chromatographed on silica gel using 50% EtOAc/hexanes to give 243 mg of a clear oil.

Step E: 6-Chloro-4-cyclopropyl-4-(3-fluoropropyl)-1,3-di(4-methoxybenzyl)-3,4-dihydroquinazolin-2(1H)-one

6-Chloro-4-cyclopropyl-4-(3-hydroxypropyl)-1,3-di(4-methoxybenzyl)-3,4-dihydroquinazolin-2(1H)-one (243 mg, 0.466 mmol) was dissolved in 10 ml methylene chloride, cooled to 0°C, and 0.246 ml (1.87 mmol) diethylaminosulfur trifluoride was added. The bath was removed and stirring continued for 1 h. 10% Sodium carbonate was added and the mixture was extracted with chloroform. The organic layers were washed with water, brine, dried over $Na_2SO_4$, filtered, and the solvent removed in vacuo to give 523 mg of a foam.

Step F: 6-Chloro-4-cyclopropyl-4-(3-fluoropropyl)-3,4-dihydroquinazolin-2(1H)-one

In a manner according to Example 14, step C, the title compound was prepared from 240 mg (0.459 mmol) of 6-chloro-4-cyclopropyl-4-(3-fluoropropyl)-1,3-di(4-methoxybenzyl)-3,4-dihydroquinazolin-2(1H)-one to give 22 mg (17%) of a colorless solid:
$^1$H NMR ($CDCl_3$): δ 0.31-0.40 (m, 1H); 0.40-0.50 (m, 2H); 0.54-0.63 (m, 1H); 1.16-1.28 (m, 1H); 1.45-1.64 (m, 1H); 1.70-1.91 (m, 2H); 2.02-2.12 (m, 1H); 4.43 (dt, J=5.7,47, 2H); 5.10 (br s, 1H); 6.67 (dd, J=2.2,7, 1H); 7.13-7.15 (m, 2H); 8.13 (br s, 1H).
mp: 169-170°C

EXAMPLE 26

(+/-) 6-Chloro-3-cyano-4-cyclopropyl-4-cyclopropylmethyl-3,4-dihydroquinazolin-2(1H)-one

Step A: 6-Chloro-3-cyano-4-cyclopropyl-4-cyclopropylmethyl-3,4-dihydro-1-(4-methoxybenzyl)quinazolin-2(1H)-one

6-Chloro-4-cyclopropyl-4-cyclopropylmethyl-3, 4-dihydro-1-(4-methoxybenzyl)quinazolin-2(1H)-one (100 mg, 0.252 mmol) was dissolved in 2 ml dry DMF and treated with 30 mg NaH (60% in oil) under argon for 5 min. Phenyl cyanate (80 mg, 0.672 mmol, prepared according to Synthesis, pp.150-1, 1980) was added and the reaction was stirred for 4 h. Water was added and the mixture was extracted with EtOAc. The organic layers were washed with 2N NaOH, water, brine, dried over $MgSO_4$ , filtered, the solvent removed in vacuo, and chromatographed on silica gel using 5% methanol/chloroform to give 71 mg of a foam.

Step B: 6-Chloro-3-cyano-4-cyclopropyl-4-cyclopropylmethyl-3,4-dihydroquinazolin-2(1H)-one

In a manner according to Example 18, step C, the title compound was prepared from 6-chloro-3-cyano-4-cyclopropyl-4-cyclopropylmethyl-3,4-dihydro-1-(4-methoxybenzyl)quinazolin-2(1H)-one (35 mg, 0.083 mmol) to give 7 mg (28%) of a solid.
$^1$H NMR ($CDCl_3$): d -0.04-(-)0.01 (m, 1H); 0.34-0.39 (m, 1H); 0.42-0.51 (m, 4H); 0.67-0.73 (m, 1H); 0.73-0.79 (m, 2H); 1.44-1.48 (m, 1H); 1.68 (dd, J=7.6,15.1, 1H); 1.97-2.02 (m, 1H); 6.76 (d, J=8.5, 1H); 7.26 (dd, J=2.2,8.4, 1H); 7.29 (d, J=2.2, 1H); 8.21 (br s, 1H). mp: 172-173°C

EXAMPLE 27

3,4-dihydro-3-methyl-4,4-dicyclopropyl-6-chloroquinazolin-2(1H)one

Step A: 3,4 dihydro-1-(4-methoxybenzyl)-4,4 dicyclopropyl-6-chloroquinazolin-2(1H)one

To a stirred suspension of 500.00 mg(1.47 mmol) of 1-(4-methoxybenzyl)-4-cyclopropyl-6-chloroquinazolin-2(1H) one and 380.00 mg(1.47 mmol) of magnesium bromide etherate in 1 ml of ether/3 ml THF was added slowly dropwise a solution of 961 mg (6.62 mmol) of cyclopropyl magnesium bromide in 10 ml ether. After completion of the addition, the reaction was stirred at room temperature for one hour and gradually became a clear solution. The reaction was poured into 50 ml of cold 10% citric acid, and the mixture stirred vigorously for 10 min. The suspension was extracted with 25 ml of ether, and a thick precipitate resulted. The bilayer was filtered, and the filtrate reseparated. The aqueous layer was extracted with 2 x 25 ml of ether, and the combined ethereal extracts washed with 15 ml water and 15 ml brine. Drying(anh. mag. sulfate) and concentration gave 400 mg of crude product as an orange oil. The oil was purified via flash chromatography over silica gel with 12:1 chloroform/ethyl acetate to give 202 mg(36%) of title compound as a tan crystalline solid, mp: 169-171°C.
$^1$H NMR($CDCl_3$): δ 0.19 (m,4H), 0.55 (m,4H), 1.11 (m,2H), 3.78 (s,3H), 5.02(s,2H), 5.40 (s, 1H), 6.71 (d,J = 6Hz,1H), 6.84 (d,J = 5 Hz,2H), 7.05 (dd,1H), 7.10 (d, J = 5 Hz, 1H), 7.20 (s,1H).

Step B: 3,4-dihydro-4,4-dicyclopropyl-6-chloroquinazolin-2(1H) one

A solution of 70.00mg(0.18 mmol) of 3,4-di hydro-1-(4-methoxybenzyl)-4,4-dicyclopropyl-6-chloroquinazolin-2(1H) one in 1ml methylene chloride/1 ml trifluoroacetic acid was stirred at room temperature in a nitrogen atmosphere for 48 hrs. The reaction was concentrated in vacuo, and the residue dissolved in 8 ml of chloroform. The solution was washed with 2 x 5 ml of saturated sodium bicarbonate, 5 ml brine, and was dried over anh. sodium sulfate. Concentration in vacuo gave 75 mg of a tan foam. The crude foam was purified via flash chromatography over silica gel with 3:1 ethyl acetate/chloroform to give 30 mg(64%) of pure title compound as a white solid, mp: 237-239°C.
$^1$H NMR($CDCl_3$): δ 0.39(m,4H), 0.68(m,4H), 1.11(m,2H), 4.82(br s,1H), 6.64(d, J = 7Hz,1H), 7.15(dd,J = 2,7Hz,1H), 7.39(s,1H),7.61(br s,1H).

Step C: 3,4-dihydro-1-(4-methoxybenzyl)-3-methyl-4,4-dicyclopropyl-6-chloroquinazolin-2(1H) one

To a suspension of 8.89 mg(0.37 mmol) of NaH in 2 ml anh. DMF was added dropwise a solution of 125.00 mg(0.33 mmol) of 3,4-dihydro-1-(4-methoxybenzyl)-4,4-dicyclopropyl-6-chloroquinazolin-2(1H) one in 4 ml anh. DMF. The resulting solution was stirred at room temp. for 30 min., and was treated dropwise with 93.68 mg(0.66 mmol) of methyl iodide. The solution was stirred for 1 hr., and was poured into 25 ml cold 10% citric acid. The mixture was extracted with 2 x 20 ml ethyl acetate, and the combined extracts washed with 2 x 10 ml water, and 10 ml brine. The extracts were dried and conc. in vacuo to give a yellow oil. The crude oil was purified via flash chromatography over silica gel with 12:1 chloroform/ethyl acetate to give 88 mg(70%) title compound as a clear glassy solid, mp:129-131°C.
$^1$H NMR ($CDCl_3$) : δ 0.41 (m,4H), 0.59 (m,4H), 1.08(m,2H), 3.30 (s,3H), 3.76 (m,3H), 5.02 (s,2H), 6.69 (d,J = 6 Hz,1H), 7.04 (d, J = 5 Hz,1H), 7.08 (dd,J = 3,8 Hz,1H), 7.19 (d, J = 2 Hz,2H), 7.62 (s,1H).

Step D: 3,4-dihydro-3-methyl-4,4-dicyclopropyl-6-chloroquinazolin-2(1H)one

In a manner similar to Example 27, step B, from 85.00 mg(0.22 mmol) of 3,4-dihydro-1-(4-methoxybenzyl)-3-methyl-4,4-dicyclopropyl-6-chloroquinazolin-2(1H)one and 1 ml methylene chloride/1 ml TFA over 4 hrs, was obtained 40 mg(67%) of title compound as a white crystalline solid, mp: 243-245°C.

$^1$H NMR($CDCl_3$): 0.50(m,4H), 0.60(m,4H), 1.07(m,2H), 3.25(s,3H), 6.63(d, J = 5 Hz,1H), 7.15(dd,1H), 7.58(s,1H), 7.86(br s,1H).

EXAMPLE 28

3,4-dihydro-3-allyl-4-cyclopropyl-4-cyclopropylmethyl-6-chloroquinazolin-2(1H)one

Step A: 3,4-dihydro-1-(4-methoxybenzyl)-3-allyl-4-cyclopropyl-4-cyclopropymethyl-6-chloroquinazolin-2(1H)one

To a stirred suspension of 76.00 mg(3.17 mmol) of NaH in 8 ml of anhydrous DMF was added a solution of 840.00 mg(2.12 mmol) of 3,4-dihydro1-(4-methoxybenzyl)-4-cyclopropyl-4-cyclopropylmethyl-6-chloroquinazolin-2(1H)one in 8 ml anh. DMF. The resulting solution was stirred at room temp. for 30 min., and was treated dropwise with 712 mg(4.24 mmol) of allyl iodide. The resulting solution was stirred at room temperature for 18 hrs., and was poured into 40 ml of cold 10% citric acid. The resulting mixture was extracted with 2 x 30 ml of ethyl acetate. The combined extracts were washed with 2 x 20 ml of water and 20 mL brine, dried(anh. magnesium sulfate) and concentrated in vacuo to give an orange oil. The oil was purified via flash chromatography over silica gel with 3:1 hexanes/ethyl acetate to give 310 mg(33%) of title compound as a clear oil.
$^1$H NMR($CDCl_3$): δ 0.01 (m,1H), 0.19 (m,1H), 0.39 (m,1H), 0.46 (m,1H), 0.61 (m,2H),0.69 (m,1H), 0.80 (m,1H), 1.30 (m,3H), 1.65 (dd, J = 4,14 Hz, 1H), 3.76 (s,3H), 4.06 (dd, J = 5,15 Hz, 1H), 4.49 (dd, J = 5,15 Hz,1H), 4.96 (d, J = 16 Hz, 1H), 5.14 (d, J = 7 Hz, 1H), 5.15 (d, J = 16 Hz, 1H), 5.27 (d, J = 15 Hz, 1H), 6.09 (m,1H), 6.69 (d, J = 8 Hz, 1H), 6.84 (d, J = 7 Hz, 2H), 7.07 ( dd, J = 2,8 Hz, 1H), 7.20 ( d, J = 7 Hz, 1H), 7.45 (s, 1H).

Step B: 3,4-dihydro-3-allyl-4-cyclopropyl-4-cyclopropylmethyl-6-chloroquinazolin-2(1H)one

In a manner similar to Example 27, step B, from 490 mg(1.12 mmol) 3,4-dihydro-1-(4-methoxybenzyl)-3-allyl-4-cyclopropyl-4-cyclopropylmethyl-6-chloroquinazolin-2(1H)one and 1 ml methylene chloride/1 ml TFA over 2 hrs., was obtained 285 mg(82%) of title compound as a white crystalline solid, mp:160-162°C.
$^1$H NMR ($CDCl_3$): δ 0.01(m,1H), 0.23(m,1H), 0.44(m,1H), 0.49(m,1H), 0.63(m,2H), 0.67(m,1H), 0.80(m,1H), 1.29(m,3H), 1.60(dd, J = 4,12 Hz, 1H), 3.95(dd, J = 4,14 Hz, 1H), 4.44(dd, J = 4, 16 Hz, 1H), 5.13(d, J = 9 Hz, 1H), 5.26(d,J = 15 Hz, 1H), 6.04(m,1H), 6.62(d, J = 7 Hz, 1H), 7.12(dd, J = 1,7Hz, 1H), 7.39(s,1H), 8.21(br s, 1H).

EXAMPLE 29

3,4-dihydro-4-cyclopropyl-4-(3-butyl-1-yl)-6-chloroquinazolin-2(1H)one

Step A: 3,4-dihydro-1-(4-methoxybenzyl)-4-cyclopropyl-4-(4-(trimethylsilyl)-3-butyn-1-yl)-6-chloroquinazolin-2(1H)one

To a vigorously stirred suspension of 0.62 g(26.14 mmol) of Mg metal, 5 ml anhydrous THF, and 6-8 drops 1,1-dibromoethane was added slowly dropwise a solution of 3.50 g(21.78 mmol) 1-trimethylsilyl-4- chloro-1-butyne and 0.82 g(4.36 mmol) 1,1-dibromoethane in 25 mL anhydrous THF. The addition rate was adjusted so that the entire addition took approx. 45 min. After completion of the addition, the reaction was stirred vigorously at reflux for 2.5 hrs., and was cooled to room temp.

A solution of 1.65 g (4.84 mmol) of 1-(4-methoxybenzyl)-4-cyclopropyl-6-chloroquinazolin- 2(1H)one and 1.25 g (4.84 mmol) of magnesium bromide etherate in 25 ml anh. THF was seperately prepared, and was stirred at room temp. for 15 min. This solution was added dropwise to the previously prepared Grignard reagent solution over 15 min., and the resulting mixture stirred at room temperature for 1 hr. The reaction was treated with 30 ml. of cold 10 % citric acid solution, and the mixture extracted with 3 x 35 ml ether. The combined ethereal extracts were washed with 30 ml water and 30 ml brine, and were dried(anh. $MgSO_4$). Concentration in vacuo gave an oily yellow solid. The crude solid was purified via flash chromatography over silica gel with 25:1 chloroform/ethyl acetate to give 0.45g(22%) of title compound as a lt. brown crystalline solid, mp:138-141°C.
$^1$H NMR($CDCl_3$): δ 0.40 (s,9H), 0.31 (m,1H), 0.42 (m,2H), 0.58 (m,1H), 1.20 (m,1H), 1.89 (m,1H), 2.19-2.28 (m,2H), 2.39 (q,1H), 3.78 (s,3H), 5.04 (q,1H), 6.70 (d,J = 8 Hz, 1H), 6.86 (d,J = 7 Hz,1H), 7.07 (dd,J = 2,8 Hz,1H), 7.12-7.19 (d,J = 7 Hz,2H, and s,1H).

Step B: 3,4-dihydro-1-(4-methoxybenzyl)-4-cyclopropyl-4-(3-butyn-1-yl)-6-chloroquinazolin-2(1H)one

To a solution of 53.45 mg(0.92 mmol) of KF in 42 ml water/2 ml DMF was added a solution of 105.00 mg(0.23 mmol)3,4-dihydro-1-(4-methoxybenzyl)-4-cyclopropyl-4-(4-trimethylsilyl-3-butyn-1-yl)-6-chloroquinazo lin-2(1H)one in 2 ml DMF. The resulting solution was stirred at room temp. for 18 hrs., and was treated with 10 ml 10% citric acid solution. The suspension was extracted with 10 ml ethyl acetate, and the extract washed with 2 x 25 ml water, 5 ml brine, and dried(anh. $MgSO_4$). Concentration in vacuo gave a yellow oil, which was purified via flash chromatography over silica gel (ethyl acetate/chloroform) to give 57 mg(63%) of title compound as a clear oil.
$^1$H NMR($CDCl_3$): δ 0.34 (m,1H), 0.42 (m,2H), 0.54 (m,1H), 1.18 (m,1H), 2.90 (m,1H , and s,1H), 2.09 (m,1H), 2.12 (m,1H), 2.26 (m,1H), 3.78 (s,1H), 5.03 (q,2H), 5.81 (br s, 1H), 6.71 (d, J = 8 Hz,1H), 6.87 (d,J = 7 Hz,2H), 7.08 (dd J = 2,8 Hz, 1H), 7.18 (s,1H, and d,J = 7 Hz, 2H).

Step C: 3,4-dihydro-4-cyclopropyl-4-(3-butyn-1-yl)-6-chloroquinazolin-2(1H)one

In a manner similar to Example 27, step B, from 55.00 mg(0.14 mmol) of 3,4-dihydro-1-(4-methoxybenzyl)-4-cyclopropyl-4-(3-butyn-1-yl)-6-chloroquinazolin-2(1H)one in 1ml methylene chloride/1ml TFA over 72 hrs., was obtained 22 mg(58%) of title compound as a clear glass.
$^1$H NMR($CDCl_3$): δ 0.39 (m,1H), 0.46 (m,1H), 0.57 (m,1H), 0.89 (m,1H), 1.20 (m,1H), 1.92 (m,1H), 1.96 (s,1H), 2.10 (t,1H), 2.23 (q,2H), 5.23 (br s,1H), 6.67 (d,J = 8 Hz, 1H), 7.12 (m,2H), 8.60 (br s, 1H).

EXAMPLE 30

3,4-dihydro-3-methyl-4-cyclopropyl-4-(2-butyn-1-yl)-6-chloroquinazolin-2(1H)one

Step A: 3,4-dihydro-1-(4-methoxybenzyl)-4-cyclopropyl-4-(2-butyn-1-yl)-6-chloroquinazolin-2(1H)one

To a suspension of 70.10 mg(2.92 mmol) of Mg metal in 3 ml anh. ether was added 2-3 mg of $HgCl_2$, followed by 89.22 mg(0.75 mmol) of propargyl bromide. The suspension was heated with vigorous stirring until the reaction began, and the remaining 267.68 mg(2.25 mmol) of propargyl bromide was added rapidly. The suspension was again heated, and a vigorous reaction began. The suspension was stirred vigorously for 45 min., at which point the reaction was almost homogenous.

The above prepared Grignard reagent was added dropwise to a solution of 250 mg(0.73 mmol) of 1-(4-methoxybenzyl)-4-cyclopropyl-6-chloroquinazolin-2(1H)one in 10 ml anh. THF. The reaction was stirred at ambient temp. for 30 min., and was poured into 25 ml of cold 10% citric acid solution. The mixture was diluted with 30 ml of ether, and the layers were separated. The aqueous layer was extracted with 2 x 25 ml of ether, and the combined ethereal extracts washed with 25 ml water and 25 ml brine. Drying(anh. $MgSO_4$) and concentration in vacuo gave 400 mg of crude product as a yellow oil. The crude oil was purified via flash chromatography over silica gel with 6:1 chloroform/ethyl acetate to give 140 mg (56%) of title compound as a white crystalline solid, mp:126-129°C.
$^1$H NMR($CDCl_3$): d 0.31 (m,1H), 0.47 (m,2H), 0.57 (m,1H),1.38 (m,1H), 2.05 (t,1H), 2.73 (t,2H), 3.77 (s,3H), 5.06 (s,2H), 5.92 (s,1H), 6.72 (d,J = 8 Hz, 1H), 6.84 (d,,J = 7Hz, 2H), 7.08 (dd, J = 2, 8 Hz, 1H), 7.20 (d, J = 7 Hz,2H), 7.21 (s,1H).

Step B: 3,4-dihydro-1-(4-methoxybenzyl)-3-methyl-4-cyclopropyl-4-(2-butyn-1-yl)-6-chloroquinazolin-2-(1H)one

In a manner similar to Example 27, step C, from 1.30 g(3.41 mmol) of 3,4-dihydro-1-(4-methoxybenzyl)-4-cyclopropyl-4-(2-butyn-1-yl)-6-chloroquinazolin-2(1H)one, 163.68 mg(3.50 mmol) NaH, and 484.00 mg(3.41 mmol) of methyl iodide was obtained 1.17g(87%) of title compound as a white crystalline solid, mp:128-129°C.
$^1$H NMR($CDCl_3$): δ 0.15 (m,1H), 0.22 (m,1H), 0.56 (m,2H), 1.26 (m,1H), 1.90 (t,1H), 2.89 (dd, J = 2,16 Hz, 1H), 2.95 (dd, J = 2,16 Hz, 1H), 3.26 (s,3H), 3.77 (s,3H), 5.07 (q,2H), 6.70 (d,J = 8Hz,1H), 6.83 (d,J = 7 Hz, 1H), 7.09 (dd, J = 2,7Hz, 1H), 7.16 (s,1H), 7.18 (d, J = 7 Hz, 2H).

Step C: 3,4-dihydro-3-methyl-4-cyclopropyl-4-(2-butyn-1-yl)-6-chloroquinazolin-2(1H)one

In a manner similar to Example 27, step B, from 1.00g(2.53 mmol) of 3,4-dihydro-1-(4-methoxybenzyl)-3-methyl-4-cyclopropyl-4-(2-butyn-1-yl)-6-chloroquinazolin-2(1H)one and 8 ml methylene chloride/8 ml TFA over 18 hrs. was obtained 0.64g(91%) of the title compound as a white solid, mp: 204.0-205.5°C.
$^1$H NMR($CDCl_3$): δ 0.27 (m,2H), 0.55 (m,2H), 1.26 (m,1H), 1.89 (t,1H), 2.80 (dd, J = 2,17 Hz, 1H), 2.96 (dd,J = 2,17 Hz, 1H), 3.70 (s,3H), 6.67 (d, J = 7 Hz, 1H), 7.12 (s,1H), 7.18 (dd,J = 1,7Hz, 1H), 7.81(br s, 1H).

EXAMPLE 31

3,4-dihydro-3-methyl-4-cyclopropyl-4-(2-oxopropan-1-yl)-6-chloroquinazolin-2(1H)one

A solution of 15.16 mg(0.07 mmol) of red HgO in 4.5 ml of 0.75M $H_2SO_4$ was heated to 60°C. To the warm solution was added dropwise a solution of 125.00 mg(0.46 mmol) of 3,4-dihydro-3-methyl-4-cyclopropyl-4(2-butyn-1-yl)-6-chloroquinazolin-2(1H)one in 4 ml THF. A thick white suspension resulted, which quickly became a yellow-green solution. The solution was stirred at 60°C for 30 min., and was cooled to room temp. The reaction was diluted with 15 ml. ether, stirred for 5 min., and the layers separated. The aqueous layer was extracted with 10 ml ether, and the combined ethereal extracts washed with 10 ml water and 10 ml brine. Drying(anh.$MgSO_4$) and concentration in vacuo gave an off-white oil/solid. The crude product was purified via flash chromatography over silica gel with ethyl acetate to give 92 mg(69%) of the title compound as a white solid mp:178-179°C.
$^1$H NMR($CDCl_3$): δ 0.29 (m,1H), 0.38 (m,1H), 0.59 (m,2H), 1.26 (m,1H), 1.98 (s,3H), 2.96 (d J = 14 Hz, 1H), 3.09 (s,3H), 3.13 (d,J = 14 Hz, 1H), 6.70 (d,J = 7 Hz,1H), 7.06 (s,1H), 7.12 (dd,J = 2,7 Hz,1H), 8.84 (br s,1H).

EXAMPLE 32 AND 33

3,4-dihydro-3-methyl-4-cyclopropyl-4-(2-(R) and (S)-hydroxypropan-1-yl)-6-chloroquinazolin-2(1H)one

To a solution of 94.00 mg(0.32 mmol) of 3,4-dihydro-3-methyl-4-cyclopropyl-4-(2-oxopropan-1-yl)-6-chloroquinazolin-2(1H)one in 10 ml of absolute EtOH was added 60.53 mg(1.60 mmol) of $NaBH_4$. After 2 hrs., an additional 15.00 mg(0.40 mmol) portion of $NaBH_4$ was added, and stirring continued. After 2.5 additional hrs., the reaction was partitioned between 15 ml of 0.5 N HCl/ 15 ml chloroform. The aqueous layer was extracted with 10 ml chloroform, and the combined extracts washed with 15 ml water and 15 ml brine. Drying(anh. $Na_2SO_4$) and concentration in vacuo gave 95 mg of a clear oil. Separation of the diastereomeric mixture via flash chromatography over silica gel with 2% methanol/chloroform gave 44 mg(46%) of the higher running (R)-alcohol as a white crystalline solid, mp: 198-199°C.
$^1$H NMR($CDCl_3$): δ 0.30 (m,1H), 0.42 (m,1H), 0.54 (m,1H), 0.54 (m,1H), 0.59 (m,1H), 1.18 (d,J = 5 Hz,1H), 1.25 (m,1H), 2.00 (dd, J = 5,14 Hz, 1H), 2.07 (dd, J = 5, 14 Hz, 1H), 3.30 (s,3H), 3.76 (q,1H), 6.69 (d, J = 7 Hz,1H), 7.05 (dd, J = 2,7Hz,1H), 7.20 (s,1H), 8.80 (br s,1H). Further elution of the column gave 19 mg(20%) of the lower running (S)-alcohol as a white solid, mp: 221.5-223.0°C. $^1$H NMR($CDCl_3$): δ 0.27 (m,1H), 0.40 (m,1H), 0.51 (m,1H), 0.59 (m,1H), 0.90 (d, J = 4 Hz,3H), 1.27 (m,1H), 1.77 (dd, J = 3,12 Hz, 1H), 2.26 (dd, J = 5, 14 Hz, 1H), 3.10 (s,3H), 3.70 (m,1H), 6.72 (d, J = 7 Hz, 1H), 7.02 (s,1H), 7.05 (dd, J = 1,7Hz, 1H), 9.07 (br s, 1H).

EXAMPLE 34

(+/-)-6-Chloro-4-cyclopropyl-3,4-dihydro-4-isopropylquinazolin-2(1H)one

Step A: (+/-)-6-Chloro-4-cyclopropyl-3,4-dihydro-1-(4-methoxybenzyl)-4-isopropylquinazolin-2(1H)-one

To a stirring solution of 0.20 g (0.59 mmol) of 6-chloro-4-cyclopropyl-1-(4-methyoxybenzyl)-quinazolin-2(1H)-one in 15 ml ether was added 151 mg (0.59 mmol) of magnesium bromide etherate. After 30 min, 0.59 ml (1.76 mmol) of a 2.0 M solution of isopropyl magnesium chloride in ether was added dropwise over 2 min. After 16 hr the reaction was quenched with 10% $NaHCO_3$ and this mixture, after stirring for 15 min, was partitioned between $H_2O$ and EtOAc. The organic layer was washed with brine, dried over $Na_2SO_4$, concentrated, and the residue chromatographed on silica gel using 1:2 EtOAC:hexane to give 95 mg (42%) of the title compound.
$^1$H NMR($CDCl_3$): δ 0.10-0.12 (m, 5H), 0.83 (d, J=6.59, 3H), 1.08 (d, J=6.84, 3H), 2.18 (m, 1H), 3.77 (s, 3H), 5.02 (br s, 1H), 5.09 (br s, 2H), 6.66 (d, J=8.79, 1H), 6.84 (d, J=8.54, 2H), 7.14 (d, J=8.30, 2H), 7.16 (dd, J=8.21, 2.42, 1H), 7.20 (d, J=2.20, 1H).

Step B: (+/-)-6-Chloro-4-cyclopropyl-3,4-dihydro-4-isopropylquinazolin-2(1H)-one

In the manner outlined in Example 42, step A, the title compound was prepared from (+/-) 6-chloro-4-cyclopropyl-3,4-dihydro-1-(4-4-methoxybenzyl)-4-(2-propyl)quinazolin-2(1H)-one.
$^1$H NMR($CDCl_3$): δ 0.15-1.26 (m, 5H), 0.83 (d, J=6.8, 3H), 1.08 (d, J=6.8, 3H), 2.13-2.23 (m, 1H), 4.87 (br s, 1H), 6.65 (d, J=8.49, 1H), 7.07 (dd, J=8.46, 2.28, 1H), 7.17 (d, J=2.20, 1H), 9.02 (br s, 1H).
mp: 239-241°C

EXAMPLE 35

(+/-)-6-Chloro-4-cyclopropyl-3-methyl-3,4-dihydro-1-methyl-4-cyclopropylmethylquinazolin-2(1H)-one

To a room temperature stiring solution of 290.8 mg (1.00 mmol) of (+/-) 6-Chloro-4-cyclopropyl-4-cyclopropylmethyl-3-methyl-3,4-dihydroquinazolin-2(1H)one in 8 ml DMF was added 79 mg (2.00 mmol) of 60% NaH in oil. After 20 min 568 mg (4.00 mmol) of iodomethane was added. After 16 hr the reaction was quenched with $H_2O$ and solvents were removed invacuo to give a residue which was partitioned between 10% $NaHCO_3$ and EtOAc. The organic layer was washed with brine,dried over $Na_2SO_4$, concentrated and the residue chromatographed on silica gel using 1:1.5 EtOAc:hexane to give 250 mg (82%) of the title compound as a colorless solid.
mp: 108-110°C.
$^1$H NMR ($CDCl_3$): δ (m,1H), 0.04 (m, 1H), 0.20-0.68 (m, 7H), 1.21 (m, 1H), 1.46 (dd, J=9.5, 5.5, 1H), 1.80 (dd, J=9.5, 5.5, 1H), 3.13 (s, 3H), 3.31 (s, 3H), 6.72 (d, J=8.65, 1H), 7.20 (dd, j=8.65, 2.44, 1H), 7.32(d, J=2.42, 1H).

EXAMPLE 36

(+/-)-6-Chloro-4-cyclopropyl-3-propargyl-3,4-dihydro-4-cyclopropylmethylquinazolin-2(1H)-one

Step A: (+/-)-6-Chloro-4-cyclopropyl-3-propargyl-3,4-dihydro-1-(4-methoxybenzyl)-4-cyclopropylmethylquinazolin-2(1H)-one

In the manner outlined in Example 35, the title compound was prepared from (+/-) 6-chloro-4-cyclopropyl-3,4-dihydro-1-(4-methoxybenzyl)-4-cyclopropylmethylquinazolin-2(1H)-one.
$^1$H NMR($CDCl_3$): δ (-) 0.40 - (-) 0.15 (m,1H), 0.00-0.86 (m, 8H), 1.42 (m,1H), 1.57 (ddd, J=174.6, 14.6, 5.9, 2H), 2.18 (t, J=2.4, 1H), 3.74 (s, 3H), 4.49 (ddd, J=98.9, 17.6,.2.4, 2H), 5.07 (dd, J=65.9, 16.3, 2H), 6.66 (d, J=8.8, 1H), 6.81 (d, J=8.6, 2H), 7.04 (dd, J=8.79, 2.44, 1H), 7.17 (d, J=8.6, 2H), 7.38 (d, J=2.38, 1H).

Step B: (+/-)-6-Chloro-4-cyclopropyl-3-propargyl-3,4-dihydro-4-cyclopropylmethylquinazolin-2(1H)-one

In the manner outlined in Example 42, step A, the title compound was prepared from (+/-)-6-chloro-4-cyclopropyl-3-propargyl-3,4-dihydro-1-(4-methoxybenzyl)-4-cyclopropylmethylquinazolin-2(1H)-one.
mp: 158-161°C
$^1$H NMR ($CDCl_3$): δ 0.19-1.83 (m, 12H), 2.19 (s, 1H), 4.22 (dd, J=17.4, 1.47, 1H), 4.58 (dd, J=17.4, 1.47, 1H), 6.60 (m, 1H), 7.13-7.41 (m, 3H).

EXAMPLE 37

(+)-6-Chloro-4-cyclopropyl-3-ethyl-3,4-dihydro-4-propylquinazolin-2(1H)-one

Step A: (+)-6-Chloro-4-cyclopropyl-3,4-dihydro-1-(4-methoxybenzyl)-4-propylquinazolin-2(1H)-one

To a stirred solution of 0.77 g (2.94 mmol) of (+) 6-chloro-4-cyclopropyl-3,4-dihydro-4-propylquinazolin-2(1H)-one, in 10 ml DMF was added 141.2 mg (3.53 mmol) of 60% NaH in oil. After 1 hr at room temperature 0.44 g (2.94 mmol) of NaI followed by 0.483 g (3.09 mmol) of 4-methoxybenzyl chloride were added to the reaction. After 16 hr the solvent was removed under reduced presure and the residue was partitioned between $H_2O$ and EtOAc. The organic layer was washed with brine, dried over $Na_2SO_4$ and concentrated to yield a residue which was chromatographed on silica gel using 97:3 $CHCl_3$:$CH_3OH$ to give 1.06 g (94%) of the title compound as a colorless solid.

$^1H$ NMR ($CDCl_3$): δ 0.15-0.6 (m, 4H), 0.89 (t, J=7, 3H), 1.1-1.22 (m, 1H), 1.4-1.65 (m, 2H), 1.80-1.92 (m, 1H), 3.77 (s, 3H), 4.95-5.12 (m, 2H), 5.32 (s, 1H), 6.69 (d, J=9, 1H), 6.83 (d, J=8, 2H), 7.05 (dd, J=9, 2, 1H), 7.14 (d, J=2, 1H), 7.17 (d, J=8, 2H ).

Step B: (+)-6-Chloro-4-cyclopropyl-3-ethyl-3,4-dihydro-1-(4-methoxybenzyl)-4-propylquinazolin-2(1H)-one

In the manner outlined in Example 35, step A, the title compound was prepared from (+)-6-Chloro-4-cyclopropyl-3,4-dihydro-1-(4-methoxybenzyl)-4-propylquinazolin-2(1H)-one.
$^1H$ NMR ($CDCl_3$): δ 0.30-0.67 (m, 4H), 0.86 (t, J=4.9, 3H), 1.02-1.29 (m, 4H), 1.32 (t, J=7.0, 3H), 1.70-1.82 (m, 1H) 2.54-2.70 (m, 2H), 3.76 (s, 3H), 5.05 (br s, 2H), 6.62 (d, J=8.9, 1H), 6.83 (d, J=8.6, 2H), 7.01 (dd, J=8.8, 2.4, 2H), 7.15 (d, J=8.7, 2H), 7.19 (d, J=2.4, 1H).

Step C: (+)-6-Chloro-4-cyclopropyl-3-ethyl-3,4-dihydro-4-propylquinazolin-2(1H)-one

In the manner outlined in Example 42, step A, the title compound was prepared from (+)-6-Chloro-4-cyclopropyl-3-ethyl-3,4-dihydro-1-(4-methoxybenzyl)-4-propylquinazolin-2(1H)-one.
$^1H$ NMR ($CDCl_3$): δ 0.38-0.68 (m, 4H), 0.85 (t, J=6.78, 3H), 0.98-1.90 (m, 10H), 6.59 (d, J=8.24, 1H), 7.09 (d, J=6.96, 1H), 7.15 (d, J=1.83, 1H), 8.21 (br s, 1H).

EXAMPLE 38

(+/-)-6-Chloro-4-cyclopropyl-3-methyl-3,4-dihydro-4-isopropylmethylquinazolin-2(1H)-one

Step A: (+/-)-6-Chloro-4-cyclopropyl-3,4-dihydro-1-(4-methoxybenzyl)-4-isopropylmethylquinazolin-2(1H)-one

In the manner outlined in Example 34, step A, the title compound was prepared from 6-chloro-4-cyclopropyl-1-(4-methoxybenzyl)quinazolin-2(1H)-one.
$^1H$ NMR($CDCl_3$): δ 0.27-0.57 (m, 3H), 0.75 (d, J=6.64, 3H), 0.95 (d, J=6.64, 3H), 1.15-1.27 (m, 2H), 1.53-1.96 (m, 3H), 3.77 (s, 3H), 4.93 (br s, 1H), 5.05 (br s, 2H), 6.68 (d, J=8.79, 1H), 6.84 (d, J=8.6, 2H), 7.04 (dd, J=8.79, 2.34, 1H), 7.14-7.21 (m, 3H).

Step B: (+/-)-6-Chloro-4-cyclopropyl-3-methyl-3,4-dihydro-1-(4-methoxybenzyl)-4-isopropylmethylquinazolin-2(1H)-one

In the manner outlined in Example 35, step A, the title compound was prepared from (+/-)-6-Chloro-4-cyclopropyl-3,4-dihydro-1-(4-methoxybenzyl)-4-isopropyl methylquinazolin-2(1H)-one.
$^1H$ NMR($CDCl_3$): δ 0.19-0.62 (m, 3H), 0.63 (d, J=6.37, 3H), 0.92 (d, J=6.38, 3H), 1.22-1.94 (m, 5H), 3.13 (s, 3H), 3.76 (s,3H), 5.08 (m, 2H), 6.62 (d, J=8.85, 1H), 6.83 (d, J=8.71, 2H), 7.01 (dd, J=8.81, 2.41, 1H), 7.11 (d, J=2.42, 1H), 7.15 (d, J=8.79, 2H).

Step C: (+/-)-6-Chloro-4-cyclopropyl-3-methyl-3,4-dihydro-4-isopropylmethylquinazolin-2(1H)-one

In the manner outlined in Example 42, step A, the title compound was prepared from (+/-)-6-Chloro-4-cyclopropyl-3-methyl-3,4-dihydro-1-(4-methoxybenzyl)-4-isopropylmethylquinazolin-2(1H)-one.
mp: 213-214°C
$^1H$ NMR($CDCl_3$): δ 0.25-0.71 (m, 3H), 0.64 (d, J=6.32, 3H), 0.90 (d, J=6.54, 3H), 1.19-1.94 (m, 5H), 3.07, (s, 3H), 6.62 (d, J=8.22, 1H), 7.07 (s, 1H), 7.10 (d, J=2.25, 1H), 8.39 (br s, 1H).

EXAMPLE 39

(+/-)-6-Chloro-4-cyclopropyl-3,4-dihydro-4-n-butylquinazolin-2(1H)-one

Step A: (+/-)-6-Chloro-4-cyclopropyl-3,4-dihydro-1-(4-methoxybenzyl)-4-n-butylquinazolin-2(1H)-one

To a stirring mixture of 126.7 mg (5.21 mmol) of magnesium turnings in 12 ml of THF was added a catalytic amount of n-butyl bromide. Reaction was initiated by addition of a catalytic amount of iodine and heating. Reaction was maintained at 60°C and the remaining n-butyl bromide, 776.3 mg (5.66 mmol), was added dropwise

over 7 min. After 30 min, 250 mg (0.73 mmol) of 6-chloro-4-cyclopropyl-1-(4-methoxybenzyl) quinazolin-2(1H)-one dissolved in 20 ml THF was added dropwise over 12 min. After 30 min the reaction was quenched with water and extracted into EtOAc. The organic layer was washed with 10% $NaHCO_3$, brine, dried over $Na_2SO_4$, concentrated and the residue chromatographed on silica gel using 1:2 EtOAc:hexane to give 120 mg (38%) of the title compound as a colorless solid.
$^1$H NMR ($CDCl_3$): δ 0.28-0.59 (m, 3H), 0.871 (t, J=7.14, 3H), 1.07 (m, 8H), 3.77 (s, 3H), 4.79 (br s, 1H), 5.04 (br s, 2H), 6.69 (d, J=8.6, 1H), 6.84 (d, J=8.54, 2H), 7.04 (dd, J=9.28, 2.44, 1H), 7.15 (d, J=1.84, 2H), 7.18 (s, 1H).

Step B: (+/-)-6-Chloro-4-cyclopropyl-3,4-dihydro-4-n-butylquinazolin-2(1H)-one

In the manner outlined in Example 42, step A, the title compound was prepared from (+/-)-6-Chloro-4-cyclopropyl-3,4-dihydro-1-(4-methoxybenzyl)-4-n-butyl-quinazolin-2(1H)-one.
mp:136-138°C
$^1$H NMR($CDCl_3$): δ 0.29-1.95 (m, 11H), 0.85 (t, J=7.03, 3H), 5.07 (br s, 1H), 6.67 (d, J=8.3, 1H), 7.08 (d, J=2.20, 1H), 7.12 (dd, J=4.12, 2.23, 1H), 8.69 (br s, 1H).

EXAMPLE 40

(+)-6-Chloro-4-cyclopropyl-3-methyl-3,4-dihydro-4-n-propylquinazolin-2(1H)-one

Step A: (+)-6-Chloro-4-cyclopropyl-3-methyl-3,4-dihydro-1-(4-methoxybenzyl)-4-n-propylquinazolin-2(1H)-one

In the manner outlined in Example 35, step A, the title compound was prepared from (+)-6-Chloro-4-cyclopropyl-3,4-dihydro-1-(4-methoxybenzyl)-4-propylquinazolin-2(1H)-one.
$^1$H NMR($CDCl_3$): δ 0.06-0.60 (m, 4H), 0.91 (t, J=7.32, 3H), 1.08-2.06 (m, 5H), 3.14 (s, 3H), 3.77 (s, 3H), 4.95-5.13 (m, 2H), 6.64 (d,J=8.92, 1H), 6.83 (d, J=8.60, 2H), 7.03 (dd, J=8.79, 2.33, 1H), 7.04-7.19 (m, 3H).

Step B: (+)-6-Chloro-4-cyclopropyl-3-methyl-3,4-dihydro-4-n-propylquinazolin-2(1H)-one

In the manner outlined in Example 42, step A, the title compound was prepared from (+)-6-Chloro-4-cyclopropyl-3-methyl-3,4-dihydro-1-(4-methoxybenzyl)-4-n-propylquinazolin-2(1H)-one.
mp: 133-135°C
$^1$H NMR($CDCl_3$): δ 0.19-0.61 (m, 4H), 0.90 (t, J=7.57, 3H), 1.05-2.00 (m, 5H), 3.08 (s, 3H), 6.59 (d, J=8.31, 1H), 7.06 (d, J=2.20, 1H), 7.10 (dd, J=8.41, 2.28, 1H), 7.71 (br s, 1H).

EXAMPLE 41

(+/-)-6-Chloro-4-allyl-3,4-dihydro-4-cyclopropylquinazolin-2(1H)-one

In the manner outlined in Example 39, step A, the title compound was prepared from 6-chloro-4-cyclopropylquinazolin-2(1H)-one.
mp: 84-85°C
$^1$H NMR($CDCl_3$): δ 0.29-1.90 (m, 5H), 2.48-2.67 (m, 2H), 4.99 (s, 1H), 5.13 T, J=10.25, 2H), 5.68-5.83 (m, 1H), 6.66 (d, J=8.49, 1H), 7.12 (dd, J=8.49, 2.25, 1H), 7.19 (d, J=2.20, 1H), 8.20 (s, 1H).

EXAMPLE 42

(+/-) 6-Chloro-4-cyclopropyl-3,4-dihydro-4-propylquinazolin-2(1H)-one

To a stirred solution of 42 mg (0.11 mmol) of (+/-) 6-chloro-4-cyclopropyl-1-(4-methoxybenzyl)-3,4-dihydro-4-propylquinazolin-2(1H)-one in 1 ml of $CH_2CL_2$ at 10°C was added 1 ml of TFA. After 1 hr at 10°C followed by 1 hr at rt, the reaction mixture was concentrated under reduced pressure and the residue was partitioned betwen EtOAc and and water. The organic layer was washed with 10% $NaHCO_3$, brine, dried over $Na_2SO_4$ and the solvent removed under reduced pressure. The resulting residue was chromatographed on silica gel using 1:1 EtOAc: hexane followed by hexane trituration to give 13 mg (45%) of the title compound as a colorless solid mp: 94-97°C

$^1H$ NMR ($CDCl_3$): δ 0.31-0.61 (m, 4H), 0.89 (t, J=7.33, 3H), 1.08-1.92 (m, 5H), 4.88 (br s, 1H), 6.64 (d, J=8.43, 1H), 7.13 (dd, J=8.24, 2.20, 1H), 7.14 (d, J=2.01, 1H), 7.89 (br s, 1H).

EXAMPLE 43

(+)-6-Chloro-4-cyclopropyl-3,4-dihydro-4-propylquinazolin-2(1H)-one

Step A: (+)-6-Chloro-4-cyclopropyl-3,4-dihydro-1-camphanyl-4-propylquinazolin-2(1H)-one

3.86 g (14.58 mmol) Of (+/-) 6-chloro-4-cyclopropyl-3,4-dihydro-4-propylquinazolin-2(1H)-one, 7.90 g (36.45mmol) of (-) camphanic chloride, and 1.78 g (14.58 mmol) of 4-dimethylaminopyridine were combined and stirred in 90 ml of $CH_2Cl_2$ at room temperature. 6.64 g (65.61 mmol) of triethylamine was added dropwise over 20 min. After 15 hr the solvent was removed under reduced pressure and the residue diluted with 500 ml of EtOAc. This was washed with 10% citric acid, water, brine, dried over $Na_2SO_4$ and concentrated to yield 11 g of a residue containing four components. The slowest moving component was isolated by silica chromatography using 1:7 EtOAc:$CHCl_3$ and characterized as the title compound, 2.21 g (34%).
$^1H$ NMR ($CDCl_3$): δ 0.13-0.18 (m, 1H), 0.39-0.56 (m, 3H), 0.80 (s, 3H), 0.92 (t, J=7.3, 3H), 1.09 (s, 3H), 1.10-1.22 (m, 1H), 1.22 (s, 3H), 1.33-1.40 (m, 1H), 1.69-1.80 (m, 3H), 1.92-2.04 (m, 2H), 2.54-2.60 (m, 2H), 6.25 (br s, 1H), 7.08 (d, J=2.44, 1H), 7.20 (dd, J=8.9, 1.4, 1H), 7.70 (d, J=9.0, 1H).

Step B: (+)-6-Chloro-4-cyclopropyl-3,4-dihydro-4-propylquinazolin-2(1H)-one

To a stirred room temperature solution of 1.49 g (3.35 mmol) of (+)-6-chloro-4-cyclopropyl-3,4-dihydro-1-camphanyl-4-propylquinazolin-2(1H)-one in 60 ml methanol was added 0.926 g (6.70 mmol) of $K_2CO_3$. After 30 min 0.926 g (6.70 mmol) additional $K_2CO_3$ was added and the mixture was warmed to 45°C. After 30 min another 0.926 g (6.70 mmol) of $K_2CO_3$ was added and the mixture was refluxed. After 16 hr the reaction was concentrated under reduced pressure and the residue was partitioned between water and EtOAc. The organic layer was washed with brine, dried over $Na_2SO_4$ and concentrated to yield a solid which was chromatographed on silica gel using 95:5 $CHCL_3$:MeOH to give 0.662 g (75%) of the title compound as a colorless solid.
mp: 188-190°C
$^1H$ NMR ($CDCl_3$): δ 0.31-0.61 (m, 4H), 0.89 (t, J=7.33, 3H), 1.08-1.92 (m, 5H), 4.88 (br s, 1H), 6.64 (d, J=8.43, 1H), 7.13 (dd, J=8.24, 2.20, 1H), 7.14 (d, J=2.01, 1H), 7.89 (br s, 1H)

EXAMPLE 44 AND 45

6-Chloro-4-cyclobutyl-4-cyclopropyl-3,4-dihydroquinazolin-2(1H)-one and

6-Chloro-4-cyclobutyl-4-cyclopropyl-3-methyl-3,4-di-hydroquinazolin-2(1H)-one

Step A: 6-Chloro-4-cyclobutyl-4-cyclopropyl-1-(4-methoxybenzyl)-3,4-dihydroquinazolin-2(1H)-one

Cyclobutyl magnesium bromide was prepared by adding dropwise a solution of 3.0 g (0.22 mol) of cyclobutylbromide in 15 mL of anhydrous THF to a stirred suspension of 450 mg (0.185 g-atom) magnesium turnings in 6 mL of THF, stirred under argon at 45-50°C. When the magnesium had been consumed (2h), the solution was diluted with 14 mL THF to a final concentration of ca. 0.55 M. The title compound was prepared by the addition of 18 mL (9-10 meq) of the cyclobutylmagnesium bromide solution to a mixture of 1.0g (3.0 mmol) of 6-chloro-4-cyclopropyl-1(4-methoxybenzyl)quinazolin-2(1H)-one and 774 mg (3.0 mmol) of magnesium bromide etherate in 40 mL of anhydrous ethyl ether as described in Example 27, Step A. Chromatographic purification of the crude product afforded 295 mg (25%) of the title compound:
$^1H$ NMR ($CDCl_3$): δ 0.10-0.60 (m, 4H); 1.16 (m, 1H); 1.62-2.07 (m, 6H); 2.87 (m, 1H); 3.77 (s, 3H); 4.92 (s, 1H); 4.99 (d, $J_A$ = 16 Hz, $1H_A$); 5.06 (d, $J_B$ = 16 Hz, $1H_B$); 6.67 (d, J = 9 Hz, 1H); 6.84 (d, J = 8.5 Hz, 2H); 7.04 (dd, J = 2, 8.9 Hz, 1H); 7.16 (m, 3H).

Step B: 6-Chloro-4-cyclobutyl-4-cyclopropyl-1-(4-methoxybenzyl)-3-methyl-3,4-dihydroquinazolin-2(1H)-one

The product from Step A (120 mg, 0.3 mmol) was treated according to the method of Example 14, Step B to yield 120 mg of the crude product. This was purified by flash chromatography on silica gel, eluting with 95/5 $CHCl_3$/EtOAc to give 90 mg of pure title compound as a white solid:

$^1H$ NMR ($CDCl_3$): δ 0.68-1.00 (m,4H); 1.20-1.88 (m, 7H); 2.55 (m, 1H); 3.25 (s, 3H); 4.76 (d, $J_A$ = 16 Hz, $1H_A$); 5.23 (d, $J_B$ = 16 Hz, $1H_B$); 6.69 (d, J = 8.7 Hz, 1H); 6.84 (d, J = 8.7 Hz, 2H); 7.09 (dd, J = 2, 8.7 Hz, 1H); 7.18 (d, J = 8.7 Hz, 2H); 7.63 (d, J = 2Hz, 1H).

Step C: 6-Chloro-4-cyclobutyl-4-cyclopropyl-3,4-dihydroquinazolin-2(1H)-one

The product from Step A (95 mg, 0.23 mmol) was stirred in a mixture of 2 mL each dichloromethane and trifluoroacetic acid under argon for 36 h. Removal of the volatiles left a residue which was dissolved in EtOAc. The EtOAc solution was washed successively with aqueous saturated $NaHCO_3$, water and brine and dried over $MgSO_4$. Evaporation of the solvent yielded 70 mg of a pale yellow solid which was triturated with acetonitrile to afford pure title compound, mp 221-222°C.
$^1H$ NMR ($CDCl_3$): δ 0.12-0.64 (m 4H); 1.16 (m, 1H); 1.60-2.10 (m, 6H); 2.83 (m, 1H); 4.93 (s, 1H); 6.63 (d, J = 8.5 Hz, 1H); 7.15 (m, 2H); 7.93 (s, 1H).

Step D: 6-Chloro-4-cyclobutyl-4-cyclopropyl-3-methyl-3,4-dihydrpquinazolin-2(1H)-one

The product from Step B (88 mg, 0.22 mmol) was treated according to Step C (Example 44) to yield the title compound as an oil which crystallized from acetonitrile: mp 231-232°C;
$^1H$ NMR ($CDCl_3$): δ 0.66-0.96 (m, 5H); 1.19-1.30 (m, 2H); 1.40-1.98 (m, 6H); 2.58 (m, 1H); 3.18 (s, 3H); 6.60 (d, J = 8.5 Hz, 1H); 7.10 (br s, 1H); 7.17 (dd, J = 2, 8.5 Hz, 1H); 7.57 (d, J = 2 Hz, 1H).

EXAMPLE 46

6-Chloro-4-cyclopropyl-4-ethynyl-3-(2-methoxyethyl)-3,4-dihydroquinazolin-2(1H)-one

Step A: 6-Chloro-4-cyclopropyl-3,4-dihydro-1-(4-methoxybenzyl)-4-(2-triisopropylsilylethynyl) quinazolin-2(1H)-one

A suspension of 14.2 g (44.0 mmol) of magnesium trifluoromethanesulfonate and 5.0 g (14.7 mmol) of 6-chloro-4-cyclopropyl-1-(4-methoxybenzyl)quinazolin-2(1H)-one in 100 mL of anhydrous ethyl ether was stirred under argon at room temperature for 30 minutes. To this mixture was added a cold solution of 1-lithio-2-(triisopropylsilyl)acetylene (prepared at -78° by dropwise addition of 17.6 mL (44.0 mmol) of n-butyllithium (2.5 N in hexane) to 9.9 mL (44.0 mmol) of trimethylsilylacetylene in 100 mL of ethyl ether); stirring was continued at rt for 18 hr. The reaction was quenched by addition of 50 mL of $CHCl_3$ followed by 15 mL of a pH 10 solution containing saturated aqueous $NH_4Cl$ (12. mL) and $NH_4OH$ (3 mL). After 30 min the organic phase was decanted and the residue was washed twice with fresh $CHCl_3$. The combined $CHCl_3$ solutions were washed with brine and dried ($Na_2SO_4$). Removal of the solvent left an oil which was flash chromatographed on silica gel (25% to 40% EtOAc gradient in hexane) to afford 6.18 g (85%) of the title compound as a white solid:
$^1H$ NMR ($CDCl_3$): δ 0.57-0.99 (m, 4H); 1.04 (s, 21 H); 3.77 (s, 3H); 5.04 (d, $J_A$= 16 Hz, $1H_A$); 5.13 (d, $J_B$= 16 Hz, $1H_B$); 5.341 (s, 1H); 6.72 (d, J = 9 Hz, 1H); 6.83 (d, J = 9 Hz, 2H); 7.09 (dd, J = 2, 9 Hz, 1H); 7.18 (d, J = 9 Hz, 2H); 7.48 (d, J = 2 Hz, 1H).

Step B: 6-Chloro-4-cyclopropyl-4-ethynyl-1-(4-methoxybenzyl)-3-(2-methoxyethyl)-3,4-dihydroquinazolin-2(1H)-one (**B1**) and 6-chloro-4-cyclopropyl-1-(4-methoxybenzyl)-4-(4-methoxybutynyl)-3-(2-methoxyethyl)-3,4-dihydroquinazolin-2(1H)-one (**B2**)

Sodium hydride (120 mg of a 60% oil dispersion, 3.0 mmol) was added to a solution of the product from Step A (750 mg, 1.5 mmol) in 15 mL of anydrous DMF under argon and the mixture was stirred at 25°C for 20 min, after which 0.75 mL (8.0 mmol) of bromomethyl methyl ether was added. After stirring for 18 h at 50°C, the solution was cooled to 25°C, treated with an additional 3.0 mmol each of sodium hydride and the bromomethyl ether and the solution was stirred at rt for 48 h. The reaction mixture was poured into 200 mL of cold water and extracted with 3 x 75 mL EtOAc. The combined organic layers were washed successively with water and brine and dried over $MgSO_4$. Evaporation of the solvent yielded 1.5 g of an oily residue which was flash chromatographed on silica gel to provide the following: 70 mg of unreacted starting material (product of Step A), then 450 mg of the monoalkylated title compound (**B1**):
$^1H$ NMR ($CDCl_3$): δ 0.32-0.52 (m, 2H), 0.62-0.89 (m, 2H), 1.30-1.43 (m, 1H), 2.73 (s, 1H); 3.39 (s, 3H); 3.60-3.86 (m, 4H); 3.77 (s, 3H); 5.01 (d, $J_A$ = 16 Hz, $1H_A$); 5.17 (d, $J_B$ = 16 Hz, $1H_B$); 6.71 (d, J = 9 Hz, 1H); 6.83 (d, J = 9 Hz, 2H); 7.11 (dd, J = 2, 9 Hz, 1H); 7.15 (d, J = 9 Hz, 2H); 7.46 (d, J = 2 Hz, 1H). Continued elution afforded

115 mg of the bis-alkylated title compound (**B2**): $^1H$ NMR ($CDCl_3$): δ 0.36 (dd, J = 8, 15 Hz, 2H); 0.56-0.96 (m, 3H); 2.58 (t, J = 7 Hz, 2H); 3.39 (s, 3H); 3.41 (s, 3H); 3.50-3.70 (m, 6H); 3.76 (s, 3H); 5.00 (d, $J_A$ = 16 Hz, $1H_A$); 5.17 (d, $J_B$ = 16 Hz, $1H_B$); 6.69 (d, J = 9 Hz, 1H); 6.82 (d, J = 9 Hz, 2H); 7.08 (dd, J = 2, 9 Hz, 1H); 7.13 (d, J = 9 Hz, 2H); 7.46 (d, J = 2 Hz, 1H).

Step C: 6-Chloro-4-cyclopropyl-4-ethynyl-3-(2-methoxyethyl)-3,4-dihydroquinazolin-2(1H)-one

In a manner according to entry Example 14, Step C, the title compound was prepared from 450 mg of monoalkylated,product (**B1**) from step B to give 170 mg of a white solid, mp 131-133°C:
$^1H$ NMR ($CDCl_3$): δ 0.39-0.50 (m, 2H); 0.60-0.90 (m, 2H); 1.35-1.48 (m, 1H); 2.72 (s, 1H); 3.39 (s, 3H); 3.59-4.12 (m, 4H); 6.70 (d, J = 8 Hz, 1H); 7.20 (dd, J = 2, 8 Hz, 1H); 7.40 (d, J = 2 Hz, 1H); 8.06 (br s, 1H).

EXAMPLE 47

6-Chloro-4-cyclopropyl-4-ethynyl-3-(3-propynyl)-3,4-dihydroquinazolin-2(1H)-one

Step A: 6-Chloro-4-cyclopropyl-3,4-dihydro-1-(4-methoxyenzyl)-4-(2-trimethylsilylethynyl) quinazolin-2(1H)-one

A suspension of 8.51 g (26.4 mmol) of magnesium trifluoromethanesulfonate and 3.0 g (8.8 mmol) of 6-chloro-4-cyclopropyl-1-(4-methoxybenzyl)quinazolin-2(1H)-one in 100 mL of anhydrous ethyl ether was stirred under argon at room temperature for 30 minutes. To this mixture was added a cold solution of 1-lithio-2-(trimethylsilyl)acetylene (prepared at -78°C by dropwise addition of 10.5 mL (26.25 mmol) of n-butyllithium (2.5 N in hexane) to 3.7 mL (26.4 mmol) of trimethylsilylacetylene in 80 mL of ethyl ether); stirring was continued at rt for 5 hr. The reaction was quenched by addition of 30 mL of $CHCl_3$, followed by 8 mL of a pH 10 solution containing saturated aqueous $NH_4Cl$ (7. mL) and $NH_4OH$ (1 mL). After 30 min the organic phase was decanted and the residue was washed twice with fresh $CHCl_3$. The combined $CHCl_3$ solutions were washed with brine and dried ($Na_2SO_4$). Removal of the solvent left a residue which was flash chromatographed on silica gel (25% to 50% EtOAc gradient in hexane) to afford 2.48 g of the title compound as a waxy white solid:
$^1H$ NMR ($CDCl_3$): δ 0.20 (s, 9H); 0.57-0.90 (m, 4H); 1.40-1:50 (m, 1H); 3.81 (s, 3H); 4.97 (d, J = 17 Hz, $1H_a$); 5.33 (d, J = 17 Hz, $1H_b$); 5.34 (s, 1H); 6.76 (d, J = 9 Hz, 1H); 6.87 (dd, J = 2, 7 Hz, 2H); 7.14 (dd, J = 2, 9 Hz, 1H); 7.22 (d, J = 9 Hz, 2H); 7.50 (d, J = 2 Hz, 1H).

Step B: 6-Chloro-4-cyclopropyl-4-ethynyl-1-(4-methoxybenzyl)-3-(3-propynyl)-3,4-dihydroquinazolin-2(1H)-one

The product from Step A (220 mg, 0.5 mmol) in 5 mL of anhydrous DMF was treated with 30 mg (0.75 mmol) of sodium hydride (60% dispersion in oil) under argon. After 10 min at room temp. the mixture was treated with 0.22 mL (2.0 mmol) of propargyl bromide (80% in toluene). The reaction proceeded at ambient temperature and was worked up as described in Example 46, step B. The 280 mg of crude product was purified by flash chromatography over silica gel (60% EtOAc in hexane) to afford 180 mg of the title compound as an oil:
$^1H$ NMR ($CDCl_3$): δ 0.42-0.63 (m, 2H); 0.68-0.81 (m, 1H);. 0.84-0.95 (m, 1H); 1.41-1.52 (m, 1H); 2.24 (t, J = 2.3 Hz, 1H); 2.73 (s, 1H); 3.77 (s, 3H); 4.55 (t, J = 2.7 Hz, 2H); 5.05 (d, $J_A$= 16 Hz, $1H_A$); 5.20 (d, $J_B$=16 Hz, 1 $H_B$); 6.73 (d, J = 9 Hz, 1H); .6.82 (d, J = 9 Hz, 2H); 7.12 (dd, J = 2, 9 Hz, 1H); 7.18 (d, J = 9 Hz, 2H); 7.48 (d, J= 2 Hz, 1H).

Step C: 6-Chloro-4-cyclopropyl-4-ethynyl-3-(3-propynyl)-3,4-dihydroquinazolin-2(1H)-one

In a manner according to Example 14, step C, 40 mg of the title compound, mp 188-190°, was prepared from 180 mg of the product from step B:
$^1H$ NMR ($CDCl_3$): δ 0.45-0.96 (m, 4H); 1.42-1.58 (m, 1H); 2.22 (s, 1H); 2.74 (s, 1H); 4.45 (d, $J_A$ = 20 Hz, $1H_A$); 4.54 (d, $J_B$ = 20 Hz, $1H_B$); 6.70 (d, J = 8.5 Hz, 1H); 7.20 (dd, J = 2, 8.5 Hz, 1H); 7.43 (d, J = 2 Hz, 1H); 7.80 (br s, 1H).

EXAMPLE 48

6-Chloro-4-cyclopropyl-4-ethynyl-3-methyl-3,4-dihydroquinazolin-2(1H)-one

Step A: 6-Chloro-4-cyclopropyl-4-ethynyl-1-(4-methoxybenzyl)-3-methyl-3,4-dihydroquinazolin-2(1H)-one

The product from step A, Example 47 (220 mg, 0.5 mmol), in 5 mL of anhydrous DMF was treated with 30 mg (0.75 mmol) of sodium hydride (60% dispersion in oil) under argon. After 5 min the mixture was treated with 0.31 mL (5.0 mmol) of iodomethane and stirring was continued at room temperature for 24 h. The mixture was poured into 50 mL cold aqueous saturated $NH_4Cl$ solution and extracted twice with EtOAc. The combined extracts were washed successively with water and brine and then dried over $MgSO_4$. Evaporation of the EtOAc gave 160 mg of the title compound as a waxy solid:
$^1H$ NMR ($CDCl_3$): $\delta$ 0.40-1.40 (m, 5H), 2.68 (s, 1H), 3.29 (s, 3H), 3.77 (s, 3H). 4.99 (d, $J_A$ =16 Hz, $1H_A$); 5.20 (d, $J_B$ =16 Hz, $1H_B$); 6.70 (d, J = 8.5 Hz 1H); 6.83 (d, J = 8.5 Hz, 2H); 7.11 (dd, J = 2, 8.5 Hz, 1H); 7.17 (d, J = 8.5 Hz, 2H); 7.49 (d, J = 2 Hz, 1H).

Step B: 6-Chloro-4-cyclopropyl-4-ethynyl-3-methyl-3,4-dihydroquinazolin-2(1H)-one

The title compound was prepared in a manner according to Example 14, step C, from the product of step A. There was obtained 44 mg of pure title compound, mp 169-170°C;
$^1H$ NMR ($CDCl_3$) $\delta$ 0.40-0.90 (m, 4H); 1.30-1.44 (m, 1H), 2.68 (s, 1H); 3.24 (s, 3H); 6.66 (d, J = 8.5 Hz, 1H); 7.19 (dd, J = 2, 8.5 Hz, 1H); 7.43 (d, J = 2 Hz, 1H); 7.65 (s, 1H).

EXAMPLE 49

6-Chloro-4-cyclopropyl-4-ethynyl-3,4-dihydroquinazolin-2(1H)-one

Step A: 6-Chloro-4-cyclopropyl-4-(2-trimethylsilylethynyl)-3,4-dihydroquinazolin-2(1H)-one

The solid from Example 47, Step A (116 mg, 0.26 mmol), was treated with trifluoroacetic acid in dichloromethane in a manner according to Example 14, step C, and the residue isolated upon workup was used directly in the next (B) step.

Step B: 6-Chloro-4-cyclopropyl-4-ethynyl-3,4-dihydroquinazolin-2(1H)-one

The crude product isolated from the previous step (Step A) was suspended in 5 mL of methanol and the suspension was treated with 25 mg of finely powdered potassium carbonate. The mixture was stirred at rt under argon for 3 h and then the methanol was removed under vacuum,.leaving a residue which was partitioned between 50 mL each of water and $CHCl_3$. The aqueous phase was extracted twice more with $CHCl_3$ and the combined $CHCl_3$ layers were washed with saturated $NH_4Cl$, brine, and dried ($MgSO_4$). The dried solution was concentrated to an oil which, upon purification by flash chromatography on silica gel (gradient, 10% to 25% EtOAc in $CHCl_3$), yielded 24 mg of the title compound; trituration with acetonitrile afforded a white solid, mp 216°.
$^1H$ NMR ($CDCl_3$): $\delta$ 0.59-0.95 (m, 4H); 1.43-1.53 (m, 1H); 2.55 (2, 1H); 5.52 (s, 1H); 6.76 (d, J = 8 Hz, 1H); 7.22 (dd, J = 2, 8 Hz, 1H); 7.47 (d, J = 2 Hz, 1H), 8.44 (s, 1H).

EXAMPLE 50

6-Chloro-4-cyclopropyl-3-methyl-4-(3-propenyloxymethyl)-3,4-dihydroquinazolin-2(1H)-one

Step A: 6-Chloro-4-cyclopropyl-4-(3-propenyloxymethyl)-1-(4-methoxybenzyl)-3,4-dihydroquinazolin-2(1H)-one

The product from Example 51, step C (214 mg, 0.58 mmol), in 3 mL of anhydrous DMF was treated with 75 mg (1.87 mmol) of sodium hydride (60% dispersion in oil) under argon. After 5 min the mixture was treated with 0.10 mL (0.98 mmol) of 1,3-dibromopropane and stirring was continued at room temperature for 24 h. The mixture was poured into 50 mL cold aqueous saturated $NH_4Cl$ solution and extracted twice with EtOAc. The combined extracts were washed successively with water and brine and then dried over $MgSO_4$. Evaporation of the EtOAc yielded a residue which was flash chromatographed on silica gel to afford 26 mg of O, N-dialky-

lated material:
$^1$H NMR ($CDCl_3$): δ 0.25-0.70 (m,4); 1.4-1.26 (m, 1H); 3.65 (d, $J_A$ = 11 Hz, $1H_A$); 3.73 (d, $J_B$ =11 Hz, $1H_B$); 3.78 (s, 3H); 3.93 (m, 1H); 4.25-4.58 (m, 2H); 4.95-5.31 (m, 6H); 5.78-5.92 (m, 1H); 6.03-6.20 (m, 1H); 6.70 (d, J = 8.6 Hz, 1H); 6.83 (d, J = 8.6 Hz, 2H); 7.07 (dd, J = 2, 8.6 Hz, 1H); 7.20 (d, J = 8.6 Hz, 2H); 7.27 (d, J = 2 Hz, 1H). Continued elution of the column provided 110 mg of the title compound: $^1$H NMR ($CDCl_3$): δ 0.24-0.65 (m, 4H), 01.23-1.41 (m, 1H); 3.59 (d, $J_A$ =9 Hz, $1H_A$); 3.67 (d, $J_B$ =9 Hz, $1H_B$); 3.77 (s, 3H); 4.01 (d, J = 5 Hz, 2H); 5.01 (d, $J_A$ = 18 Hz, $1H_A$); 5.08 (d, $J_B$ =18 Hz, $1H_B$); 5.17-5.28 (m, 3H); 5.43 (s, 1H); 5.79-5.92 (m, 1H); 6.69 (d, J = 8.7 Hz, 1H); 6.83 (d, J = 8.7 Hz, 2H); 7.06 (dd, J = 2, 8.7 Hz, 1H); 7.05-7.24 (m, 3H).

Step B: 6-Chloro-4-cyclopropyl-4-(3-propenyloxymethyl)-3-methyl-1-(4-methoxybenzyl)-3,4-dihydroquinazolin-2(1H)-one

The title compound was prepared in a manner according to Example 48, step A, from the 110 mg of title compound of Step A. There was obtained 110 mg of crude title compound which was used in step C without further purification:
$^1$H NMR ($CDCl_3$): δ 0.11-0.30 (m, 2H); 0.48-0.58 (m, 2H); 1.03-1.15 (m, 1H), 3.73 (d, $J_A$ =11 Hz, $1H_A$); 3.81 (d, $J_B$ =11 Hz, $1H_B$); 3.76 (s, 3H); 3.96 (d, $J_A$ =1 Hz, $1H_A$); 3.98 (d, $J_B$ =1 Hz, $1H_B$); 5.78-5.91 (m, 1H); 6.67 (d, J = 8.7 Hz, 1H); 6.82 (d, J = 8.7 Hz, 2H); 7.04 (dd, J = 2, 8.7 Hz, 1H); 7.13-7.22 (m, 3H).

Step C: 6-Chloro-4-cyclopropyl-3-methyl-4-(3-propenyloxymethyl)-3,4-dihydroquinazolin-2(1H)-one

The product from Step A (110 mg), was treated with trifluoroacetic acid in dichloromethane in a manner according to Example 14, Step C and the oily solid isolated upon workup was purified by flash chromatography on silica gel (1:1 EtOAc/hexane), to yield 35 mg of the title compound, mp 132.5-133°C:
$^1$H NMR ($CDCl_3$): δ 0.21-0.32 (m, 2H); 0.45-0.60 (m, 2H); 1.03-1.15 (m, 1H); 3.18 (s, 3H); 3.75 (d, $J_A$ =11 Hz, $1H_A$); 3.81 (d, $J_B$ =11 Hz, $1H_B$); 5.18-5.30 (m, 2H); 5.75-5.90 (m, 1H); 6.63 (d, J = 8.5 Hz, 1H); 7.10-7.18 (m, 2H); 7.90 (br s, 1H).

EXAMPLE 51

(+/-) 6-Chloro-4-cyclopropyl-4-(2-ethoxymethyl)-3-ethyl-3,4-dihydroquinazolin-2(1H)-one

Step A: Chloromagnesiomethyl(diisopropoxy)methyl silane

Freshly distilled chloromethyl(diisopropoxy) methylsilane (39.2 g, 186 mmol) was converted to the Grignard reagent by reaction with magnesium powder (5.20 g, 214 mmol) in THF initiated by the addition of dibromoethane (100 ml) at room temperature. The reaction mixture was stirred for 24h to afford a 1M THF solution of chloromagnesiomethyl(diisopropoxy) methylsilane.

Step B: 6-chloro-4-cyclopropyl-3,4-dihydro-1-(4-methyoxybenzyl)-4-[methyl(diisopropoxy)silylmethyl]quinazolin-2(1H)-one

A suspension of 12.3 g (36.1 mmol) of 6-chloro-4-cyclopropyl-1-(4-methyoxybenzyl)quinazolin-2(1H)-one and magnesium bromide etherate (10.25g, 39.7 mmol) in 250 mL of dry diethyl ether was vigorously stirred at room temperature under argon prior to the addition of 1M THF solution of the Grignard reagent from Step A at room temperature. The reaction was stirred for 3h before quenching into cold1M HCl. The resulting mixture was extracted with two portions of ethyl acetate and the organic layers washed with water, dried over $Na_2SO_4$ then filtered and concentrated to dryness to afford a residue which was dissolved in 1:1 diethyl ether-petroleum ether. The insolubles (1.48 g) were removed via filtration and the filtrate evaporated to give the title compound as a foam (15.52 g, 83% yield).

Step C: 6-chloro-4-cyclopropyl-3,4-dihydro-1-(4-methyoxybenzyl)-4-[hydroxymethyl]quinazolin-2(1H)-one

Oxidation of 6-chloro-4-cyclopropyl-3,4-dihydro-1-(4-methyoxybenzyl)-4-[methyl(diisopropoxy)silylmethyl] quinazolin-2(1H)-one (3.62 g, 7.0 mmol) in 25 ml DMF using peracetic acid (14.7 ml of 32% in acetic acid, 70 mmol) and potassium fluoride (2.03g, 35 mmol) was complete after 24h at room temperature. The desired product was isolated as a light yellow foam in high yield (2.55 g) following aqueous workup using sodium thiosulfate, sodium bicarbonate, water and brine.

NMR ($CDCl_3$): δ 0.23-0.65(m, 4H), 1.21-1.32(m, 1H), 2.78(br s, 1H), 3.80(s, 2H), 4.84(s, 1H), 5.04(s, 2H), 5.83(s, 1H), 6.71(d, J=8.8 Hz, 1H) 6.83(d, J=8.6 Hz, 2H), 7.08(d J=7.5 Hz, 1H),7.16(dd, J=8.9, 4.0 Hz, 3H).

Step D: 6-Chloro-4-cyclopropyl-4-(2-ethoxymethyl)-3-ethyl-1-(4-methoxybenzyl)-3,4-dihydroquinazolin-2(1H)-one

The title compound was prepared from 6-chloro-4-cyclopropyl-4-(2-hydroxymethyl)-1-(4-methoxybenzyl)-3,4-dihydroquinazolin-2(1H)-one (1.05g, 2.93 mmol) after treatment with sodium hydride (470mg 60%in mineral oil, 11.7mmol) and ethyl iodide (1.17ml, 14.65mmol) in DMF. Aqueous extractive workup similiar to that of Example 14, Step B afforded 1.25g of an oil carried onto Step B.

Step E: 6-Chloro-4-cyclopropyl-4-(2-ethoxymethyl)-3-ethyl-3,4-dihydroquinazolin-2(1H)-one

In a manner according to Example 14, step C, the title compound was prepared from 6-chloro-4-cyclopropyl-4-(2-ethoxymethyl)-3-ethyl-1-(4-methoxybenzyl)-3, 4-dihydroquinazolin-2(1H)-one (210 mg, 0.226 mmol) using 50% trifluoroacetic acid in methylene chloride to give 250 mg (23%, two steps) of a white solid after silica gel chromatography (30:70 ethyl acetate-hexane) and recrystallization from ethyl acetate-hexane:
$^1$H NMR ($CDCl_3$): δ 0.39 (d, 2H); 0.55 (m, 2H); 0.95 (m,1H), 1.16 (t, J=6.9 Hz, 3H); 1.30 (t, J=6.9 Hz, 3H); 3.48 (q, J=6.95 Hz, 2H) 3.74 (d, J=2.4, 2H), 3.67 (q, J=4.9 Hz, 2H); 3.88 (q, J=7.1, 2H); 6.66 (d, J=8.3, 1H); 7.15 (dd, J=2.2,8.4, 1H); 7.21 (d, J=2.1, 1H); 7.79 (br s, 1H).
mp: 128-130°C

EXAMPLE 52

(+/-) 6-Chloro-4-cyclopropyl-4-(2-ethoxymethyl)-3,4 4-dihydroquinazolin-2(1H)-one

Step A: 6-Chloro-4-cyclopropyl-4-(2-ethoxymethyl)-1-(4-methoxybenzyl)-3,4-dihydroquinazolin-2(1H)-one

The title compound was prepared from 6-chloro-4-cyclopropyl-4-(2-hydroxymethyl)-1-(4-methoxybenzyl)-3,4-dihydroquinazolin-2(1H)-one (2.80 g, 7.55 mmol) after treatment with sodium hydride (755 mg 60% in mineral oil, 18.9 mmol) and ethyl iodide (1.0 ml, 12.5 mmol) in DMF (30 ml). Aqueous extractive workup of a portion of the DMF reaction mixture (5ml) was carried out as described in Example 14, Step B to afford 220 mg of an oil after silica gel chromatography.

Step B: 6-Chloro-4-cyclopropyl-4-(2-ethoxymethyl)-3,4-dihydroquinazolin-2(1H)-one

In a manner according to Example 14, step C, the title compound was prepared from 6-chloro-4-cyclopropyl-4-(2-ethoxymethyl)-1-(4-methoxybenzyl)-3,4-di-hydroquinazolin-2(1H)-one (220 mg, 0.55 mmol) using 50% trifluoroacetic acid in methylene chloride to give 75 mg of a white solid after silica gel chromatography (30:70 ethyl acetate-hexane) and recrystallization from ethyl acetate-hexane:
$^1$H NMR ($CDCl_3$): δ 0.35 (m, 1H); 0.45 (m, 2H); 0.58 (m,1H), 1.22 (t, J=6.8 Hz, 3H); 1.33(m, 1H); 3.56 (m, 2H) 3.66, 3.72 (dd, J=9.0, 34, 2H), 5.41(s, 1H), 6.71 (d, J=8.4, 1H); 7.17 (m, 2H); 8.57 (br s, 1H).
mp: 172-173°C

EXAMPLE 53

(+/-) 6-Chloro-4-cyclopropyl-4-(methoxymethyl)-3-methyl-3,4-dihydroquinazolin-2(1H)-one

Step A: 6-Chloro-4-cyclopropyl-4-(methoxymethyl)-3-methyl-1-(4-methoxybenzyl)-3,4-dihydroquinazolin-2(1H)-one

The title compound was prepared from 6-chloro-4-cyclopropyl-4-(2-hydroxymethyl)-1-(4-methoxybenzyl)-3 4-dihydroquinazolin-2(1H)-one (340 mg, 0.92 mmol) after treatment with sodium hydride (110 mg 60% in mineral oil, 2.76mmol) and methyl iodide (285 ml, 4.59 mmol) in DMF. Aqueous extractive workup similiar to that of Example 14, Step B afforded 700 mg of an oil.

Step B: 6-Chloro-4-cyclopropyl-4-(methoxymethyl)-3-ethyl-3,4-dihydroquinazolin-2(1H)-one

In a manner according to Example 14, step C, the title compound was prepared from 6-chloro-4-cyclopropyl-4-(methoxymethyl)-3-methyl-1-(4-methoxybenzyl)-3, 4-dihydroquinazolin-2(1H)-one (210mg, 0.226 mmol) using 20% trifluoroacetic acid in methylene chloride overnight to give 107 mg (30%, two steps) of a white solid after silica gel chromatography (30:70 ethyl acetate-hexane) and recrystallization from ethyl acetate-hexane:
$^1$H NMR ($CDCl_3$): δ 0.23 (m, 2H); 0.51 (m, 2H); 1.10 (m,1H), 3.19 (s, 3H) 3.37(s, 3H), 3.80 (dd, J=11.0, 18.4 Hz, 2H);6.68 (d, J=8.3, 1H); 7.11(d, J=2.2, 1H); 7.16 (dd, J=2.2,8.4, 1H); 8.17 (br s, 1H).
mp: 194-196°C

EXAMPLE 54

(+/-) 6-Chloro-4-cyclopropyl-4-(2-propynyloxymethyl)-3-(2-propynyl)-3,4-dihydroquinazolin-2(1H)-one

Step A: 6-Chloro-4-cyclopropyl-4-(2-propynyloxymethyl)-3-(2-propynyl)-1-(4-methoxybenzyl)-3,4-dihydroquinazolin-2(1H)-one

The title compound was prepared from 6-chloro-4-cyclopropyl-4-(2-hydroxymethyl)-1-(4-methoxybenzyl)-3,4-dihydroquinazolin-2(1H)-one (300 mg, 0.845 mmol) after treatment with sodium hydride (106 mg 60% in mineral oil, 2.54 mmol) and propargyl bromide (841 ml, 8.45 mmol) in DMF. Aqueous extractive workup similiar to that of Example 14 Step B afforded 450 mg of an oil.

Step B: 6-Chloro-4-cyclopropyl-4-(2-propynyloxymethyl)-3-(2-propynyl)-3,4-dihydroquinazolin-2(1H)-one

In a manner according to Example 14, step C, the title compound was prepared from 6-chloro-4-cyclopropyl-4-(propynyloxymethyl)-3-(2-propynyl)-1-(4-methoxybenzyl)-3,4-dihydroquinazolin-2(1H)-one (210 mg, 0.226 mmol) using 50% trifluoroacetic acid in methylene chloride to give 75 mg (26%, two steps) of a white solid after silica gel chromatography (30:70 ethyl acetate-hexane) and crystallization from diethyl ether-petroleum ether:
$^1$H NMR ($CDCl_3$): δ 0.33 (m, 2H); 0.58 (m, 2H); 1.54 (m,1H), 2.26(t,J=2.4Hz, 1H); 2.5(t,J=2.4Hz, 1H); 5(4.02 (q, J=11.0 Hz, 2H); 4.19 (d, J=2.3 Hz, 2H); 4.30(dd, J=2.5, 18.2 Hz, 1H); 4.78 (dd, J=2.5, 18.0 Hz, 1H); 5.18 (m, 4H);.6.70 (d, J=8.5, 1H); 7.18 (dd, J=2.2,8.4, 1H) 7.22(d, J=2.1, 1H); 8.00 (br s, 1H).
mp: 124-126°C

EXAMPLE 55

(+/-) 6-Chloro-4-cyclopropyl-4-(2-propenyloxymethyl)-3-(2-propenyl)-3,4-dihydroquinazolin-2(1H)-one

Step A: 6-Chloro-4-cyclopropyl-4-(2-propenyloxymethyl)-3-(2-propenyl)-1-(4-methoxybenzyl)-3,4-dihydroquinazolin-2(1H)-one

The title compound was prepared from 6-chloro-4-cyclopropyl -4-(2-hydroxymethyl)-1-(4-methoxy-benzyl)-3,4-dihydroquinazolin-2(1H)-one (250 mg, 0.71 mmol) after treatment with sodium hydride (84.2 mg 60% in mineral oil, 2.11 mmol) and allyl bromide (610 μl, 7.05 mmol) in DMF. Aqueous extractive workup similiar to that of Example 14, Step B afforded 500 mg of an oil.

Step B: 6-Chloro-4-cyclopropyl-4-(2-propenyloxymethyl)-3-(2-propenyl)-3,4-dihydroquinazolin-2(1H)-one

In a manner according to Example 14, step C, the title compound was prepared from 6-chloro-4-cyclopropyl-4-(propenyloxymethyl)-3-(2-propenyl)-1-(4-methoxy benzyl)-3,4-dihydroquinazolin-2(1H)-one (210mg, 0.226 mmol) using 30% trifluoroacetic acid in methylene chloride to give 70 mg (30%, two steps) of a white solid after silica gel chromatography (30:70 ethyl acetate-hexane) and recrystallization from methylene chloride-petroleum ether:
$^1$H NMR ($CDCl_3$): δ 0.23 (m, 2H); 0.51 (m, 2H); 1.10 (m,1H), 3.69 (d, J=2.Hz, 2H);.3.92 (m, 2H); 4.35 (dd, J=6.3, 35.7 Hz, 2H); 5.18 (m, 4H); 5.82(m,1H); 6.05 (m,1H), 6.64 (d, J=8.5, 1H); 7.14 (dd, J=2.2,8.4, 1H) 7.22(d, J=2.2, 1H); 7.89 (br s, 1H).
mp: 121-121.5°C

EXAMPLE 56

(+/-) 6-Chloro-4-cyclopropyl-4-(phenoxymethyl)-3,4-dihydroquinazolin-2(1H)-one

Step A: 6-Chloro-4-cyclopropyl-4-(3-nitrobenzenesulfonyloxymethyl)-1-(4-methoxybenzyl)-3, 4-dihydroquinazolin-2(1H)-one

The title compound was prepared from 6-chloro-4-cyclopropyl -4-(2-hydroxymethyl)-1-(4-methoxybenzyl)-3,4-dihydroquinazolin-2(1H)-one (570 mg, 1.55 mmol) after treatment with 3-nitrobenzenesulfonyl chloride (346 mg, 1.56 mmol) and 4-N,N-dimethylamino- pyridine (191 mg, 1.56 mmol) in methylene chloride at 0°C warming to ambient temperature overnight. Aqueous extractive workup in ethyl acetate using 10% citric acid, sodium bicarbonate, water and brine afforded 540 mg of an oil after concentrating to dryness. After silica gel chromatography (1:1 ethyl acetate-hexane) 250 mg of the title compound was isolated as a colorless solid.

Step B: 6-Chloro-4-cyclopropyl-4-(phenoxymethyl)-1-(4-methoxybenzyl)-3,4-dihydroquinazolin-2(1H)-one

The reaction of 6-chloro-4-cyclopropyl-4-(3-nitrobenzene- sulfonyloxymethyl)-1-(4-methoxybenzyl)-3,4-dihydroquinazolin-2(1H)-one (110 mg, 0.197 mmol) with phenol (70.7 mg, 0.751 mmol) and cesium carbonate (245 mg, 0.751 mmol) was carried out overnight at 80°C in dioxane. Aqueous extractive workup using 10% sodium hydroxide, water and brine afforded 120 mg crude product which was purified by column chromatography to afford 75 mg of the title compound.

Step C: 6-Chloro-4-cyclopropyl-4-(phenoxymethyl)-3,4-dihydroquinazolin-2(1H)-one

In a manner according to Example 14, step C, the title compound was prepared from 6-chloro-4-cyclopropyl-4-(phenoxymethyl)-1-(4-methoxybenzyl)-3,4-dihydro quinazolin-2(1H)-one (75 mg, 0.167 mmol) using 50% trifluoroacetic acid in methylene chloride to give 25 mg of a white amorphous solid after purifying the crude product by preparative HPLC (C18 reverse-phase) and lyophilizing from dioxane-water:
$^1$H NMR ($CDCl_3$): δ 0.39-0.56 (m, 4H); 0.51 (m, 2H); 1.45 (m,1H); 4.11-4.17(m,2H); 5.53(s,1H); 6.60-7.42(m, 8H); 8.65 (br s, 1H)

EXAMPLE 57

(+/-) 6-Chloro-4-(2-furyl)-3-methyl-4-(2-propenyl)-3,4-4-dihydroquinazolin-2(1H)-one

Step A: 6-Chloro-4-(2-furyl)-quinazolin-2(1H)-one

In a manner according to Example 72, step A., the title compound was prepared from 3.0 g (19.7 mmol) 5-chloroanthranilonitrile and 98.3 mmol 2-furyllithium in THF to give 4 g of a solid.

Step B: 6-Chloro-4-(2-furyl)-4-(2-propenyl)-3,4-dihydroquinazolin-2(1H)-one

In a manner according to Example 14, step A, the title compound was prepared from 6-chloro-4-(2-furyl)-quinazolin-2(1H)-one (100 mg, 0.405 mmol) to give 78 mg of an oil.

Step C: 6-Chloro-4-(2-furyl)-1-(4-methoxybenzyl)-4-(2-propenyl)-3,4-dihydroquinazolin-2(1H)-one

In a manner according to Example 25, step C, the title compound was prepared from 6-chloro-4-(2-furyl)-4-(2-propenyl)-3,4-dihydroquinazolin-2(1H)-one (78 mg, 0.270 mmol) to give 39 mg of an oil.

Step D: 6-Chloro-4-(2-furyl)-1-(4-methoxybenzyl)-3-methyl-4-(2-propenyl)-3,4-dihydroquinazolin-2(1H)-one

In a manner according to Example 14, step B, the title compound was prepared from 6-chloro-4-(2-furyl)-1-(4-methoxybenzyl)-4-(2-propenyl)-3,4-dihydroquinazolin-2(1H)-one (39 mg, 0.090 mmol) to give 53 mg of an oil.

Step E: 6-Chloro-4-(2-furyl)-3-methyl-4-(2-propenyl)-3,4-dihydroquinazolin-2(1H)-one

In a manner according to Example 14, step C, the title compound was prepared from 6-chloro-4-(2-furyl)-1-(4-methoxybenzyl)-3-methyl-4-(2-propenyl)-3, 4-dihydroquinazolin-2(1H)-one (53 mg, 0.09 mmol) to give 6 mg (22%) of a solid.
$^1$H NMR ($CDCl_3$): δ 2.78 (s, 3H); 2.74-2.85 (m, 1H); 3.09 (dd, J=10,16.5, 1H); 5.05-5.17 (m, 2H); 5.53-5.69 (m, 1H); 6.40 (dd, J=1.8,3.3, 1H); 6.47 (d, J=3.3, 1H); 6.66 (d, J=2.2, 1H); 6.70 (d, J=8.4, 1H); 7.10 (dd, J=2.2,8.4, 1H); 7.40 (t, J=0.8, 1H); 8.66 (br s, 1H). (amorphous)

EXAMPLE 58

6-Chloro-3,4-dihydro-4-ethynyl-4(2-propenyl)quinazolin-2(1H)-one

Step A: 4-Chloro-2(3-triisopropylsilylpropynoyl)aniline

(Triisopropylsilyl)acetylene (5.05 ml, 22.51 mmol) was added dropwise over 5 min to a stirred solution of butyllithium (2.5 M solution in hexanes, 7.5 ml, 18.75 mmol) in dry THF (22.5 ml) at -78°C under argon. The solution was warmed to 0°C and a solution of 6-amino-3-chloro-N-methoxy-N-methylbenzamide (1.61 g, 7.50 mmol) in dry THF (7.5 ml) was added dropwise over 5 min. Residual amide was washed in with dry THF (2 ml) and after 1 h saturated ammonium chloride solution (10 ml) and water (10 ml) were added. The mixture was extracted with ethyl acetate (300 ml) and was washed with brine, dried ($Na_2SO_4$) and evaporated in vacuo to a dark orange oil. The crude product was purified by flash column chromatography on silica (ethyl acetate/hexane gradient, 0-10% ethyl acetate) to give the title compound (2.34 g, 93%) as a bright yellow oil:
$^1$H NMR ($CDCl_3$): δ 1.13-1.21 (m, 21 H), 5.80 (br s, 2 H), 6.61 (d, J = 8.8 Hz, 1 H), 7.23 (dd, J = 8.8 and 2.4 Hz, 1 H), 8.16 (d, J = 2.4 Hz, 1 H).

Step B: 6-Chloro-4-triisopropylsilylethynylquinazolin-2(1H)-one

A solution of potassium cyanate (0.949 g, 11.70 mmol) in water (1.4 ml) was added to a stirred solution of 4-chloro-2(3-triisopropylsilylpropynoyl) aniline (1.961 g, 5.84 mmol) in 24:1 glacial acetic acid/water (25 ml) at 0°C. The solution was stirred for 1 h and was warmed to RT. After a further 1 h, potassium cyanate (0.953 g, 11.74 mmol) was added. After 2 h, the mixture was diluted with ethyl acetate (150 ml) and was washed with water (3x40 ml) and brine, dried ($Na_2SO_4$) and evaporated in vacuo azeotroping with toluene (3x50 ml), to a yellow oil. The crude product was purified by flash column chromatography on silica (methanol/chloroform gradient, 0-4% methanol) to give a yellow solid. The solid was crystallised from boiling hexane to give the title compound (0.735 g, 35%) as bright yellow crystals: mp 202-204°C (dec);
$^1$H NMR ($CDCl_3$): δ 1.16-1.30 (m, 21 H), 7.48 (d, J = 8.8 Hz, 1 H), 7.68 (dd, J = 8.8 and 2.1 Hz, 1 H), 8.12 (d, J = 2.1 Hz, 1 H), 13.21 (br s, 1 H).

Step C: 6-Chloro-3,4-dihydro-4(2-propenyl)-4-triisopropylsilylethynylquinazolin-2(1H)-one

Allylmagnesium bromide (1 M solution in diethyl ether, 3.2 ml) was added dropwise to a stirred solution of 6-chloro-4-triisopropylsilylethynylquinazolin-2(1H)-one (463 mg, 1.28 mmol) in dry THF (6.4 ml) at 0°C under argon. After 20 min, 1 M hydrochloric acid (5 ml) was added and the mixture was extracted with ethyl acetate (50 ml), washed with saturated sodium hydrogen carbonate solution and brine, dried ($Na_2SO_4$) and evaporated in vacuo to a glass. The crude product was purified by flash column chromatography on silica (eluting with 0.4% ammonium hydroxide/4% methanol/chloroform) to give the title compound (503 mg, 97%) as a pale yellow glass:
$^1$H NMR ($CDCl_3$): δ 1.06-1.08 (m, 21 H), 2.63 (d, J = 7.6 Hz, 2 H), 5.16 (dd, J = 17.1 and 1.7 Hz, 1 H), 5.25 (dd, J = 10.3 and 1.7 Hz, 1 H), 5.47 (br s, 1 H), 5.81-5.92 (m, 1 H), 6.74 (d, J = 8.5 Hz, 1 H), 7.14 (dd, J = 8.5 and 2.2 Hz, 1 H), 7.40 (d, J = 2.2 Hz, 1 H), 9.33 (br s, 1 H).

Step D: 6-Chloro-3,4-dihydro-4-ethynyl-4(2-propenyl)-quinazolin-2(1H)-one

Tetrabutylammonium fluoride (1M solution in THF, 0.179 ml) was added to a stirred solution of 6-chloro-3,4-dihydro-4(2-propenyl)-4-triisopropylsilylethynylquinazolin-2(1H)-one (66 mg, 0.163 mmol) in dry THF (1.0 ml). After 30 min, saturated ammonium chloride solution (0.5 ml) and water (5 ml) were added and the mixture was extracted with ethyl acetate (20 ml), washed with brine, dried ($Na_2SO_4$) and evaporated in vacuo to a glass.

The crude product was crystallised from methylene chloride/hexane to give the title compound (23.8 mg, 59%): mp: 162-164°C.
$^1$H NMR ($CDCl_3$): δ 2.63 (dd, J = 13.4 and 8.5 Hz, 1 H), 2.67 (s, 1 H), 2.72 (dd, J = 13.4 and 6.3 Hz, 1 H), 5.20 (d, J = 17.1 Hz, 1 H), 5.29 (d, J = 10.0 Hz, 1 H), 5.35 (br s, 1 H), 5.79-5.90 (m, 1 H), 6.69 (d, J = 8.5 Hz, 1 H), 7.20 (dd, J = 8.5 and 2.3 Hz, 1 H), 7.39 (d, J = 2.3 Hz, 1 H), 7.72 (br s, 1 H).

EXAMPLE 59

6-Chloro-3,4-dihydro-4-ethynyl-3-methyl-4(2-propenyl)-quinazolin-2(1H)-one

Step A: 6-Chloro-3,4-dihydro-1(4-methoxybenzyl)-4(2-propenyl)-4-triisopropylsilylethynylquinazolin-2(1H)-one

Sodium hydride (35 mg, 1.46 mmol) was added to a stirred solution of 6-chloro-3,4-dihydro-4(2-propenyl)-4-triisopropylsilylethynylquinazolin-2(1H)-one (464 mg, 1.15 mmol), sodium iodide (16 mg, 0.11 mmol) and 4-methoxybenzyl chloride (0.156 ml, 1.15 mmol) in dry DMF (5 ml). After 1 h more sodium hydride (24 mg, 1.00 mmol) was added. After a further 1 h, 1 M hydrochloric acid (5 ml) was added and the mixture was extracted with ethyl acetate, washed with water (3x20 ml), sodium hydrogen carbonate solution and brine, dried ($Na_2SO_4$) and evaporated in vacuo. The resulting oil was purified by flash column chromatography on silica (dry loaded, eluting with an ethyl acetate/hexane gradient, 5-15% ethyl acetate) to give the title compound (165 mg, 32%) as an oil:
$^1$H NMR ($CDCl_3$): δ 1.10 (s, 21 H), 2.57-2.71 (m, 2 H), 3.77 (s, 3 H), 5.08 (d, J = 18.3 Hz, 1 H), 5.10 (d, J = 18.3 Hz, 1 H), 5.17 (dd, J = 17.1 and 1.7 Hz, 1 H), 5.27 (dd, J = 10.3 and 1.7 Hz, 1 H), 5.79-5.92 (m, 1 H), 6.73 (d, J = 8.6 Hz, 1 H), 6.84 (dd, J = 6.6 and 2.0 Hz, 2 H), 7.10 (dd, J = 8.6 and 2.4 Hz, 1 H), 7.17 (dd, 6.6 and 2.0 Hz, 2 H), 7.45 (d, J = 2.4 Hz, 1 H).

Step B: 6-Chloro-3,4-dihydro-4-ethynyl-3-methyl-4 (2-propenyl)quinazolin-2(1H)-one

Sodium hydride (11 mg, 0.458 mmol) was added to a stirred solution of 6-chloro-3,4-dihydro-1(4-methoxybenzyl)-4(2-propenyl)-4-triisopropylsilylethynylquinazolin-2(1H)-one (160 mg, 0.306 mmol) and methyl iodide (0.021 ml, 0.337 mmol) in dry DMF (1.5 ml). After 30 min, 1 M hydrochloric acid (1.0 ml) was added and the mixture was extracted with ethyl acetate, washed with water, saturated sodium hydrogen carbonate solution and brine, dried ($Na_2SO_4$) and evaporated in vacuo to an oil. The crude product was dissolved in dry THF (1.0 ml) and tetrabutylammonium fluoride (1 M solution in THF, 0.4 ml) was added with stirring. After 30 min, saturated ammonium chloride solution (1.0 ml) and water (5 ml) were added and the mixture was extracted with ethyl acetate (20 ml), washed with water and brine, dried ($Na_2SO_4$) and evaporated in vacuo. The resulting oil was purified by flash column chromatography on silica (dry loaded, eluting with an ethyl acetate/hexane gradient, 10-40% ethyl acetate) to give a colorless glass. This product was dissolved in dry methylene chloride (4 ml) and trifluoroacetic acid (2 ml) was added with stirring. After 16 h, the solution was evaporated in vacuo. The residue was dissolved in ethyl acetate and was washed with saturated sodium hydrogen carbonate solution and brine, dried ($Na_2SO_4$) and evaporated in vacuo. The resulting glass was purified by flash column chromatography on silica (dry loaded, eluting with an ethyl acetate/hexane gradient, 20-50% ethyl acetate) to give a colorless solid, which was crystallised from methylene chloride/hexane to give the title compound (49 mg, 61%): mp 165-166°C;
$^1$H NMR ($CDCl_3$): δ 2.62 (dd, J = 13.9 and 6.6 Hz, 1 H), 2.75-2.83 (m, 1 H), 2.79 (s, 1 H), 3.22 (s, 1 H), 4.98-5.11 (m,.2 H), 5.50-5.66 (m, 1 H), 6.73 (d, J = 8.5 Hz, 1 H), 7.16 (dd, J = 8.5 and 2.2 Hz, 1 H), 7.39 (d, J = 2.2 Hz, 1 H), 9.18 (br s, 1 H).

EXAMPLE 60

6-Chloro-4-cyclopropylmethyl-3,4-dihydro-4-ethynylquinazolin-2(1H)-one

1-Methyl-3-nitro-3-nitrosoguanidine (1.47 g, 10.0 mmol) was added portionwise to a stirred mixture of diethylether (15 ml) and 40% potassium hydroxide (4.5 ml) at 0°C. After 15 min the resulting yellow ether solution of diazomethane (8 ml) was added to a stirred solution of 6-chloro-3,4-dihydro-4(2-propenyl)-4-triisopropylsilylethynylquinazolin-2(1H)-one (236 mg, 0.586 mmol) in ether (4 ml) at 0°C. Palladium acetate (3 mg, 0.013 mmol) was added and after 20 min, more diazomethane solution (1 ml) was added. After a further 20 min, glacial acetic acid (2 ml) was added and the solution was diluted with ethyl acetate, washed with water,

saturated sodium hydrogen carbonate solution and brine; dried ($Na_2SO_4$) and evaporated in vacuo to a glass. The crude product was dissolved in dry THF (4 ml) and tetrabutylammonium fluoride (1 M solution in THF, 0.6 ml) was added with stirring. After 15 min, saturated ammonium chloride solution (1 ml) and water (5 ml) were added and the mixture was extracted with ethyl acetate, washed with brine, dried ($Na_2SO_4$) and evaporated in vacuo. The resulting oil was purified by flash column chromatography on silica (dry loaded, eluting with an ethyl acetate/hexane gradient, 10-100% ethyl acetate) to give the title compound (59.1 mg, 39%) as a glass:
$^1$H NMR ($CDCl_3$): δ 0.06-0.15 (m, 1 H), 0.45-0.58 (m, 1 H), 0.80-0.92 (m, 1 H), 1.75 (dd, J = 13.9 and 7.6 Hz, 1 H), 1.99 (dd, J = 13.9 and 5.85 Hz, 1 H), 2.66 (s, 1 H), 5.59 (br s, 1 H), 6.69 (d, J = 8.5 Hz, 1 H), 7.18 (dd, J = 8.5 and 2.2 Hz, 1 H), 7.40 (d, J = 2.2 Hz, 1 H), 8.07 (br s, 1 H).

EXAMPLE 61

6-Chloro-3,4-dihydro-4-ethynyl-4-propylquinazolin-2-(1H)-one

Step A: 6-Chloro-3,4-dihydro-4-propyl-4-triisopropylsilylethynylquinazolin-2(1H)-one

Propylmagnesium chloride (2 M solution in diethylether, 1.5 ml), was added dropwise over 15 minutes to a stirred mixture of 6-chloro-4-triisopropylsilylethynylquinazolin-2(1H)-one (361 mg, 1.00 mmol) and magnesium bromide etherate (516 mg, 2.00 mmol) in dry diethylether (5 ml) at 0°C. After 30 min the mixture was poured into 1 M hydrochloric acid solution (5 ml), extracted with ethyl acetate (10 ml) and was washed with saturated sodium hydrogen carbonate solution (2x10 ml) and brine (10 ml), dried ($Na_2SO_4$) and evaporated in vacuo to give the title compound as a yellow oil (330 mg, 88%):
$^1$H NMR ($CDCl_3$): δ 0.90 (t, J = 7.3 Hz, 3 H), 1.08 (s, 21 H), 1.39-1.55 (m, 2 H), 1.79-1.95 (m, 2 H), 5.50 (br s, 1 H), 6.72 (d, J = 8.5 Hz, 1 H), 7.14 (dd, J = 8.5 and 2.2 Hz, 1 H), 7.39 (d, J = 2.2 Hz, 1 H), 8.91 (br s, 1 H).

Step B: 6-Chloro-3,4-dihydro-4-ethynel-4-propylquinazolin-2(1H)-one

Tetrabutylammonium fluoride (1M solution in THF, 0.087 ml) was added to a stirred solution of 6-chloro-3,4-dihydro-4-propyl-4-triisopropylsilylethynylquinazolin-2(1H)-one (30 mg, 0.079 mmol) in dry THF (1 ml). After 10 minutes the reaction was quenched with a saturated ammonium chloride solution (10 ml) and was extracted into ethyl acetate, washed with water (5 ml) and brine (5 ml), dried ($Na_2SO_4$) and evaporated in vacuo. The resulting yellow oil was triturated with hexanes to give the title compound (13 mg, 66%) as a crystalline solid: mp 87-87.5°C;
$^1$H NMR ($CDCl_3$): δ 0.92 (t, J = 7.3 Hz, 3 H), 1.35-1.52 (m, 2 H), 1.80-2.18 (m, 2 H), 2.64 (s, 1 H), 5.37 (br s, 1 H), 6.68 (d, J = 8.5 Hz, 1 H), 7.19 (dd, J = 8.5 and 2.1 Hz, 1 H), 7.37 (d, J = 2.1 Hz, 1 H), 7.69 (br s, 1 H).

EXAMPLE 62

6-Chloro-3,4-dihydro-4-ethynyl-4-propan-2-ylquinazolin-2(1H)-one

Isopropylmagnesium chloride (2 M solution in diethyl ether, 1.0 ml) was added to a stirred mixture of 6-chloro-4-triisopropylsilylethynylquinazolin-2(1H)-one (180 mg, 0.50 mmol) and magnesium bromide etherate (258 mg, 1.00 mmol) in dry diethyl ether (2.5 ml) at 0°C under argon. After 30 min the mixture was warmed to RT for 15 min. 1 M hydrochloric acid (5 ml) was added at 0°C and after stirring for 15 min was extracted with ethyl acetate (30 ml), washed with saturated sodium hydrogen carbonate solution and brine, dried ($Na_2SO_4$) and evaporated in vacuo. The resulting oil was dissolved in dry THF (5 ml) and tetrabutylammonium fluoride (1 M solution in THF, 0.6 ml) was added with stirring. After 15 min, saturated ammonium chloride solution (2 ml) and water (2 ml) were added and the mixture was extracted with ethyl acetate (20 ml), washed with water and brine, dried ($Na_2SO_4$) and evaporated in vacuo. The resulting oil was purified by flash column chromatography on silica (dry loaded, eluting with an ethyl acetate/hexane gradient, 10-100% ethyl acetate). The resulting glass was further purified by preparative HPLC (C-18, acetonitrile/water/0.1% TFA) to give the title compound (51.5 mg, 41%) as a colorless glass:
$^1$H NMR ($CDCl_3$): δ 0.94 (d, J = 6.7 Hz, 1 H), 1.05 (d, J = 6.7 Hz, 1 H), 2.12 (septet, J = 6.7 Hz, 1 H), 2.65 (s, 1 H), 5.43 (br s, 1 H), 6.68 (d, J = 8.5 Hz, 1 H), 7.18 (dd, J = 8.5 and 2.2 Hz, 1 H), 7.37 (d, J = 2.2 Hz, 1 H), 7.91 (br s, 1 H).

EXAMPLE 63

9-Chloro-1,11-dihydro-11-ethynyl-3-oxo-oxazolo[4,3-c]- quinazolin-2(1H)-one

Step A: 6-Chloro-3,4-dihydro-4-hydroxymethyl-4-triisopropylsilylethynylquinazolin-2(1H)-one

Diisopropoxy(methyl)silylmethyl magnesium chloride (as a saturated solution in THF, 4.5 ml) was added to a stirred mixture of 6-chloro-4-triisopropylsilylethynylquinazolin-2(1H)-one (1.08 g, 3.00 mmol) and magnesium bromide etherate (1.55 g, 6.00 mmol) in dry diethyl ether (24 ml) at 0°C under argon. Grignard solution (4.5 ml) was added twice more at 15 min intervals, at which point the starting material had been consumed. After a further 14 h, 1 M hydrochloric acid (25 ml) was added and the mixture was extracted with ethyl acetate (100 ml), washed with saturated sodium hydrogen carbonate solution and brine, dried ($Na_2SO_4$) and evaporated in vacuo. The resulting oil was purified by flash column chromatography on silica (eluting with an ethyl acetate/hexane gradient, 5-40% ethyl acetate)to give an oil which was dissolved in 32% peracetic acid solution in acetic acid (10 ml) diluted with glacial acetic acid (5 ml). Potassium fluoride (1.05 g, 18.07 mmol) was added at 0°C and after 16 h at room temperature the mixture was diluted with ethyl acetate (100 ml) and was washed with water (3x20 ml), 10% sodium thiosulfate solution (2x20 ml), sodium hydrogen carbonate solution (3x20 ml) and brine, dried ($Na_2SO_4$) and evaporated in vacuo. The resulting gum was triturated with dichloromethane to give the title compound (0.815 g, 69%) as a pale yellow solid: mp 87-92°C;
$^1$H NMR ($d_6$-DMSO): δ 1.01 (s, 21 H), 3.57 (s, 2 H), 5.31 (br s, 1 H), 6.74 (d, J = 8.5 Hz, 1 H), 7.20 (dd, J = 8.5 and 2.4 Hz, 1 H), 7.29 (d, J = 2.4 Hz, 1 H), 9.35 (br s, 1 H).

Step B: 9-Chloro-1,11-dihydro-3-oxo-11-triisopropylsilylethynyloxazolo[4,3-c]quinazolin-2(1H)-one

Carbonyldiimidazole (20.59 mg, 0.127 mmol) was added in one portion to a stirred solution of 6-chloro-3,4-dihydro-4-hydroxymethyl-4-triisopropylsilylethynylquinazolin-2(1H)-one (50 mg, 0.127 mmol) in dry DMF (2 ml) at room temperature. After 22 hours, sodium hydride (97%, 12.56 mg, 0.523 mmol), was added and after an additional 30 min the mixture was poured into ethyl acetate, washed with water (3x15 ml) and brine (2x15 ml), dried ($Na_2SO_4$) and evaporated in vacuo. The resulting solid was purified by flash column chromatography on silica (eluting with 5% methanol/chloroform) to give the title compound (48 mg, 90%) as a crystalline solid: mp 189-190°C;
$^1$H NMR ($CDCl_3$): δ 0.98 (s, 21 H), 4.64 (d, J = 7.9 Hz, 1 H), 4.92 (d, J = 8.2 Hz, 1 H), 6.97 (d, J = 8.5 Hz, 1 H), 7.10 (d, J = 2.1 Hz, 1 H), 7.37 (dd, J = 8.5 and 2.1 Hz, 1 H) 8.55 (br s, 1 H).

Step C: 9-Chloro-1,11-dihydro-11-ethynyl-3-oxo-oxazolo[4,3-c]quinazolin-2(1H)-one

Tetrabutylammonium fluoride (1 M solution in THF, 0.125 ml) was added to a stirred solution of 9-chloro-1,11-dihydro-3-oxo-11-triisopropylsilylethynyloxazolo[4,3-c]quinazolin-2(1H)-one (48 mg, 0.114 mmol) in dry THF (2 ml) at room temperature. After 1 h the reaction was quenched with saturated ammonium chloride solution (10 ml), extracted into ethyl acetate, washed with water (10 ml) and brine (10 ml), dried ($Na_2SO_4$) and evaporated in vacuo. The resulting solid was purified by flash column chromatography on silica (eluting with 10% methanol/chloroform) to give a solid which was recrystallized from ethyl acetate/hexanes to give the title compound (25.4 mg, 83%): mp 234-235°C;
$^1$H NMR ($CDCl_3$ + 2 drops $CD_3OD$): δ 2.80 (s, 1 H), 4.62 (d, J = 8.2 Hz, 1 H), 4.95 (d, J = 8.5 Hz, 1 H), 6.96 (d, J = 8.5 Hz, 1 H), 7.10 (d, J = 2.1 Hz, 1 H), 7.32 (dd, J = 8.5 and 2.1 Hz, 1 H).

EXAMPLE 64

6-Chloro-3,4-dihydro-4-ethynyl-4-hydroxymethylquinazolin-2(1H)-one

Tetrabutylammonium fluoride (1 M solution in THF 0.895 ml) was added to a stirred solution of 6-chloro-3,4-dihydro-4-hydroxymethyl-4-triisopropylsilylethynylquinazolin-2(1H)-one, (320 mg, 0.814 mmol) in THF (2 ml). After 20 min the reaction was quenched with saturated ammonium chloride solution (5 ml), extracted with ethyl acetate (15 ml), washed with water (10 ml) and brine (10 ml), dried ($Na_2SO_4$) and evaporated in vacuo. The crude residue was triturated with cold methylene chloride to give the title compound (170 mg, 89%): mp 268-269°C;
$^1$H NMR ($d_6$-DMSO): δ 3.54-3.67 (m, 3 H), 5.38 (t, J = 6.1 Hz, 1 H), 6.78 (d, J = 8.5 Hz, 1 H), 7.23 (dd, J = 8.5 and 2.1 Hz, 1 H), 7.30 (d, J = 2.1 Hz, 1 H), 7.36 (s, 1 H), 9.41 (br s, 1 H).

EXAMPLE 65

9-Chloro-1,11-dihydro-11-ethynyloxazolo[4, 3-c]quinazolin-2(1H)-one

6-Chloro-3,4-dihydro-4-ethynyl-4-hydroxymethylquinazolin-2(1H)-one (60 mg, 0.253 mmol) was added to a solution of formaldehyde (37 wt.% in water, 1 ml) and formic acid, (96%, 1 ml) and the mixture was heated at 95-100°C for 3 h. The mixture was cooled, basified with saturated sodium hydrogen carbonate solution, extracted into ethyl acetate, washed with brine (2x10 ml), dried ($Na_2SO_4$) and evaporated. Methanol (5 ml) and 1M NaOH (1 ml) were added to the residue and after stirring for 0.5 h the solvent volume was reduced in vacuo and the residue was extracted into ethyl acetate, washed with brine, dried ($Na_2SO_4$) and evaporated in vacuo. The crude residue was purified by flash column chromatography on silica (eluting with 10% methanol/chloroform) to give the title compound as a crystalline solid (56 mg, 90%): mp 201-203°C;
$^1H$ NMR ($CDCl_3$): δ 2.60 (s, 1 H), 4.00 (d, J = 7.63 Hz, 1 H), 4.60 (d, J = 7.63 Hz, 1 H), 5.12 (d, J = 4.27 Hz, 1 H), 5.48 (d, J = 4.27 Hz, 1 H), 6.82 (d, J = 8.55 Hz, 1 H), 7.18 (d, J = 2.14 Hz, 1 H), 7.25 (dd, J = 8.55 and 2.14 Hz, 1 H), 8.55 (br s, 1 H).

EXAMPLE 66

6-Chloro-3,4-dihydro-4-ethyloxymethyl-4-ethynylquinazolin-2(1H) -one

Sodium hydride (16 mg, 0.667 mmol) was added to a stirred solution of 6-chloro-3,4-dihydro-4-ethynyl-4-hydroxy-methylquinazolin-2(1H)-one (48 mg, 0.203 mmol) in dry DMF (0.4 ml). After 2 min ethyl iodide (18 ml, 0.225 mmol) was added and after a further 1 h, saturated ammonium chloride solution (1 ml) was added and the mixture was extracted with ethyl acetate (50 ml), washed with water and brine, dried ($Na_2SO_4$) and evaporated in vacuo. The resulting oil was purified by flash column chromatography on silica (dry loaded, eluting with an ethyl acetate/hexane gradient, 25-100% ethyl acetate) to give the title compound (23 mg, 43%) as a glass:
$^1H$ NMR ($CDCl_3$): δ 1.20 (t, J = 6.8 Hz, 1 H), 2.62 (s, 1 H), 3.56-3.69 (m,4 H), 5.59 (br s, 1 H), 6.69 (d, J = 8.5 Hz, 1 H), 7.21 (dd, J = 8.5 and 2.2 Hz, 1 H), 7.37 (d, J = 2.2 Hz, 1 H), 7.77 (br s, 1 H).

EXAMPLE 67

6-Chloro-3,4-dihydro-4-ethynyl-4(2-methoxyethoxymethyloxymethyl)quinazolin-2(1H)-one

Step A: 6-Chloro-3,4-dihydro-4(2-methoxyethoxymethyloxymethyl)-4-triisopropylsilylethynylquinazolin-2(1H)-one

2-Methoxyethoxymethyl chloride (0.171 ml, 1.50 mmol) was added to a stirred solution of 6-chloro-3,4-dihydro-4-hydroxymethyl-4-triisopropylsilylethynylquinazolin-2(1H)-one (196 mg, 0.50 mmol) and N,N-diisopropylethylamine (0.261 ml, 1.50 mmol) in dry THF (0.5 ml). After 45 min the mixture was diluted with ethyl acetate, washed with 1M hydrochloric acid solution, saturated sodium hydrogen carbonate solution and brine, dried ($Na_2SO_4$) and evaporated in vacuo. The resulting oil was purified by flash column chromatography on silica (eluting with 5% methanol/chloroform) to give the title compound (240 mg, 100%) as an oil:
$^1H$ NMR ($CDCl_3$): δ 1.07 (s, 21 H), 3.36 (s, 3 H), 3.37-3.80 (m, 6 H), 4.68 (d, J = 6.9 Hz, 1 H), 4.74 (d, J = 6.9 Hz, 1 H), 5.80 (d, J = 1.5 Hz, 1 H), 6.74 (d, J = 8.5 Hz, 1 H), 7.14 (1H dd, J = 8.5 and 2.1 Hz, 1 H), 7.39 (d, J = 2.1 Hz, 1 H), 9.56 (br s, 1 H).

Step B: 6-Chloro-3,4-dihydro-4-ethynyl-4-(2-methoxyethoxymethyloxymethyl)quinazolin-2(1H)-one

Tetrabutylammonium fluoride (1 M solution in THF, 57 μl) was added to a stirred solution of 6-chloro-3,4-dihydro-4(2-methoxyethoxymethyloxymethyl)-4-triisopropylsilylethynylquinazolin-2(1H)-one (250 mg, 0.519 mmol) in dry THF (1 ml). After one hour the solution was poured into saturated ammonium chloride solution and was extracted with ethyl acetate (15 ml), washed with water (2x10 ml) and brine (2x15 ml), dried ($Na_2SO_4$) and evaporated in vacuo. The resulting oil was purified by flash column chromatography on silica (eluting with 10% methanol/chloroform) to give an oil which was further purified by preparative HPLC (C-18, acetonitrile/water/0.1% $H_3PO_4$) to give the title compound (21 mg, 12%) as an oil:
$^1H$ NMR ($CDCl_3$): δ 2.64 (s, 1 H), 3.37 (s, 3 H), 3.59 (m, 4 H), 3.76 (d, J = 9.7 Hz, 1 H), 3.83 (d, J = 9.7 Hz, 1 H), 4.73 (d, J = 6.9 Hz, 1 H), 4.77 (d, J = 6.9 Hz, 1 H), 6.11 (br s, 1 H), 6.75 (d, J = 8.5 Hz, 1 H), 7.18 (dd, J =

8.5 and 2.1 Hz, 1 H), 7.3(d, J = 2.1 Hz, 1 H), 9.27 (br s, 1 H).

EXAMPLE 68

6-Chloro-3,4-dihydro-4-ethynyl-4-methoxymethyloxymethyl quinazolin-2(1H)-one

Chloromethylmethyl ether (1.20 ml 16.1 mmol) was added to a stirred solution of 6-chloro-3,4-dihydro-4-ethynyl-4-hydroxymethylquinazolin-2(1H)-one (80 mg, 0.338 mmol), and N,N-diisopropylethylamine (2.8 ml, 16.1 mmol) in dry THF (10 ml). After 1 h the mixture was poured into ethyl acetate, washed with water (15 ml) and brine (2x15 ml), dried ($Na_2SO_4$) and evaporated in vacuo. The resulting yellow solid was purified by flash column chromatography on silica (eluting with 5% methanol/chloroform) to give an oil which was further purified by preparative HPLC (C-18, acetonitrile/water/0.1% $H_3PO_4$) to give the title compound (10 mg, 11%) as a crystalline solid: mp 101-102°C;
$^1H$ NMR ($CDCl_3$): δ 2.65 (s, 1 H), 3.33 (s, 3 H), 3.71 (d, J = 9.8 Hz, 1 H), 3.77 (d, J = 9.8 Hz, 1 H), 4.65 (d, J = 6.7 Hz, 1 H), 4.69 (d, J = 6.7 Hz, 1 H), 5.63 (br s, 1 H), 6.69 (d, J = 8.5 Hz, 1 H), 7.21 (dd, J = 8.5 and 2.4 Hz, 1 H), 7.39 (d, J = 2.4 Hz, 1 H), 7.91 (br s, 1 H).

EXAMPLE 69

6-Chloro-3,4-dihydro-4-ethynyl-4(2-methyl-2-propyloxymethyl)-quinazolin-2(1H)-one

2-Methyl-2-propene (5 ml) was condensed on to a frozen solution of 6-chloro-3,4-dihydro-4-ethynyl-4-hydroxymethylquinazolin-2(1H)-one (89 mg, 0.376 mmol) and concentrated sulfuric acid (70 ml) in dioxane (5 ml) at -78°C. The mixture was warmed 0°C, stoppered and then stirred at RT for 96 h. The solution was poured into saturated sodium hydrogen carbonate solution, extracted with ethyl acetate, washed with water and brine, dried ($Na_2SO_4$) and evaporated in vacuo. The resulting oil was purified by flash column chromatography on silica (dry loaded, eluting with 2% methanol in chloroform) to give a glass which was crystallised and then recrystallised from methylene chloride/hexane to give the title compound (47 mg, 43%): mp 193-195°C;
$^1H$ NMR ($CDCl_3$): δ 1.16 (s, 9 H), 2.57 (s, 1 H), 3.51 (d, J = 8.6 Hz, 1 H), 3.59 (d, J = 8.6 Hz, 1 H), 5.62 (br s (1 H), 6.70 (d, J = 8.5 Hz, 1 Hz), 7.20 (dd, J = 8.5 and 2.2 Hz, 1 H), 7.36 (dd, J = 2.2 Hz, 1 H), 8.10 (br s, 1 H).

EXAMPLE 70

6-Chloro-3,4-dihydro-4-ethynyl-4(2-furyl)quinazolin-2(1H)-one

Step A: 6-Chloro-3,4-dihydro-4(2-furyl)-4-triisopropylsilylethynylquinazolin-2(1H)-one

Butyllithium (2.5 M solution in hexanes, 20 ml) was added over 5 min to a stirred solution of furan (4.40 ml, 60.5 mmol) in dry THF (50 ml) at 0°C under argon. After 30 min the resulting solution (20 ml) was added dropwise to a stirred mixture of 6-chloro-4-triisopropylsilylethynyl-2(1H)-quinazolinone (1.083 g, 3.00 mmol) and magnesium bromide etherate (1.551 g, 6.01 mmol) in dry diethyl ether (15 ml) at 0°C under argon. After 1 h more a-lithiofuran (50 ml) was added and the mixture was warmed to RT. After 1 h, saturated ammonium chloride solution (20 ml) was added and the mixture was extracted with ethyl acetate (100 ml), washed with brine, dried ($Na_2SO_4$) and evaporated in vacuo. The crude product was purified by flash column chromatography on silica (dry loaded, eluting with an ethyl acetate/hexane gradient, 20-40% ethyl acetate) to give the title compound (1.057 g, 82%) as a tan solid:
$^1H$ NMR ($CDCl_3$): δ 1.09 (s, 1 H), 5.47 (br s, 1 H), 6.31 (dd, J = 3.2 and 1.9 Hz, 1 H), 6.38 (dd, J = 3.2 and 0.9 Hz, 1 H), 6.72 (d, J = 8.5 Hz, 1 H), 7.20 (dd, J = 8.5 and 2.4 Hz, 1 H), 7.33-7.36 (m, 2 H), 7.79 (br s, 1 H).

Step B: 6-Chloro-3,4-dihydro-4-ethynyl-4(2-furyl) quinazolin-2(1H)-one

Tetrabutylammonium fluoride (1 M solution in THF, 3.3 ml) was added to a stirred solution of 6-chloro-3,4-dihydro-4(2-furyl)-4-triisopropylsilylethyn ylquinazolin-2(1H)-one (0.999 g, 2.328 mmol) in dry THF (6 ml). After 15 min, saturated ammonium chloride solution (5 ml) and brine (5 ml) were added and the mixture was extracted with ethyl acetate (50 ml), washed with brine, dried ($Na_2SO_4$) and evaporated in vacuo. The residue was purified by flash column chromatography on silica (eluting with 2% methanol in chloroform) to give a solid which was recrystallised from methylene chloride to give the title compound (0.538 g, 85%): mp 213-215°C;
$^1H$ NMR ($CDCl_3$): δ 2.79 (s, 1 H), 6.07 (br s, 1 H), 6.32 (dd, J = 3.4 and 2.0 Hz, 1 H), 6.38 (dd, J = 3.4 and 0.9

Hz, 1 H), 6.77 (d, J = 8.5 Hz, 1 H), 7.19 (dd, J = 8.5 and 2.2 Hz, 1 H), 7.29 (d, J = 2.2 Hz, 1 H), 7.38 (dd, J = 2.0 and 0.9 Hz, 1 H), 9.04 (br s, 1 H).

EXAMPLE 71

6-Chloro-3,4-dihydro-4-ethynyl-4(2-furyl)-3-methylquinazolin-2(1H)-one

Step A: 6-Chloro-3,4-dihydro-4-ethynyl-4(2-furyl)-1(4-methoxybenzyl)quinazolin-2(1H)-one

Sodium hydride (33.6 mg, 1.40 mmol) was added to a stirred solution of 6-chloro-3,4-dihydro-4-ethynyl-4(2-furyl)quinazolin-2(1H)-one (0.333 g, 1.22 mmol) and sodium iodide (22.5 mg, 1.50 mmol) in dry DMF (2 ml). After 10 min 4-methoxybenzyl chloride (0.180 ml, 1.33 mmol) was added and after a further 30 min saturated ammonium chloride solution (2 ml), water (2 ml) and e hyl acetate (20 ml) were added, and the mixture was washed with water (2x20 ml) and brine, dried ($Na_2SO_4$) and evaporated in vacuo. The resulting oil was purified by flash column chromatography on silica (dry loaded, eluting with an ethyl acetate/hexane gradient, 30-40% ethyl acetate) to give the title compound (0.264 g, 55%) as a pale tan solid:
$^1$H NMR ($CDCl_3$): δ 2.82 (s, 1 H), 3.77 (s, 3 H), 5.08(d, J = 16.4 Hz, 1 H), 5.15 (d, J = 16.4 Hz, 1 H), 5.63 (br s, 1 H), 6.35 (dd, J = 3.3 and 1.7 Hz, 1 H), 6.37 (dd, J = 3.3 and 1.0 Hz, 1 H), 6.77 (d, J = 8.8 Hz, 1 H), 6.82 (d, J = 8.8 Hz, 2 H), 7.12-7.18 (m, 3 H), 7.31 (d, J = 2.4 Hz, 1 H), 7.41 (dd, J = 1.7 and 1.0 Hz, 1 H).

Step B: 6-Chloro-3,4-dihydro-4-ethynyl-4(2-furyl)-1(4-methoxybenzyl)-3-methylquinazolin-2(1H)-one

Sodium hydride (14.4 mg, 0.600 mmol) was added to a stirred solution of 6-chloro-3,4-dihydro-4-ethynyl-4(2-furyl)-1(4-methoxybenzyl)quinazolin-2(1H)one (202.1 mg, 0.514 mmol) in dry DMF (2 ml). After 10 min methyl iodide (32 ml, 0.514 mmol) was added and after a further 20 min saturated ammonium chloride solution (2 ml), water (2 ml) and ethyl acetate (20 ml) were added, and the mixture was washed with water (2x20 ml) and brine, dried ($Na_2SO_4$) and evaporated in vacuo. The resulting oil was purified by flash column chromatography on silica (dry loaded, eluting with 20% ethyl acetate/hexane) to give a glass which was crystallized from ethyl acetate/hexane to give the title compound (134.2 mg, 64%):
$^1$H NMR ($CDCl_3$): δ 2.85 (s, 1 H), 3.06 (s, 3 H), 3.77 (s, 3 H), 5.11 (d, J = 16.4 Hz, 1 H), 5.19 (d, J = 16.4 Hz, 1 H), 6.37 (dd, J = 3.4 and 1.8 Hz, 1 H), 6.58 (dd, J = 3.4 and 1.0 Hz, 1 H), 6.73 (d, J = 8.8 Hz, 1 H), 6.84 (d, J = 8.8 Hz, 2 H), 7.10 (dd, J = 8.8 and 2.4 Hz, 1 H), 7.16 (d, J = 2.4 Hz, 1 H), 7.19 (d, J = 8.8 Hz, 2 H), 7.38 (dd, J = 1.8 and 1.0 Hz, 1 H).

Step C: 6-Chloro-3,4-dihydro-4-ethynyl-4(2-furyl)-3-methylquinazolin-2(1H)-one

Trifluoroacetic acid (2 ml) was added to a solution of 6-chloro-3,4-dihydro-4-ethynyl-4(2-furyl)-1(4-methoxybenzyl)-3-methylquinazolin-2(1H)-one (85.1 mg, 0.209 mmol) in dry dichloromethane (2 ml). After 40 min the resulting deep purple solution was evaporated in vacuo to a glass. Triethylamine (4 ml) and dichloromethane (2 ml) were added and the solution was evaporated in vacuo to an orange glass which was purified by flash column chromatography on silica (dry loaded, eluting with an ethyl acetate/hexane gradient, 30-40% ethyl acetate) to give a solid which was recrystallized (ethyl acetate/hexane) to give the title compound (10.7 mg, 18%): mp 194-198 °C (dec);
$^1$H NMR ($CDCl_3$): δ 2.84 (s, 1 H), 3.00 (s, 3 H), 6.36 (dd, J = 3.3 and 1.7 Hz, 1 H), 6.61 (dd, J = 3.3 and 0.7 Hz, 1 H), 6.71 (d, J = 8.5 Hz, 1 H), 7.12 (d, J = 2.4 Hz, 1 H), 7.18 (dd, J = 8.5 and 2.4 Hz, 1 H), 7.37 (dd, J = 1.7 and 0.7 Hz, 1 H), 7.94 (br s, 1 H).

EXAMPLE 72

(+/-) 6-Chloro-4,4-dicyclopropylmethyl-3,4-4-dihydroquinazolin-2(1H)-one

Step A: 6-Chloro-4,4-di-(2-propenyl)-3,4-dihydroquinazolin-2(1H)-one

Allyl magnesium bromide (32.8 ml, 1.0M in ether), was added dropwise to a solution of 5-chloroanthranilonitrile in 40 ml dry ether at 0°C. The bath was removed and stirred for 30 min. Cooled to 0°C and dimethylcarbonate (3.3 ml, 39.3 mmol) was added. The bath was removed and stirred for 2 h. Ether was added and 8.79 g of a yellow solid was filtered off and dried by suction.

Step B: 6-Chloro-4,4-di-(2-propenyl)-1-(4-methoxybenzyl)-3,4-dihydroquinazolin-2(1H)-one

In a manner according to Example 25, step C, the title compound was prepared from 6-chloro-4,4-di-(2-propenyl)-3,4-dihydroquinazolin-2(1H)-one (8.79 g, 13.1 mmol) to give 990 mg of a yellow solid.

Step C: 6-Chloro-4,4-dicyclopropylmethyl-1-(4-methoxybenzyl)-3,4-dihydroquinazolin-2(1H)-one

In a manner according to Example 24, step B, the title compound was prepared from 300 mg (0.783 mmol) 6-chloro-4,4-di-(2-propenyl)-1-(4-methoxybenzyl)-3,4-dihydroquinazolin-2(1H)-one to give 303 mg of a solid.

Step D: 6-Chloro-4,4-dicyclopropylmethyl-3,4-dihydroquinazolin-2(1H)-one

In a manner according to Example 14, step C, the title compound was prepared from 108 mg (0.263 mmol) 6-chloro-4,4-dicyclopropylmethyl-1-(4-methoxybenzyl)-3,4-dihydroquinazolin-2(1H)-one to give 40 mg (53%) of a solid.
$^1$H NMR ($CDCl_3$): δ -0.05-0.02 (m, 2H); 0.06-0.16 (m, 2H); 0.35-0.43 (m, 2H); 0.43-0.54 (m, 2H); 0.58-0.69 (m, 2H); 1.68 (dd, J=6.7,14.3, 2H); 1.80 (dd, J=6.6,14.5, 2H); 5.36 (br s, 1H); 6.68 (d, J=8.4, 1H); 7.06 (d, J=2.2, 1H); 7.12 (dd, J=2.2,8.4, 1H); 8.13 (br s, 1H). mp: 183-185°C

EXAMPLE 73

(+/-) 9-Chloro-10b-(2-furyl)-1,2,3,10b-tetrahydropyrrolo[1,2-c]quinazolin-5(6H)-one

Step A: 6-Chloro-4-(2-furyl)-4-(3-hydroxypropyl)-3,4-4-dihydroquinazolin-2(1H)-one

In a manner according to Example 25, step A, the title compound was prepared from 6-chloro-4(2-furyl)-4-(2-propenyl)-3,4-dihydroquinazolin-2(1H)-one (192 mg, 0.665 mmol) to give 124 mg of a foam.

Step B: (+/-) 9-Chloro-10b-(2-furyl)-1,2,3,10b-tetrahydropyrrolo[1,2-c]quinazolin-5(6H)-one

In a manner according to Example 25, step E, the title compound was prepared as the unexpected product from 124 mg (0.381 mmol) of 6-Chloro-4-(2-furyl)-4-(3-hydroxypropyl)-3,4-dihydroquinazolin-2(1H)-one to give 8 mg (8%) of a solid:
$^1$H NMR ($CDCl_3$): δ 2.02-2.09 (m, 2H); 2.29-2.37 (m, 1H); 2.90 (ddd, J=2.2,6.1,12, 1H); 3.71-3.75 (m, 2H); 6.05 (dd, J=0.9,3.3, 1H); 6.23 (dd, J=1.8,3.1, 1H.); 6.72 (d, J=8.4, 1H); 7.16 (dd, J=2.4,8.4, 1H); 7.27 (d, J=2.4, 1H); 7.31-7.32 (m, 1H).
mp: 264°C (dec)

EXAMPLE 74

(+/-) 10-Chloro-11b-cyclopropylmethyl-1,11b-dihydro-2H, 6H-[1,3]oxazino[4,3-c]quinazolin-6(7H)-one

Step A: 6-Chloro-4-cyclopropylmethyl-4-(2-propenyl)-3,4-dihydroquinazolin-2(1H)-one

In a manner according to Example 24, step B, the title compound was prepared from 6-chloro-4,4-di-(2-propenyl)-3,4-dihydroquinazolin-2(1H)-one (1.95 g, 7.42 mmol) to give 1.8 g of a foam.

Step B: 6-Chloro-4-cyclopropylmethyl-4-(2-hydroxyethyl)-3,4-dihydroquinazolin-2(1H)-one

In a manner according to Example 22, step A the title compound was prepared from 6-chloro-4-cyclopropylmethyl-4-(2-propenyl)-3,4-dihydroquinazolin-2(1H)-one (1.8 g, 6.64 mmol) to give 847 mg of a foam.

Step C: (+/-) 10-Chloro-11b-cyclopropylmethyl1b,11b-dihydro-2H,6H-[1,3]oxazino[4,3-c]-quinazolin-6(7H)-one

6-Chloro-4-cyclopropylmethyl-4-(2-hydroxy ethyl)-3,4-dihydroquinazolin-2(1H)-one (100 mg, 0.356 mmol) was refluxed in 1 ml formic acid and 1 ml formalin under argon for 1 h. The reaction was cooled, basified

with 10% $Na_2CO_3$, extracted with chloroform, washed with water, brine, dried over $Na_2SO_4$, filtered and the solvent removed in vacuo to give an oil. The oil was dissolved in 3 ml methanol and 2 ml 2N NaOH and stirred for 15 min. The solvent was removed, water was added, extracted with EtOAc, washed with water, brine, dried over $MgSO_4$, and the solvent removed in vacuo to give 56 mg (54%) of a solid.
$^1$H NMR ($CDCl_3$): δ -0.25-(-)0.17 (m, 1H); -0.05-0.02 (m, 1H); 0.28-0.36 (m, 1H); 0.38-0.46 (m, 1H); 0.48-0.57 (m, 1H); 1.49 (dd, J=6.6,14.5, 1H); 2.01 (dt, J=1.8,13.4, 1H); 2.27-2.36 (m, 2H); 3.94 (td, J=2.3, 12.4, 1H); 4.11 (dd, J=5.1,12.1, 1H); 4.48 (d, J=10.3, 1H); 5.86 (d, J=10.3, 1H); 6.60 (d, J=8.4, 1H); 7.00 (d, J=2.2, 1H); 7.14 (dd, J=2.2,8.4, 1H); 7.67 (br s, 1H).
mp: 197-198°C

EXAMPLE 75

(+/-) 10-Chloro-11b-cyclopropylmethyl-1,11b-dihydro-2H,6H-[1,3]oxazino[4,3-c]quinazolin-4,6(7H)-dione

Step A: 6-Chloro-4-cyclopropylmethyl-1-(4-methoxybenzyl)-4-(2-propenyl)-3,4-dihydroquinazolin-2(1H)-one

In a manner according to Example 24, step B, the title compound was prepared from 6-chloro-4,4-di-(2-propenyl)-1-(4-methoxybenzyl)-3,4-dihydroquinazolin-2 (1H)-one (356 mg, 0.914 mmol) to give 304 mg of an oil.

Step B: 6-Chloro-4-cyclopropylmethyl-4-(2-hydroxyethyl)-1-(4-methoxybenzyl)-3,4-dihydroquinazolin-2(1H)-one

In a manner according to Example 22, step A, the title compound was prepared from 6-chloro-4-cyclopropylmethyl-1-(4-methoxybenzyl)-4-(2-propenyl)-3,4-di hydroquinazolin-2(1H)-one (304 mg, 0.766 mmol) to give 155 mg of an oil.

Step C: 6-Chloro-4-cyclopropylmethyl-4-(2-hydroxyethyl)-3,4-dihydroquinazolin-2(1H)-one

In a manner according to Example 14, step C, the title compound was prepared from 6-chloro-4-cyclopropylmethyl-4-(2-hydroxyethyl)-1-(4-methoxybenzyl)-3,4-dihydroquinazolin-2(1H)-one (130 mg, 0.324 mmol) to give 67 mg of a solid.

Step D: (+/-) 10-Chloro-11b-cyclopropylmethyl-1,11b-dihydro-2H,6H-[1,3]oxazino[4,3-c]quinazolin-4,6(7H)-dione

6-Chloro-4-cyclopropylmethyl-4-(2-hydroxyethyl)-3,4-dihydroquinazolin-2(1H)-one (35 mg, 0.125 mmol), carbonyl diimidazole (22 mg, 0.137 mmol), and diisopropylethylamine (0.024 ml, 0.137 mmol) were dissolved in 2 ml dry THF and stirred under argon for 7 h. Diluted with EtOAc, washed with 1 M citric acid, water, brine, dried over $MgSO_4$, filtered and solvent removed in vacuo to give an oil. The oil was dissolved in 1 ml DMF and 20 mg NaH (60% in oil) was added and stirred for 2 h. The reaction was quenched with water and extracted with EtOAc. The organic layers were washed with water, brine, dried over $MgSO_4$, filtered, the solvent removed in vacuo, and chromatographed on silica gel using 5% methanol/chloroform to give 6 mg (16%) of a solid.
$^1$H NMR ($CDCl_3$): δ -0.24-(-)0.14 (m, 1H); 0.00-0.09 (m, 1H); 0.30-0.48 (m, 2H); 0.48-0.58 (m, 1H); 1.84 (dd, J=6.7,14.3, 1H); 1.98 (dd, J=6.5,14.4, 1H); 2.43-2.55 (m, 1H); 2.76-2.87 (m, 1H); 4.37-4.56 (m, 2H); 6.94 (d, J=8.4, 1H); 7.12 (d, J=2.0, 1H); 7.28 (dd, J=2.1,8.4, 1H); 9.14 (br s, 1H). mp: 195°C (dec).

EXAMPLE 76

(+/-)-6-Chloro-4-allyl-3,4-dihydro-4-isopropylquinazolin-2(1H)-one

Step A: (+/-)-6-Chloro-4-isopropylquinazolin-2(1H)-one

In the manner outlined in Example 72, step A, the title compound was prepared from 5-chloroanthranilonitrile.
$^1$H NMR($CDCl_3$): δ 0.75 (d, J=6.78, 3H), 1.06 (d, J=6.77, 3H), 2.22-2.35 (m, 1H), 6.79 (d, J=8.24, 1H), 7.26 (dd, J=8.06, 2.20, 1H), 7.63 (dd, J=8.79, 2.20, 1H), 7.92 (s, 1H).

Step B: (+/-)-6-Chloro-4-allyl-3,4-dihydro-4-isopropylquinazolin-2(1H)-one

In the manner outlined in Example 39, the title compound was prepared from (+/-)-6-Chloro-4-isopropylquinazolin-2(1H)-one.
mp: 145-149°C
$^1$H NMR ($CDCl_3$): δ 0.85 (d, J=6.78, 3H), 0.98 (d, J=6.77, 3H), 2.01 (m, 1H), 2.55 (d, J=7.11, 2H), 5.01-5.09 (m, 3H), 5.54-5.62 (m, 1H), 6.63 (d, J=8.61, 1H), 7.03 (d, J=2.20, 1H), 7.10 (dd, J=8.42, 2.19, 1H), 7.97-8.18 (m, 1H).

EXAMPLE 77

(+/-)-6-Chloro-4-n- propyl-3,4-dihydro-4-isopropylquinazolin-2(1H)-one

In the manner outlined in Example 92, the title compound was prepared from (+/-)-6-chloro-4-allyl-3,4-dihydro-4-isopropylquinazolin-2(1H)-one.
mp: 200-201°C
$^1$H NMR($CDCl_3$): δ 0.75-2.10 (m, 5H), 0.81 (d, J=5.66, 3H), 0.87 (t, J=6.41, 3H), 0.97 (d, J=6.60, 3H), 5.09 (br s, 1H), 6.65 (d, J=8.36, 1H), 6.99 (d, J=2.01, 1H), 7.09 (d, J=8.25, 1H), 8.58 (br s, 1H)

EXAMPLE 78

(+)-6-Chloro-4-n- propyl-3-methyl-3,4-dihydro-4-isopropylquinazolin-2(1H)-one

100 mg (0.36 mmol) of (+)-6-Chloro-4-cyclopropyl-3-methyl-3,4-dihydro-4-n-propylquinazolin-2(1H)one was dissolved in 20 ml methanol, treated with 30 mg Pd(OH) 10% on carbon and hydrogenated for 16 hr at 40 psi. Removal of the catalyst by filtration followed by concentration yielded a yellow solid. This solid was dissolved in 5 ml acetic acid, and treated with 17 mg Pt oxide under a 1 atm hydrogen atmosphere . After 24 hr another 35 mg of Pt oxide added. After 16 hr the catalyst was filtered off and the acid solution was made pH=8 with cold 40 % NaOH and was extracted into EtOAc. The organic layer was washed with brine, dried over $NaSO_4$ and concentrated to yield a residue which was chromatographed on silica gel using 1:3 EtOAc:$CHCl_3$ to give 35 mg (40%) of the title compound as a colorless solid.
mp: 119-121°C
$^1$H NMR($CDCl_3$): δ 0.79 (d, J=6.77, 3H), 0.87 (d, J=6.78, 3H), 0.87-2.38 (m ,8H), 2.99 (s, 3H), 6.69 (d, J=7.88, 1H), 6.88-6.92 (m, 1H), 7.02 (d, J=7.87, 1H), 7.12-7.16 (m, 1H), 8.20 (br s, 1H).

EXAMPLE 79

(+/-)-6-Chloro-4-cyclopropyl-4-ethoxymethyl-3-ethyl-3,4-dihydro-2(1H)-cyanoiminoquinazoline

Step A: (+/-)-2,6-Dichloro-4-cyclopropyl-4-ethoxymethyl-3-ethyl-3,4-dihydroquinazoline

To a stirring solution of 210 mg (0.68 mmol) of (+/-) 6-chloro-4-cyclopropyl-4-ethoxymethyl-3-ethyl-3,4-dihydroquinazolin-2(1H)-one in 10 ml of $POCl_3$, was added 80.2 mg (1.50 mmol) of $NH_4Cl$ and the mixture was heated at reflux for 2.5 hr. The $POCl_3$ was removed under reduced pressure. The residue partitioned between $CHCl_3$ and 10% $NaHCO_3$ then washed with water and brine, dried over $Na_2SO_4$ and concentrated to dryness to yield 170 mg of the title compound as a light yellow foam.
$^1$H NMR ($CDCl_3$): δ 0.48-.52 (m, 2H); 0.63-.76 (m, 2H); 1.16(t, J=7.1Hz, 3H); 1.40 (t, J=7.1Hz, 3H); 3.48 (q, J=7.0Hz, 3H); 3.72(d, J=1.5Hz, 3H); 3.74-3.94(m, 2H); 7.15-7.25 (m, 3H).

STEP B: (+/- )-6-Chloro-4-cyclopropyl-4-ethoxymethyl-3-ethyl-3,4-dihydro-2(1H)-cyanoiminoquinazoline

(+/-)-2,6-Dichloro-4-cyclopropyl-4-ethoxymethyl-3-ethyl-3,4-dihydroquinazoline (170 mg, 0.523 mmol) was combined with cyanamide (1.70 g) and the mixture was melted in a 60°C oil bath. After 6h the mixture was partitioned between water and EtOAc. The organic layer was washed with water, brine, dried over $Na_2SO_4$ and concentrated to give a residue which was chromatographed on silica gel using 30:70 ethyl acetate-hexane. Crystallization from pet ether-diethyl ether afforded 41 mg (23%) of the title compound as a colorless solid.
$^1$H NMR ($CDCl_3$): δ 0.37(m, 2H); 0.65(m, 2H); 1.13(t, 6.9Hz, 3H);1.30(m, 4H); 3.45(dd, J=7.0, 14.0Hz, 2H); 3.66-3.83(m, 4H); 7.00(d, J=9.1Hz, 1H); 7.20-7.24 (m, 2H); 8.47 (s, 1H).

mp: 148-150°C (dec)

EXAMPLE 80

(+/-)-6-Chloro-4-cyclopropyl-4-ethoxymethyl-3-ethyl-3,4-dihydro-2(1H)-methoxyliminoquinazoline

(+/-)-2,6-Dichloro-4-cyclopropyl-4-ethoxymethyl-3-ethyl-3,4-dihydroquinazoline (68 mg, 0.28 mmol) as prepared in Example 79, Step A and methoxylamine hydrochloride (255 mg, 1.68 mmol) were dissolved in 8.0 ml of pyridine to which cesium carbonate (339 mg, 1.04 mmol) was added and the reaction mixture was stirred at 50°C for 17h. The solvent was removed under reduced pressure and the residue partitioned between water and EtOAc. The organic layer was washed with water brine, dried over $Na_2SO_4$ and concentrated to yield a residue which was chromatographed on silica gel using 30:70 ethyl acetate-hexane then crystallized from diethyl ether to give 65 mg colorless solid (23%) of the title compound isolated as the dihydrochloride salt:
$^1$H NMR ($CDCl_3$): δ 0.28(m, 2H); 0.50(m, 2H); 1.02(m, 1H); 1.17(t, 6.9Hz, 3H); 1.27(t, J= 7.0Hz, 4H); 3.44-3.82(m, 9H); 3.66-3.83(m, 4H); 7.00(d, J=8.3Hz, 1H); 7.12(dd, J=6.3, 8.5Hz, 1H); 7.19(d, J=2.3Hz, 1H); 7.47 (s, 1H).
mp: 114-115°C

EXAMPLE 81

(+/-)-6-Chloro-4-cyclopropyl-4-ethoxymethyl-3-ethyl-3,4-dihydro-2(1H)-hydroxyliminoquinazoline

(+/-)-2,6-Dichloro-4-cyclopropyl-4-ethoxymethyl-3-ethyl-3,4-dihydroquinazoline (150 mg, 0.458 mmol) as prepared in Example 79, Step A and hydroxylamine hydrochloride (160 mg, 2.29 mmol) were dissolved in 10.0 ml of pyridine to which cesium carbonate (746 mg, 2.29 mmol) was added. The reaction mixture was stirred at 60°C for 4h. The solvent was removed under reduced pressure, the residue partitioned between water and EtOAc, and the organic layer washed with water, brine, dried over $Na_2SO_4$. The EtOAc was concentrated to dryness followed by silica gel chromatography using 30:70 ethyl acetate -hexane to afford the title compound crystallized from diethyl ether -petroleum ether 89 mg colorless solid (60%):
$^1$H NMR ($CDCl_3$): δ 0.31(d, J= 5.3Hz, 2H); 0.52(dd, J= 8.6, 16.7Hz, 2H); 1.02(m, 1H); 1.06(m, 1H); 1.16 (t, 6.8Hz, 3H); 1.27(t, J= 6.8Hz, 3H); 3.43-3.72(m, 4H); 3.74(s 2H); 6.66(d, J=8.6Hz, 1H); 7.14(dd, J=2.0, 8.3Hz, 1H); 7.21(d, J=2.0Hz, 1H); 7.60(br s, 2H).

EXAMPLE 82

(+/-)-6-Chloro-4-phenyl-4-ethynyl-3-methyl-3,4-dihydro-2(1H)-methoxyliminoquinazoline

Step A: (+/-)-2,6-Dichloro-4-phenyl-4-ethynyl-3-methyl-3,4-dihydroquinazoline

To a stirring solution of 1.50 g (4.80 mmol) of (+/-) 6-chloro-4-phenyl-4-ethynyl-3-methyl-3,4-dihydroquinazolin-2(1H)-thione in in dry methylene chloride, 405 μl of distilled sulfuryl chloride was added, and the bright yellow solution was stirred overnight under argon. The solvent was removed under reduced pressure, the crude iminochloride was dissolved in petroleum ether and the suspension filtered to remove inorganic solids. The title compound was isolated after evaporation of the solvent and used directly in Step B.

Step B: (+/-)-6-Chloro-4-phenyl-4-ethynyl-3-methyl-3,4-dihydro-2(1H)-methoxyliminoquinazoline

(+/-)-2,6-Dichloro-4-phenyl-4-ethynyl-3-methyl-3,4-dihydroquinazoline (100 mg, 0.317 mmol) and methoxylamine hydrochloride (265 mg, 3.17 mmol) were dissolved in 8.0 ml of dimethoxyethane to which cesium carbonate (310 mg, 0.95 mmol) was added and the reaction mixture was stirred at room temperature for 17 hr. The solvent was removed under reduced pressure and the residue partitioned between water and EtOAc. The organic layer was washed with water, brine, dried over $Na_2SO_4$ and concentrated to yield a residue which was chromatographed on silica gel using 30:70 ethyl acetatehexane then crystallized from petroleum ether to give the title compound, 136 mg colorless solid (85%).
$^1$H NMR ($CDCl_3$): δ 2.56(s, 3H); 2.86(s, 1H); 3.83(s 3H); 6.59(d, J=2.2Hz, 1H); 6.90(d, J=8.5Hz, 1H); 7.11(dd, J=2.3, 8.5Hz, 1H); 7.39-7.42 (m, 3H); 7.59-7.65 (m, 3H); 7.47 (s, 1H).

EXAMPLE 83

(+/-)-6-Chloro-4-phenyl-4-ethynyl-3-methyl-3,4-dihydro-2(1H)-iminoquinazoline

(+/-)-2,6-Dichloro-4-phenyl-4-ethynyl-3-methyl-3,4-dihydroquinazoline (100 mg, 0.317 mmol) as prepared in Example 82, Step A was first dissolved in dimethoxyethane to which liquid ammonia was added before heating to 110°C for 17h in a stainless steel bomb. The reaction mixture solvent was removed under reduced pressure and the residue was chromatographed on silica gel using ammonia saturated chloroform and the product was lyophilized from dioxane to afford 53 mg of the title compound as a white solid.
$^1$H NMR.($CDCl_3$): δ 2.86(s, 1H); 3.01(s, 1H); 3.83(s 3H); 4.30-4.90(br s, 2H); 6.75(d, J=2.3Hz, 1H); 6.87(d, J=8.5Hz, 1H); 7.08(dd, J=2.3, 8.5Hz, 1H); 7.35-7.40 (m, 3H); 7.62-7.65 (m, 2H); 7.47 (s, 1H).

EXAMPLE 84

(+/-)-6-Chloro-4-phenyl-4-ethynyl-3-methyl-3,4-dihydro-2(1H)-cyanoiminoquinazoline

(+/-)-2,6-Dichloro-4-phenyl-4-ethynyl-3-methyl-3,4-dihydroquinazoline (530 mg, 0.317 mmol) as prepared in Example 82, Step A was combined with cyanamide (1.50 g) and the mixture was melted in a 60°C oil bath. After 3h the mixture was partitioned between water and EtOAc. The organic layer was washed with water, brine, dried over $Na_2SO_4$ and concentrated to give a residue which was chromatographed on silica gel using 30:70 ethyl acetate-hexane, and recrystallized from ethyl acetate-hexane to give 250 mg (48%) of the title compound as a colorless solid.
$^1$H NMR ($CDCl_3$): δ 2.94(s,3H); 3.04(s, 1H); 6.90(d, J=1.9, 1H), 7.20(m, 2H), 7.38-7.46(m, 1H); 7.55-7.58(m, 2H); 9.54(s, 1H).
mp: 238-239°C (dec)

EXAMPLE 85

(+/-)-6-Chloro-4-phenyl-4-ethyl-3-methyl-3,4-dihydro-2(1H)-methyliminoquinazoline

Step A: (+/-)-2,6-Dichloro-4-phenyl-4-ethyl-3-methyl-3,4-dihydroquinazoline

To a stirring solution of 1.50 g (4.80 mmol) of (+/-) 6-chloro-4-phenyl-4-ethyl-3-methyl-3, 4-dihydroquinazolin-2(1H)-thione in dry methylene chloride, 405 μl of distilled sulfuryl chloride was added, and the bright yellow solution was stirred overnight under argon. The solvent was removed under reduced pressure, the crude iminochloride was dissolved in petroleum ether and the suspension filtered to remove inorganic solids. The title compound was isolated after evaporation of the solvent and used as is in Step B.

Step B: (+/-)-6-Chloro-4-phenyl-4-ethyl-3-methyl-3,4-dihydro-2(1H)-methyliminoquinazoline

(+/-)-2,6-Dichloro-4-phenyl-4-ethyl-3-methyl-3,4-dihydroquinazoline (100 mg, 0.313 mmol) was dissolved in 10.0 ml of ethanol in a 60 ml gas-tight bomb and cooled to 0°C before condensing ~5 ml methylamine gas into the reaction mixture. The reaction mixture was stirred for 5 days at ambient temperature before removing the solvent under reduced pressure. The residue was partitioned between water and chloroform and the organic layer was washed with water, brine, dried over $Na_2SO_4$ and concentrated to yield a residue which was chromatographed on silica gel using chloroform. The product was recrystallized from ethyl acetate-hexane to afford 90 mg colorless solid (95%) of the title compound.
$^1$H NMR ($CDCl_3$): δ 0.92(t, J= 7.3Hz, 3H); 2.24(q, J= 7.1Hz, 1H); 2.35(q, J= 7.1Hz, 1H); 2.64(s 3H); 3.15(s 3H); 5.2(br s, 1/2H); 6.39(d, J= 2.4Hz, 1H); 7.02(dd, J= 2.3, 8.5Hz, 1H); 7.18 (br s, 1/2H); 7.29-7.48(m, 6H)
mp: 192-195°C (dec)

EXAMPLE 86

(+/-)-6-Chloro-4-phenyl-4-ethyl-3-methyl-3,4-dihydro-2 (1H)-hydrazinoquinazoline

(+/-)-2,6-Dichloro-4-phenyl-4-ethyl-3-methyl-3,4-dihydroquinazoline (100 mg, 0.313 mmol) as prepared in Example 85, Step A was dissolved in 10.0 ml of ethanol to which hydrazine (100 mg, 98.3 ml, 3.13 mmol) was added and the reaction mixture was stirred at room temperature for 17 hr. The solvent was removed under

reduced pressure and the residue was chromatographed on silica gel using chloroform : chloroform-methanol-ammonia 80:30:3 gradient to give the title compound as a lyophilized solid 28 mg (85%).
$^1$H NMR ($CDCl_3$): δ 0.91(t, J=7.3Hz, 3H); 1.90( br s, 2H); 2.29(q, J= 7.2Hz, 1H); 2.51(q, J= 7.1Hz, 1H); 2.94(s 3H); 6.59(d, J=2.0Hz, 1H); 7.04(d, J=8.8Hz, 1H); 7.22(dd, J=2.2, 8.7Hz, 1H); 7.37-7.49 (m, 5H).

EXAMPLE 87

(+/-)-6-Chloro-4-phenyl-4-ethyl-3-methyl-3,4-dihydro-2(1H)-iminoquinazoline hydrochloride

(+/-)-2,6-Dichloro-4-phenyl-4-ethyl-3-methyl-3,4-dihydroquinazoline (100 mg, 0.313 mmol) as prepared in Example 85, Step A was dissolved in absolute ethanol in a stainless steel bomb in which liquid ammonia was condensed then heated to 110°C for 17h. The reaction mixture solvent was removed under reduced pressure and the residue was chromatographed on silica gel using ammonia saturated chloroform and the product was lyophilized from dioxane to afford 43 mg (20%) of the title compound:
$^1$H NMR ($CDCl_3$): δ 0.93(t, J=7.3Hz, 3H); 2.48(q, J= 7.3Hz, 1H); 2.53(q, J= 7.3Hz, 1H); 2.89(s 3H); 6.53(d, J=2.2Hz, 1H); 7.01(d, J=8.6Hz, 1H); 7.15(dd, J=2.2, 8.6Hz, 1H); 7.37-7.48 (m, 5H); 8.01(s, 2H); 11.22 (s,1H).

EXAMPLE 88

(+/-)-6-Chloro-4-cyclopropyl-3-methyl-3,4-dihydro-4-cyclopropylmethyl-2(1H)-methoxyliminoquinazoline

Step A: (+/-)-2,6-Dichloro-4-cyclopropyl-3-methyl-4-cyclopropylmethyl-3,4-dihydroquinazoline

To a stirring solution of 50 mg (0.17 mmol) of (+/-) 6-Chloro-4-cyclopropyl-4-cyclopropylmethyl-3-methyl-3,4-dihydroquinazolin-2(1H)-one in 3 ml of $POCl_3$ was added 18.4 mg (0.34 mmol) of $NH_4Cl$ and the mixture was heated at reflux for 2 hr. The $POCL_3$ was removed under reduced pressure and the residue partitioned between $CHCl_3$ and ice water. The organic layer was washed with 10% $NaHCO_3$, brine, dried over $Na_2SO_4$ and concentrated to yield 90 mg of the title compound as an oil which was used as is.
$^1$H NMR ($CDCL_3$): δ (-) 0.17-1.48 (m, 10H), 1.72 (d, J=6.84, 2H), 3.42 (s, 3H), 7.20-7.35 (m, 3H).

Step B: (+/-)-6-Chloro-4-cyclopropyl-3-methyl-3,4-dihydro-4-cyclopropylmethyl-2(1H)-methoxy-Liminoquinazoline

To a stirring solution of 165 mg (0.56 mmol) of (+/-)-2,6-Dichloro-4-cyclopropyl-3-methyl-4-cyclopropylmethyl-3,4-dihydroquinazoline in 2.4 ml of pyridine was added 141 mg (1.68 mmol) of methoxylamine hydrochloride and the mixture was stirred at 100°C for 16 hr. The solvent was removed under reduced pressure and the residue partitioned between water and EtOAc. The organic layer was washed with water, brine, dried over $Na_2SO_4$ and concentrated to yield a residue which was chromatographed on silica gel using1:4 EtOAc:$CHCl_3$ to give 99 mg (55%) of the title compound as a white solid. mp: 90-92°C
$^1$H-NMR ($CDCl_3$): δ (-) 0.19-1.21 (m, 10H), 1.73 (ddd, J=111, 15.6, 5.0, 2H), 2.98 (s, 3H), 3.77 (s, 3H), 6.70 (m, 1H), 7.11 (dd, J=8.49, 2.31, 1H), 7.21 (d, J=2.25, 1H), 7.66 (br s, 1H).

EXAMPLE 89

(+/-)-6-Chloro-4-cyclopropyl-3-methyl-3,4-dihydro-1-methyl-4-cyclopropylmethyl-2(1H)-cyanoiminoquinazoline

53.2 mg (0.17 mmol) of (+/-)-2,6-Dichloro-4-cyclopropyl-3-methyl-4-cyclopropylmethyl-3,4-dihydroquinazoline was combined with an excess of cyanamide and the mixture was stirred at 50°C. After 16 hr the mixture was partitioned between water and $CHCl_3$ and filtered through a celite pad. The organic layer was washed with water, brine, dried over $Na_2SO_4$ and concentrated to give a residue which was chromatographed on silica gel using1:5 EtOAc:$CHCl_3$ to give 12 mg (22%) of the title compound as a colorless solid.
mp: 222-226°C (dec)
$^1$H NMR ($CDCl_3$): δ (-) 0.12-1.13 (m, 9H), 1.43-2.05 (m, 3H), 3.39 (s, 3H), 7.30-7.50 (m, 3H), 9.21 (s, 1H).

EXAMPLE 90

6-chloro-4-cyclopropyl-2,2-dioxo-1H-2,1,3-benzothiadiazine

A solution of 1.429 g (7.3 mmol) of cyclopropyl (4-chloroanilin-2-yl) ketone and 769 mg (8.0 mmol) of sulfamide in 35 mL of toluene was heated at reflux for 6 h using a Dean-Stark trap to remove water. After cooling, the solvents were removed and the residue triturated with 95:5 $CHCl_3$-$CH_3OH$. The resulting solution was chromatographed on 100 g of fine $SiO_2$ using 90:10 $CHCl_3$-$CH_3OH$ to give 210 mg of a yellow solid. An anaytical sample was obtained by crystallization from butyl chloride to give a yellow crystals: mp: 202-204°C,
$^1H$ NMR ($CDCl_3$-$CD_3OD$): δ 1.23-1.30 (m, 2H), 1.42-1.48 (m, 2H), 2.36-2.46 (m, 1H), 4.20 (br s, 1H), 7.025 (d, J=8.8 Hz, 1H), 7.49 (dd, J=8.8, 2.3 Hz, 1H), 7.968 (d, J=2.3 Hz, 1H). Anal. Calcd for $C_{10}H_9ClN_2O_2S$ : C 46.79 H 3.53 N 10.91 Found: C 46.85 H 3.58 N 10.79.

EXAMPLE 91

4-Allyl-6-chloro-4-cyclopropyl-3,4-dihydro-2,2-dioxo-1H-2,1,3-benzothiadiazine

A solution of 179 mg (0.744 mmol) of 6-chloro-4-cyclopropyl-2,2-dioxo-1H-2,1,3-benzothiadiazine in 5 mL of dry THF was added to 1.9 mL of a 1M ethereal solution of allylmagnesium bromide (Aldrich) dropwise under Ar. A slight yellow color persisted, so an additional 0.5 mL of allylmagnesium bromide solution was added. The reaction was worked up by adding ice-water and extracting with two portions of $CHCl_3$, drying over $MgSO_4$ and removing the solvents to give an oil which was chromatographed on 10 g fine $SiO_2$ using 1:3 EtOAc-hexane to give 115 mg of a colorless solid: mp: 112-113°C,
$^1H$ NMR (CDCl3): δ 0.28-0.45 (m, 2H), 0.50-0.70 (m, 2H), 1.43-1.53 (m, 1H), 2.47 (dd, J=14.4, 9.1 Hz, 1H), 2.85 (dd, J=14.4, 5.2 Hz, 1H), 4.63 (s, 1H), 5.20-5.32 (m, 2H), 5.58-5.72 (m, 1H), 6.70 (d, J=8.3 Hz, 1H), 6.88 (s, 1H), 7.188 (dd, J=8.3, 2.3 Hz, 1H), 7.23 (d, J=2.3 Hz, 1H). Anal. Calcd for $C_{13}H_{15}ClN_2O_2S$: C 52.26 H 5.06 N 9.38 Found : C 52.14 H 4.96 N 9.11.

EXAMPLE 92

6-chloro-4-cyclopropyl-3,4-dihydro-2,2-dioxo-4-propyl-1H-2,1,3-benzothiadiazine

A solution of 110 mg (0.37 mmol) of 4-Allyl-6-chloro-4-cyclopropyl-3,4-dihydro-2,2-dioxo-1H-2,1,3-benzothiadiazine in 12 mL of absolute ethanol was hydrogenated at 47 psi in the presence of 23 mg of 5% Rh-C for 1h. After filtration of the catalyst, the solution was evaporated to give a white solid which was crystallized from butyl chloride to afford 25 mg of a colorless solid: mp: 135-136°C,
$^1H$ NMR ($CDCl_3$): δ 0.25-0.45 (m, 2H), 0.46-0.70 (m, 2H), 0.93(t, J=7.3 Hz, 3H), 1.25-1.54 (m, 3H), 1.74-1.97 (m, 2H), 4.49 (s, 1H), 6.68 (d, J=9.1 Hz, 1H), 6.88 (s, 1H), 7.12-7.22 (m, 2H). Anal. Calcd for $C_{13}H_{17}ClN_2O_2S$: C 51.91 H 5.70 N 9.31 Found: C 52.13 H 5.63 N 9.18.

EXAMPLE 93

(+/-)-6-Chloro-4-ethynyl-3-hydroxymethyl-4-phenyl-3,4-dihydro-2(1H)-quinazolinone

To 15 ml round bottomed flask containing (+/-)-6-chloro-4-ethynyl-4-phenyl-3,4-dihydro-2(1H)-quinazolinone (56 mg, 0.20 mmol) dissolved in 300 ml dioxane was added pyridinium p-toluenesulfonate (50.3mg, 0.20 mmol) and 5ml formalin solution (37% aqueous), and the reaction mixture was heated to 60°C for 96h. The reaction mixture was quenched into saturted sodium bicarbonate and extracted with ethyl acetate. The organic layer was washed with water, brine, dried over $Na_2SO_4$ and concentrated to yield a residue which was chromatographed on silica gel using 10:90 isopropanol-hexane to afford 45 mg (72%) of the title compound.
$^1H$ NMR ($CDCl_3$): δ 3.02(s 1H); 4.85(d, J=11.0Hz, 1H); 5.02(d, J=10.7Hz, 1H); 6.74(d, J=8.6Hz, 1H); 6.94(d, J=2.2Hz, 1H); 7.16(dd, J=2.2, 7.8Hz, 1H); 7.35-7.45 (m, 3H); 7.63-7.66 (m, 2H); 8.07 (br s,1H).
mp = 138-140°C

EXAMPLE 94

(+/-)-4-ethynyl-6-methanesulfonamido-3-methyl-4-phenyl-3,4-dihydro-2(1H)-quinazolinone

Step A: 5-nitro-2-(1-imidazolecarbonylamino) benzophenone

In a manner similiar to Example 96, step D, 2-amino-5-nitrobenzophenone (24.22 g, 100 mmol) was reacted with 1,1′-carbonyldiimidazole (20.3 g, 125 mmol) in dry, distilled THF (200 ml) at 60°C to which 10% additional 1,1′-carbonyldiimidazole and imidazole (20.0 g, 294 mmol) were added to improve the yield of imidazolide. After aqueous workup, the product was crystallized from a minimum volume of ethyl acetate to afford 10.95 g (33%) of the title compound.

Step B: 6-Nitro-4-hydroxy-3-methyl-4-phenyl-3,4-dihydro-2(1H)-quinazolinone

To a 100 ml round bottomed flask with a stir bar and argon inlet was added 5-nitro-2-(1-imidazolecarbonylamino) benzophenone (10.3 g, 30.6 mmol), imidazole (2.09 g, 30.6 mmol) and distilled, dry THF (200 mL). To this well stirred suspension was sparged methyl amine gas for 45 min and the resulting solution was stirred at room temperature for 17h. The solvents were removed in vacuo, the residue was partitioned between ethyl acetate and 10% citric acid, and the organic layer was washed with water, brine, dried over $Na_2SO_4$. The solvent was removed to yield a residue which was triturated with 1:2 ethyl acetate-hexane and dried at 60°C in vacuo to afford 8.35 g orange solid (95%) of the title compound.

Step C: 6-Nitro-3-methyl-4-phenyl-3,4-dihydro-2(1H)-quinazolinone

In a manner similiar to Example 96, step F, 6-nitro-4-hydroxy-3-methyl-4-phenyl-3,4-dihydro-2(1H)quinazolinone (7.00 g, 23.4 mmol) and 250 ml toluene was heated to reflux with water removal for 18h. The bright yellow-orange tetraene product was collected on a frit and dried in vacuo to afford 5.00 g (73%) of the title compound.

Step D: 4-Ethynyl-3-methyl-6-nitro-4-phenyl-3,4-dihydro-2(1H)quinazolinone

In a manner similiar to Example 96, Step G, 6-nitro-3-methyl-4-phenyl-3,4-dihydro-2(1H)-quinazolinone (500 g, 17.8mmol) was reacted with sodium acetylide suspension (8.07 ml of an 18% wt. suspension in light petroleum, 30.3 mmol) in dry, distilled THF/DMF (1:1). Addition of 18-crown-6 and vigorous stirring at room temperature for three days was necessary to improve the yield. The reaction was quenched into 10% citric acid and extracted with ethyl acetate. The organic layer was washed with water, brine, dried over $Na_2SO_4$ and concentrated to yield a dark red-brown oil (6.80 g). The desired product was isolated by silica gel chromatography using 25:75 ethyl acetate-hexane affording a low isolated yield of the title compound (160 mg, 3% yield).

Step E: 6-Amino-4-ethynyl-3-methyl-4-phenyl-3,4-dihydro-2(1H)-quinazolinone

To a 25 ml three-neck round bottomed flask fitted with reflux condenser and stirring bar was added sodium carbonate (152 mg, 1.43 mmol) and sodium dithionite (198 mg, 1.14 mmol) dissolved in water and heated to 65°C in an oil bath. A methanol solution of 4-ethynyl-3-methyl-6-nitro-4-phenyl-3,4-dihydro-2(1H)-quinazolinone (100 mg, 0.325 mmol in 3 ml MeOH) was added by syringe to the reaction mixture and heated to reflux at 85°C for 2h. Ethyl acetate extraction of the aqueous reaction followed by water and brine washes afforded 70 mg crude amino analog after drying over $Na_2SO_4$, concentrating to dryness. After silica gel chromatography (80:20 ethyl acetate-hexane), 38 mg (42%) of the title compound was isolated.

Step F: (+/-)-4-Ethynyl-6-methanesulfonamido-4-phenyl-3-methyl-3,4-dihydro-2(1H)-quinazolinone

In a dry 10ml round bottomed flask, 6-amino-4-ethynyl-3-methyl-4-phenyl-3,4-dihydro-2(1H)-quinazolinone (10 mg, 0.031 mmol) was dissolved in 4 ml methylene chloride to which 0.50 ml dry, distilled pyridine and N,N-4-dimethylaminopyridine was added. Methanesulfonic anhydride (18.9 mg, 0.108 mmol) was added at 0°C, the reaction mixture was then warmed to room temperature and stirred for 4 days during which additional methanesulfonic anhydride (0.108 mmol) was added. The reaction was quenched into 10% citric acid and extracted with ethyl acetate. The organic layer was washed with water, brine, dried over $Na_2SO_4$ and concentrated to yield the desired product after silica gel chromatography using 25:75 ethyl acetate-hexane The

title compound (8.1 mg, 74% yield) was lyophilized from dioxane.
$^1$H NMR ($CDCl_3$): δ 2.84(s 3H); 2.89(s 3H); 2.97(s, 1H); 6.75-6.82(m, 3H); 7.12(dd, J=2.4 8.5Hz, 1H); 7.36-7.43 (m, 3H); 7.63-7.60 (m, 2H); 8.17 (s,1H).

EXAMPLE 95

(+/-)-6-Chloro-4-ethynyl-4-phenyl-3,4-dihydro-2(1H)-quinazolin-one

Step A: 6-Chloro-4-hydroxy-3-(4-methoxybenzyl)-4-phenyl-3,4-dihydro-2(1H)-quinazolinone

To a 100 ml round bottomed flask with a stir bar and argon inlet was added 5-chloro-2-(1-imidazolecarbonylamino)benzophenone (4.0 g, 12.3 mmol), imidazole (837 mg, 12.3 mmol) and distilled, dry THF (70 mL). To this well stirred suspension, 4-methoxybenzyl amine (8.42 g, 61.4 mmol) was added and the resulting solution stirred at 40°C for 96h. The reaction mixture was partioned between ethyl acetate and 10% citric acid, the organic layer was washed with water, brine, dried over $Na_2SO_4$ and concentrated to yield a residue which was triturated with 1:2 ethyl acetate-hexane and dried at 60°C in vacuo to afford 4.81 g white solid (99%) of the title compound.

Step B: 6-Chloro-3-(4-methoxybenzyl)-4-phenyl-3,4-dihyro-2(1H)-quinazolinone

To a 250 ml round bottomed flask with a stirring bar, Dean-Stark trap, reflux condenser and an argon inlet was added 6-chloro-4-hydroxy-3-(4-methoxybenzyl)-4-phenyl-3,4-dihydro-2(1H)-quinazolinone (4.81 g, 12.18 mmol) and 100 ml toluene. The reaction was heated to reflux with water removal for 18h. Toluene was removed in vacuo and the residue triturated with hexane to crystallize the bright yellow tetraene product, which was collected on a frit and dried in vacuo to afford 4.35 g (95%).

Step C: 6-Chloro-4-ethynyl-3-(4-methoxybenzyl)-4-phenyl-3,4-dihydro-2(1H)-quinazolinone

To an oven dried, three-neck flask with a stirring bar, argon inlet and a thermometer was added 6-chloro-3-(4-methoxybenzyl)-4-phenyl-3,4-dihydro-2(1H)-quinazolinone (3.80 g, 10.1 mmol) and distilled, dry (70 mL). This solution was cooled in an ice bath and a suspension of sodium acetylide (8.07 ml of an 18% wt. suspension in light petroleum, 30.3 mmol) was added in a syringe. The mixture was warmed to 0°C for 30 minutes at which point the yellow slurry changed to an orange solution. Further warming to room temperature and stirring for an additional 1h caused no additional color change. The reaction was quenched into 10% citric acid and extracted with ethyl acetate. The organic layer.was washed with water, brine, dried over $Na_2SO_4$ and concentrated to yield a yellow oil (9.08g) of which the 2 major products were the desired product, 6-chloro-4-ethynyl-3-(4-methoxybenzyl)-4-phenyl-3,4-di-hydro-2(1H)-quinazolinone, and the hydroxy precursor, 6-chloro-4-hydroxy-3-(4-methoxybenzyl)-4-phenyl-3,4-dihydro-2(1H)-quinazolinone. Chromatography using 30:70 ethyl acetate-hexane afforded a low isolated yield of the title compound (380 mg, 10% yield).

Step D: 6-Chloro-4-ethynyl-4-phenyl-3,4-dihydro-2(1H)-quinazolin-one

In a manner according to Example 14, step C, the title compound was prepared from 6-chloro-4-ethynyl-3-(4-methoxybenzyl)-4-phenyl-3,4-dihydro-2(1H)-quinazolin-one (180 mg, 0.447 mmol) using 20% trifluoroacetic acid in methylene chloride after 17h to give 128 mg (99%) of a white solid after silica gel chromatography (1:1 ethyl acetate-hexane) and crystallization from chloroform:
$^1$H NMR ($CDCl_3$): δ 2.93(s 1H); 5.66(s,1H), 6.78(d,J=8.6Hz, 1H); 6.94(d, J=2.4Hz, 1H); 7.18(dd, J=2.2, 10.7Hz, 1H); 7.39-7.45 (m, 3H); 7.65-7.68 (m, 2H); 8.53 (s,1H).
mp = 174-175°C

EXAMPLE 96

(+/-) 6-fluoro-3,4-dihydro-3-methyl-4-phenyl-4-ethynyl-2(1H)-quinazolinone

Step A: 6-fluoro-2-methyl-4-benzoxazocinone

To a 250 mL round bottomed flask with a stirring bar and an argon inlet was added 5-fluoro-2-aminobenzoic acid (10.00g, 64.46 mmol) and acetic anhydride (100 mL, 1.060 mol). This mixture was heated at reflux for 6h

during which time the brown solid dissolved. The cooled reaction mixture was concentrated in vacuo and the solid residue was recrystallized from boiling ethyl acetate to give 8.02g of 6-fluoro-2-methyl-4-benzoxazocinone as white crystals.
$^1H$ NMR ($CDCl_3$): δ 2.47 (s, 3 H), 7.40-7.60 (m, 2 H), 7.83 (dd, J= 2.93, 7.91Hz, 1 H).

Step B: N-(2-benzoyl-4-fluorophenyl)acetamide

To a 300 mL round bottomed flask with a stirring bar, argon inlet and a septum was added 6-fluoro-2-methyl-4-benzoxazocinone (5.37g, 30.0 mmol) and 100 mL of dry THF. This solution was cooled to -78°C and a solution of phenyl magnesium bromide (10.0 mL of a 3M solution in ethyl ether) was added slowly with a syringe. The cooling bath was allowed to expire and the mixture was allowed to warm to room temperature. The reaction mixture was treated with saturated aqueous sodium potassium tartrate solution and extracted with ethyl acetate. The ethyl acetate extract was washed with water and brine. Drying ($MgSO_4$), filtration, and removal of the solvent in vacuo, gave 7.78g of N-(2-benzoyl-4-fluorophenyl) acetamide as an off-white crystalline solid. This material was used without further purification.

Step C: 2-amino-5-fluorobenzophenone

To a 1L round bottomed flask with a stirring bar and a reflux condenser was added N-(2-benzoyl-4-fluorophenyl)acetamide (7.78g, 30 mmol), ethanol (120 mL), and 6N HCl (120 mL). This mixture was heated at reflux for 10h. The cooled reaction mixture was concentrated in vacuo to give a yellow semi-solid. This mixture was dissolved in water and the solution was made basic with 10N NaOH solution. The mixture was extracted with chloroform and this extract was washed with brine, dried ($MgSO_4$), filtered and concentrated in vacuo. The crude product was chromatographed on 250g of silica gel using 10% ethyl acetate in hexane as eluant. There was obtained 2.33g of 2-amino-5-fluorobenzophenone as a bright yellow crystalline solid.
$^1H$ NMR ($CDCl_3$): δ 5.90(br, 1 H), 6.70 (dd, J=4.5, 9.0, 1 H), 7.10 (m, 2 H), 7.56 (m, 3 H), 7.65 (m, 2 H).

Step D: 5-fluoro-2-(1-imidazolecarbonylamino)-benzophenone

To a 100 mL round bottomed flask with a stirring bar, argon inlet and a reflux condenser was added 2-amino-5-fluorobenzophenone (2.33g 10.8 mmol), 1,1′-carbonyldiimidazole (2.19g, 13.53 mmol) and dry methylene chloride (15 mL). This mixture was heated at reflux for 18h. The mixture was concentrateed in vacuo and the residue was partitioned between ethyl acetate and water. The ethyl acetate layer was separated and washed sequentially with 10% aqueous citric acid, water and brine. Drying ($MgSO_4$), filtration, removal of the solvent in vacuo, and trituration with hexanes gave 2.2g of 5-fluoro-2-(1-imidazolecarbonylamino)-benzophenone as a solid.
$^1H$ NMR ($CDCl_3$): δ 6.51 (dd, J=4.4, 9.1, 1 H), 6.98 (s, 1 H), 7.05 (s, 1 H), 7.15 (m, 3 H), 7.31 (m, 2 H), 7.50 (m, 3 H).

Step E: 6-fluoro-3,4-dihydro-4-hydroxy-3-methyl-4-phenyl-2(1H)-quinazolinone

To 100 mL round bottomed flask with a stirring bar was added 5-fluoro-2-(1-imidazolecarbonyl-amino)-benzophenone (2.22g, 7.18 mmol) and distilled, dry THF (50 mL). To this well stirred suspension was sparged methylamine gas for 15 min, during which time all of the solid dissolved. The resulting mixture was stirred at room temperature for 24h. The solvents were then removed in vacuo the residue was triturated with ethyl acetate and the solid product was collected on a frit and washed with a little 1:1 ethyl acetate-hexane solution. The product was dried in vacuo at room temperature. Yield: 1.75g.
$^1H$ NMR (DMSO-$d_6$): δ 2.63 (s, 3 H), 6.59 (dd, J=2.8, 9.3, 1 H), 6.80-7.00 (m, 2 H), 7.10 (s, 1 H), 7.20-7.45 (m, 5 H), 9.80 (br s, 1 H).

Step F: 6-fluoro-3-methyl-4-phenyl-2(3H)-quinazolinone

To a 100 mL round bottomed flask with a stirring bar, Dean-Stark trap, reflux condenser, and an argon inlet was added 6-fluoro-3,4-dihydro-4-hydroxy-3-methyl-4-phenyl-2(1H)-quinazolinone (1.75g, 6.43 mmol) and toluene (50 mL). This mixture was heated at reflux with water removal for 18h. The cooled reaction mixture precipitated yellow crystalline 6-fluoro-3-methyl-4-phenyl-2(3H)-quinazolinone which were collected on a frit and dried in vacuo. Yield: 1.44g. This material was used in the final step without further purification.

Step G: 6-fluoro-3,4-dihydro-4-ethynyl-3-methyl-4-phenyl-2(1H)-quinazolinone

To an oven dried, three necked round bottomed flask with a stirring bar, argon inlet, and a thermometer was added 6-fluoro-3-methyl-4-phenyl-2(3H)-quinazolinone (1.44g, 5.66 mmol), and distilled, dry THF. This solution was cooled in an ice bath and a suspension of sodium acetylide (7.55 mL of an 18% wt. suspension in light petroleum, 28.32 mmol) was added with a syringe. The mixture was warmed to +21°C at which point the yellow solution became white. The mixture was maintained at 21°C for an additional 10 min. then the reaction was quenched with water. The resulting mixture was diluted with ethyl acetate and the layers were separated. The organic phase was washed with brine, dried ($MgSO_4$), filtered, and concentrated in vacuo. The syrupy residue was exposed to high vacuum for 24h to remove the light petroleum. The solid residue was then chromatographed on 90g of silica gel using 1:1 ethyl acetate-hexane as eluant. There was obtained 821 mg of (+/-) 6-fluoro-3,4-dihydro-4-ethynyl-3-methyl-4-phenyl-2(1H)-quinazolinone as a white solid. An analytical sample was prepared by recrystallization from ethyl acetate-hexane. mp: 186-188°C.
$^1$H NMR ($CDCl_3$): δ 2.99 (s, 3 H), 2.95 (s, 1 H), 6.62 (dd, J=2.8, 9.2, 1 H), 6.76 (dd, J=4.8, 8.8, 1 H), 6.85 (dt, J=4.8, 8.1, 1 H), 7.38 (m, 3H), 7.62 (m, 2 H), 8.88 (br s, 1 H).

EXAMPLE 97

(+/-) 4-Ethynyl-3-methyl-4-phenyl-3,4-dihydroquinazolin-2(1H)-one

In a manner similar to Example 96, step G, the title compound was prepared from 3-methyl-4-phenyl-2(3H)quinazolinone.
$^1$H NMR ($CDCl_3$): δ 2.85 (s, 3H); 2.99 (s, 1H); 6.77 (d, J=8, 1H); 6.81-6.91 (m, 2H); 7.13-7.18 (m, 1H); 7.33-7.41 (m, 3H); 7.57-7.65 (m, 2H).
mp: 201-202°C

EXAMPLE 98

(+/-) 6-Bromo-4-ethynyl-3-methyl-4-phenyl-3,4-4-dihydroquinazolin-2(1H)-one

In a manner similar to Example 96, step G, the title compound was prepared from 6-bromo-3-methyl-4-phenyl-2(3H)quinazolinone.
$^1$H NMR ($CDCl_3$): δ 2.87 (s, 3H); 2.96 (s, 1H); 6.63 (d, J=8.4, 1H); 7.03 (d, J=2.0, 1H); 7.24-7.27 (m, 1H); 7.31-7.44 (m, 3H); 7.56-7.64 (m, 2H); 7.93 (br s, 1H).
mp: 228-230°C

EXAMPLE 99

6-Chloro-4-ethynyl-4-(3-furyl)-3-methyl-3,4-dihydroquinazolin-2(1H)-one

Step A: (2-amino-5-chlorophenyl)-3-furyl ketone

A solution of 3-bromofuran (.628 ml, 6.99 mmol) in 10 ml ethyl ether was cooled to -78°C under argon n-Butyl lithium (4.3 ml of 2.5M sol. in hexane) was added dropwise and solution was stirred for 1h. N-Methoxy-N-methyl 2-amino-5-chlorophenylamide (500mg, 2.33mmol, prepared according to J. Org. Chem., 56, pp 3750-2, 1991) was added to the anion in 15 ml ethyl ether and stirred at -20°C for 2h. Quench with water and extract with ethyl acetate. Wash organics with water and brine and dry over $MgSO_4$ . Rotovap off solvent to give a solid which was chromatographed on $SiO_2$ using ethyl acetate/hexanes to give 410 mg yellow solid.

Step B: 6-chloro-4-(3-furyl)-3-methylquinazolin-2-one

The solid from step A (150 mg, 0.729 mmol) was dissolved in 3 ml acetonitrile and 0.5 ml methyl isocyanate and refluxed with DABCO under Ar for 18 h. Replace solvent with toluene and reflux with a Dean-Stark trap for 24 h. Cool to room temperature and filter off yellow solid (82mg).

Step C: 6-Chloro-4-ethynyl-4-(3-furyl)-3-methyl-3,4-dihydroquinazolin-2(1H)-one

The solid from step B (82 mg, 0.315mmol) was slurried in 5 ml dry THF and sodium acetylide (350mg,

1.32mmol, 18% slurry in xylene) was added dropwise via syringe. The reaction was stirred at room temperature for 18 h. The mixture was poured into ethyl acetate and washed with water, brine dried over $MgSO_4$, filtered and concentrated in vacuo. The crude product was chromatographed on silica gel using 40% EtOAc in hexane as eluant to give 15 mg (17% yield) of the title compound as a colorless solid:
$^1H$ NMR (DMSO-$d_6$): δ 2.78 (s, 3H); 4.02 (s, 1H); 6.31 (s, 1H); 6.87-6.93 (m, 2H); 7.29 (dd, J=1.7,8.2, 1H); 7.72 (s, 1H); 7.86 (s, 1H); 10.00 (br s, 1H).
mp: 253-255°C

EXAMPLE 100

6-Chloro-4-cyclopropyl-4-phenyl-3-methyl-3,4-dihydroquinazolin-2(1H)-one

6-Chloro-3-methyl-4-phenyl-2(3H)-quinazolinone (740 mg, 2.7 mmol), suspended in 10 mL of anhydrous THF at -30°C, was treated with an excess of cyclopropylmagnesium bromide in a manner similar to Example 96, Step G. The crude reaction product was purified by flash chromatography on silica gel (60/40 EtOAc/hexane), to afford 210 mg of the title compound, mp 187-189°C.
$^1H$ NMR ($CDCl_3$): δ 0.12-0.32 (m, 2H); 0.52-0.63 (m, 1H); 0.70-0.84 (m, 1H); 1.65 (m, 1H); 2.78 (s, 3H); 6.26 (d, J = 2 Hz, 1H); 6.69 (d, J = 8.5 Hz, 1H); 7.07 (dd, J = 2, 8.5 Hz, 1H); 7.29-7.45 (m, 3H); 7.58 (d, J = 7 Hz, 2H); 8.52 (s, 1H).

EXAMPLE 101

9-chloro-10b-(1-propyl)-2,3,6,10b-tetrahydro(5H)-oxazolo(3,2c)quinazolin-5-one

Step A:

A solution of 2.0mmol of 2-amino-5-chlorobutyrophenone (396mg) and 1.62g (10.0mmol) of 1,1''-carbonyldiimidazole in 5mL of $CH_2Cl_2$ was stirred 6 hr. at room temperature under $N_2$. The mixture was diluted with 8mL of 10% aq. citric acid and stirred until precipitation of a white solid was complete. The solid was collected and washed with water by suction and recrystallized from 15mL of $CH_3CN$ to give 347mg of the N-imidazolyl carbonyl derivative of the starting ketone. mp:164°C.

Step B:

A solution of 292mg (1.00mmol of the imidazolyl intermediate) and ethanolamine (150mL) in 10mL of toluene was refluxed 2 hr. under $N_2$, cooled, washed with 5mL of 10% aq. citric acid and brine, dried by filtering through cotton, and concentrated under vacuum to give 270mg of white solid. Recrystallization from n-BuCl/hexane gave 220mg white crystalline title compound mp: 201-203°C);
$^1H$ NMR($CDCl_3$): δ 0.853 (t, J=7.2Hz, 3H, CH3), 1.16 (m, 1H, $CH_aH_b$), 1.30 (m, 1H, $CH_aH_b$), 1.74 (m, 2H, $CH_aH_b$), 3.61 (m, 1H, $CH_aN$), 3.85 (m, 1H, $CH_bN$), 4.16 (m, 2H, $CH_2O$), 6.78 (d, J=8.31, 1H, $H_8$), 7.21 (dd, J=2.26,8.31, 1H, H7), 7.33 (d,J=2.26, 1H, $H_5$), 8.15 (br s, 1H, NH).

EXAMPLE 102

(+/-) 9-chloro-10b-phenyl-1,2,3,10b-tetrahydropyrrolo[1,2-c]quinazolin-5(6H)-one

Step A: 6-chloro-3,4-dihydro-4-hydroxy-3-(4-methoxybenzyl)-4-phenyl-2(1H)-quinazolinone

In a manner similar to that described for Example 96, step E, 4-methoxybenzylamine (4.42g, 32.23 mmol) was reacted with 5-chloro-2-(1-imidazolecarbonylamino)-benzophenone (10.0g, 30.70 mmol) in 200 mL of dry THF to give 11.6g of 6-chloro-3,4-dihydro-4-hydroxy-3-(4-methoxybenzyl)-4-phenyl-2(1H)-quinazolinone as a white crystaline solid.

Step B: 6-chloro-3-(4-methoxybenzyl)-4-phenyl-2(3H)-quinazolinone

In a manner similar to that described for Example 96, step F, 6-chloro-3,4-dihydro-4-hydroxy-3-(4-methoxybenzyl)-4-phenyl-2(1H)-quinazolinone (11.5g, 29.12 mmol) was heated at reflux in toluene for 24h, with water removal. There was obtained 10.21g of 6-chloro-3-(4-methoxybenzyl)-4-phenyl-2(3H)-quinazolinone as a bright yellow solid.

Step C: 6-chloro-3,4-dihydro-4-(2-propenyl)-3-(4-methoxybenzyl)-4-phenyl-2(1H)-quinazolinone

In a manner similar to that described for Example 96, step G, 6-chloro-3-(4-methoxybenzyl)-4-phenyl-2(3H)-quinazolinone (8.00g, 21.23 mmol) was reacted in dry THF with allyl magnesium chloride (12.5 mL, of a 2.0 M solution in ethyl ether) to give after chromatography, 6.0g of 6-chloro-3,4-dihydro-4(2-propenyl)-3-(4-methoxybenzyl)-4-phenyl-2(1H)-quinazolinone as a white, crystalline solid. mp: 185-187°C.

Step D: 6-chloro-3,4-dihydro-4-(2-propenyl)-4-phenyl-2(1H)-quinazolinone

To a 50 mL round bottomed flask with a stirring bar and an argon inlet was added 6-chloro-3, 4-dihydro-4-(2-propenyl)-3-(4-methoxybenzyl)-4-phenyl-2(1H)-quinazolinone (5g, 11.94 mmol), $CH_2Cl_2$ (25 mL) and TFA (25 mL). This solution was stirred 24h at ambient temperature. The solvents were removed in vacuo and the residue was chromatographed on 200g of silica gel using 1:1 EtOAc-hexanes as eluant. There was obtained 3.62g of 6-chloro-3,4-dihydro-4-(2-propenyl)-4-phenyl-2(1H)-quinazolinone as a crystalline solid. An analytical sample was prepared by trituration with $CH_2Cl_2$-hexane solution. mp: 110-113°C.

Step E: 6-chloro-3,4-dihydro-4-(3-hydroxypropyl)-4-phenyl-2(1H)-quinazolinone

To an oven dried 3 necked 100 mL flask with a stirring bar, argon inlet, and a septum was added 6-chloro-3,4-dihydro-4-(2-propenyl)-4-phenyl-2(1H)-quinazolinone (500 mg, 1.67 mmol) and dry THF (20 mL). To this well stirred solution was added a solution of 9-borabicyclo[3.3.1]nonane (11.72 mL of a 0.5M solution in THF) with a syringe. This mixture was stirred at ambient temperature for 17h. The reaction was quenched with 5 mL of 10% aqueous NaOH solution and 5 mL of 30% $H_2O_2$ solution. The resulting mixture was stirred at ambient temperature for 4h. The mixture was diluted with EtOAc and the layers were separated. The organic phase was washed withwater and brine. Drying ($MgSO_4$), filtration, and removal of the solvent in vacuo gave the crude product as a colorless foam. This material was chromatographed on 80g of silica gel using 2.5% methanol in EtOAc as eluant to give 400mg of 6-chloro-3,4-dihydro-4-(3-hydroxypropyl)-4-phenyl-2(1H)quinazolinone as a colorless foam.

Step F: 6-chloro-3,4-dihydro-4-(3-(4-toluenesulfonyloxy)propyl)-4-phenyl-2(1H)-quinazolinone

To a 100 mL round bottomed flask with a stirring bar and an argon inlet was added 6-chloro-3, 4-dihydro-4-(3-hydroxypropyl)-4-phenyl-2(1H)-quinazolinone (400 mg, 1.26 mmol), dry $CH_2Cl_2$ (10 mL), pyridine (0.40 mL, 5.00 mmol), and p-toluenesulfonyl chloride (265 mg, 1.39 mmol). This mixture was stirred at ambient temperature for 18h than an addition 50 mg of p-toluenesulfonyl chloride was added. This solution was stirred an additional 6h. This solution was diluted with chloroform and washed with 10% aqueous citric acid, water, and brine. Drying ($MgSO_4$), filtration, and removal of the solvent in vacuo gave 450mg of crude product. This material was chromatographed on 80g of silica gel using 4:1 EtOAc-hexane as eluant. There was obtained 362 mg of pure 6-chloro-3,4-dihydro-4-(3-(4-toluenesulfonyloxy)propyl)-4-phenyl-2(1H)-quinazolinone as a clorless foam.

Step G: (+/-) 9-chloro-10b-phenyl-1,2,3,10b-tetrahydropyrrolo[1,2-c]quinazolin-5(6H)-one

To a 25 mL round bottomed flask with a stirring bar, and an argon inlet was added 6-chloro-3, 4-dihydro-4-(3-(4-toluenesulfonyloxy)propyl)-4-phenyl-2(1H)-quinazolinone (100 mg, 0.212 mmol), finely powdered potassium carbonate (250 mg, 1.809 mmol), and dry DMF. This well stirred mixture was heated at 100°C for 2h. The cooled reaction mixture was diluted with EtOAc (100 mL) and this solution was washed with water (3X 30 mL), and brine (50 mL). Drying ($MgSO_4$), filtration, removal of the solvent in vacuo, and recrystallization from EtOAc-hexanes gave 50 mg of (+/-) 9-chloro-10b-phenyl-1,2,3,10b-tetrahydropyrrolo[1,2-c]quinazolin-5(6H)-one as white crystals. mp: 231-233°C.

$^1$H NMR ($CDCl_3$): δ 1.60-1.83 (m, 1 H), 1.97-2.10 (m, 1 H), 2.45 (m, 1 H), 2.79 (dd, J=5.8, 10.8 Hz, 1 H), 3.75 (m, 2 H), 6.67 (d, J=8.5 Hz, 1 H), 7.20-7.40 (m, 7 H), 7.77 (br s, 1 H).

EXAMPLE 103

6-Chloro-3,4-dicyclopropyl-3,4-dihydroquinazolin-2(1H)-one

Step A: N-[(2-(Cyclopropylcarbonyl)-4-chlorophenyl]-N′-cyclopropylurea

A mixture containing 800 mg (2.76 mmol) of N-[(2-cyclopropylcarbonyl)-4-chlorophenyl]-N′-(1-imidazolyl-)urea and 0.57 mL (8.3 mmol) of cyclopropylamine in 5 mL each of acetonitrile and THF was stirred at 40°C for 4 hours. The mixture was then concentrated under vacuum and the solid which remained was triturated in 1-2 mL of acetonitrile to afford the title compound as a white solid, mp 142.5-143°C;
$^1H$ NMR ($CDCl_3$) δ 0.67 (m, 2H); 0.97 (m, 2H); 1.12-1.18 (m, 2H); 1.27 (m, 2H); 2.66 (m, 2H); 4.99 (s, 1H); 7.50 (dd, J = 2.5, 9 Hz, 1H); 8.06 (d, J = 2.5 Hz, 1H); 8.64 (d, J = 9 Hz, 1H).

Step B: 6-chloro-3,4-dicyclopropyl-3,4-dihydroquinazolin-2(1H)-one

140 mg (0.50 mmol) of N-[(2-(Cyclopropylcarbonyl)-4-chlorophenyl]-N′-cyclopropylurea was dissolved in 0.5mL glacial HOAc and the stirred yellow solution treated with 62mg (1.0mmol) of powdered sodium cyanoborohydride in portions during 10 min. at room temperature. The mixture was stirred one hour, diluted with ice water, and filtered to give 108 mg of the title compound. Recrystallization from n-butyl chloride hexane gave a white solid mp 197-198°C.
$^1H$ NMR ($CDCl_3$): δ 0.305 (m, 1H; $CH_AH$), 0.450 (m, 2H; $CH_2$), 0.625 (m, 1H; $CH_2$), 0.750 (m, 2H; $CH_2$), 0.822 (m, 1H; $CH_2$), 1.12 (m, 1H; $CH_2$), 1.26 (m, 1H, CH), 2.93 (m, 1H; CH N), 3.641 (1H, d, J = 8.79 Hz; $H_4$), 6.688 (1H, d, J = 8.36; $H_5$), 7.01 (1H, d, J = 2.2 Hz; $H_5$), 7.162 (1H, dd, J = 8.38 Hz, 2.34 Hz; $H_7$), 7.28 (1H, S(b), NH).

REVERSE TRANSCRIPTASE ASSAY

The assay measures the incorporation of tritiated deoxyguanosine monophosphate by recombinant HIV reverse transcriptase (HIV $RT_R$) (or other RT) into acid-precipitable cDNA at the Km values of dGTP and poly r(C)·oligo $d(G)_{12-18}$. The inhibitors of the present invention inhibit this incorporation.

Thirty μL of a reaction mixture containing equal volumes of: 500 mM Tris·HCl (pH 8.2), 300 mM $MgCl_2$, 1200 mM KCl, 10 mM DTT, 400 μg/mL poly r(c)·oligo d(G) [prepared by dissolving 1.5 mg (25 U) poly r(C)·oligo d(G) in 1.5 ml sterile distilled $H_2O$ and diluting to 400 μg/ml], 0.1 μCi/μl [$^3H$] dGTP, 160 μM dGTP, was added to 10 μl sterile distilled $H_2O$, 2.5 μl of potential inhibitor and 10 μL of 5 nM purified HIV $RT_R$ in tubes. The mixture was incubated at 37°C for 45 minutes.

After incubation is complete, the tubes were cooled in ice for 5 minutes. Ice-cold 13% TCA containing 10 mM $NaPP_i$ (200 μl) is added and the mixture incubated on ice for 30 minutes. The precipitated cDNA is removed by filtration using presoaked glass filters [TCA, $NaPP_i$]. The precipitate is then washed with 1N HCl, 10 mM $NaPP_i$.

The filter discs are then counted in a scintillation counter.

Under these conditions [dGTP] and poly r(C)·oligo $d(G)_{12-18}$ each are approximately equal to the appropriate Km value. Approximately 5-6,000 cpm of [$^3H$] GMP are incorporated into acid-precipitable material. The RT reaction is concentration- and time-dependent. DMSO (up to 5%) does not affect enzyme activity. Calculated $IC_{50}$ values for the tested compounds of this invention vary from about 12 nM to more than 300 μM, with the most preferred compounds having values of between about 30 nM and about 60 μM. The (+) optical isomer of compound 4 has a $IC_{50}$ value of about 0.06 nM.

CELL SPREAD ASSAY

Inhibition of the spread of HIV in cell culture was measured according to Nunberg, J. H. et al., J. Virol. 65, 4887 (1991). In this assay, MT-4 T-lymphoid cells were infected with HIV-1 by using a predetermined inoculum, and cultures were incubated for 24h. At this time, ≦1% of the cells were positive by indirect immunofluorescence. Cells were then extensively washed and distributed into 96-well culture dishes. Serial twofold dilutions of inhibitor were added to the wells, and cultures were continued for 3 additional days. At 4 days postinfection, 100% of the cells in control cultures were infected. HIV-1 p24 accumulation was directly correlated with virus spread. The cell culture inhibitory concentration was defined as the inhibitor concentration which reduced the spread of infection by at least 95%, or $CIC_{95}$. Because all virus populations appeared monodisperse and displayed parallel dose-response curves, resistance was quantified as the ratio of inhibitor concentrations that

reduced the spread of infection by at least 95%.

While the foregoing specification teaches the principles of the present invention, with examples provided for the purpose of illustration, it will be understood that the practice of the invention encompasses all of the usual variations, adaptations, and modifications, as come within the scope of the following claims and its equivalents.

## Claims

1. Compounds of the formulae:

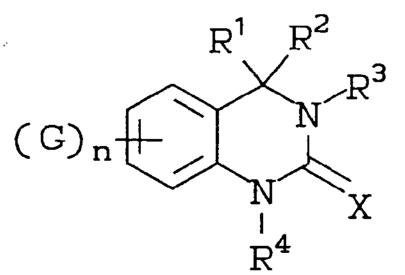

I

or

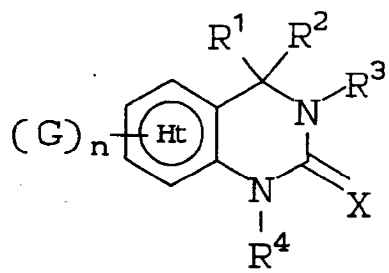

II

wherein:

X is O, S, or NR, and R is CN, H, $NH_2$, OH, $C_{1-3}$alkoxy, or $C_{1-3}$ alkyl;

G, when present, is 1-4 substituents independently selected from $C_{1-4}$ alkyl; halo; amino; nitro; cyano; carboxy; carbamoyl C1-4 alkyl;

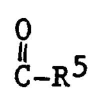

wherein $R^5$ is $CH_3$, H or $NH_2$; sulfamoyl; $C_{1-3}$ alkylsulfonamido; methanesulfonyl; $CF_3$; hydroxy; or $C_{1-4}$alkoxy;

n is 0-4;

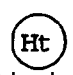 in formula II denotes heterocycle of 5-6 members (with 1-3 heteroatoms selected from O, N or S) in place of benzo in formula I;

$R^1$ is H; $C_{1-8}$ alkyl; $C_{2-4}$ alkenyl; $C_{2-5}$ alkynyl; $C_{3-4}$ cycloalkyl; hydroxy; $C_{1-4}$ alkoxy; $C_{1-3}$ alkyl substituted one or more times with halo, carboxy, $C_{3-4}$ cycloalkyl, CN, hydroxy, methylsulfinyl, methanesulfonyl, $C_{1-4}$ alkoxy, $C_{2-4}$ alkenyl- $C_{1-4}$ alkoxy, $C_{2-4}$ alkynyl-$C_{1-4}$ alkoxy, aryloxy, $C_{1-4}$ alkylcarbonyl, or nitro; or aryl unsubstituted or substituted with $(G)_n$; furanyl; thienyl; benzofuranyl; $R^1$ and $R^3$ may be joined to form a fused heterocycle of 5 or 6 members containing up to one additional heteroatom selected from 0, S or NR, provided that the heteroatoms are not directly linked to each other;

$R^2$ is $C_{1-3}$ alkyl; $C_{2-3}$ alkenyl; $C_{3-4}$ cycloalkyl; $C_{2-5}$ alkynyl; aryl unsubstituted or substituted at the meta position with halo, methoxy or cyano; heterocycle;

$R^3$ is H; $C_{1-3}$ alkyl unsubstituted or substituted with one or more of A, wherein A is halo, hydroxy, amino, $C_{3-8}$ cycloalkyl, $C_{1-4}$ alkoxy, di-($C_{1-4}$ alkyl)amino, $C_{1-4}$ alkylamino, or carbamoyl; $C_{2-8}$ alkenyl un-

substituted or substituted with one or more of A; $C_{2-8}$ alkynyl unsubstituted or substituted with one or more of A; $C_{3-8}$ cycloalkyl; aryl $C_{1-4}$ alkyl wherein aryl is optionally substituted with up to three substituents selected from methyl, halo, CN, or methoxy; heterocycle of 5 or 6 members containing up to 3 heteroatoms selected from O, S or NR; hydroxyl; CN; or carbamoyl;

$R^4$ is H; $C_{1-8}$ alkyl unsubstituted or substituted with one or more of A; $C_{2-8}$ alkenyl unsubstituted or substituted with one or more of A; $C_{2-8}$ alkynyl unsubstituted or substituted with one or more of A; $C_{1-3}$ alkanoyl; benzoyl unsubstituted or substituted with one to three of halo, methoxy, carboxy, or $C_{1-4}$ alkyl optionally substituted with carboxy, amino, $C_{1-4}$ alkylamino or di-$C_{1-4}$ alkylamino; carbamoyl, methylcarbamoyl, dimethylcarbamoyl, formyl, hydroxy, alpha-amino $C_{1-4}$ alkanoyl, or meta-cyanobenzyl;
or pharmaceutically acceptable salt or ester thereof

**2.** A compound of Claim 1, wherein X is neither 0 nor S.

**3.** A compound according to Claim 1, of the formula:

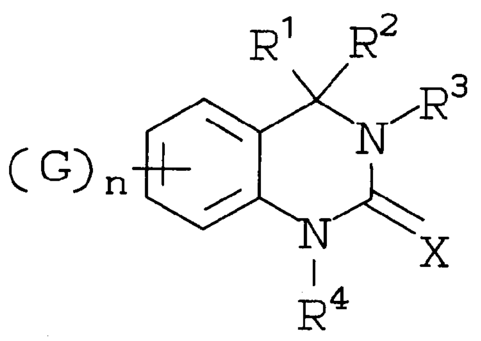

III

wherein:

X is O, S, or NR, and R is CN, $NH_2$, OH, $C_{1-3}$ alkoxy, H or $C_{1-3}$ alkyl;

G, when present, is 1-4 substituents independently selected from $C_{1-4}$ alkyl; halo; amino; nitro; cyano; carboxy; carbamoyl $C_{1-4}$ alkyl;

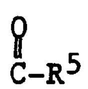

wherein $R^5$ is $CH_3$, H or $NH_2$; sulfamoyl; $C_{1-3}$ alkylsulfonamido; methanesulfonyl; $CF_3$; hydroxy; or $C_{1-4}$alkoxy;

n is 0-4;

$R^1$ is $C_{1-8}$ alkyl; $C_{2-4}$ alkenyl; $C_{2-5}$ alkynyl; $C_{3-4}$ cycloalkyl; $C_{1-3}$ alkyl substituted one or more times with halo, carboxy,

$C_{3-4}$cycloalkyl, CN, hydroxy, methylsulfinyl, methanesulfonyl, $C_{1-4}$alkoxy, $C_{2-4}$alkynyl-$C_{1-4}$alkoxy, or nitro; or aryl unsubstituted or substituted with $(G)_n$; $R^1$ and $R^3$ may be joined by a carba bridge to form a ring of 5 to 6 members;

$R^2$ is $C_{1-8}$ alkyl; $C_{2-8}$ alkenyl; $C_{3-4}$ cycloalkyl; $C_{2-5}$ alkynyl; aryl unsubstituted or substituted at the meta position with halo, methoxy or cyano; heterocycle of 5 or 6 members containing up to 3 heteratoms selected from O, S, or NR;

$R^3$ is H; $C_{1-8}$ alkyl unsubstituted or substituted with one or more of A, wherein A is halo, hydroxy, amino, $C_{3-8}$ cycloalkyl, $C_{1-3}$ alkoxy, di-($C_{1-4}$ alkyl)amino, $C_{1-4}$ alkylamino, or carbamoyl; $C_{2-8}$ alkenyl unsubstituted or substituted with one or more of A; $C_{2-8}$ alkynyl unsubstituted or substituted with one or more of A; $C_{3-8}$ cycloalkyl; aryl $C_{1-4}$ alkyl wherein aryl is optionally substituted with up to three substituents selected from methyl, halo, CN, or methoxy;

$R^4$ is H; $C_{1-8}$ alkyl unsubstituted or substituted with one or more of A; $C_{2-8}$ alkenyl unsubstituted or substituted with one or more of A; $C_{2-8}$ alkynyl unsubstituted or substituted with one or more of A; $C_{1-3}$ alkanoyl; benzoyl unsubstituted or substituted with one to three of halo, methoxy, carboxy, or $C_{1-4}$ alkyl optionally substituted with carboxy, amino, $C_{1-4}$ alkylamino or di-$C_{1-4}$ alkylamino, carbamoyl, methylcarbamoyl, dimethylcarbamoyl, formyl, hydroxy, alpha-amino $C_{1-4}$ alkanoyl, or meta-cyanobenzyl;

with the proviso that when $R^1$ is $C_{1-2}$ alkyl, then $R^2$ is not $C_{1-2}$ alkyl; or pharmaceutically acceptable salt or ester thereof.

4. A compound according to Claim 1, of the formula:

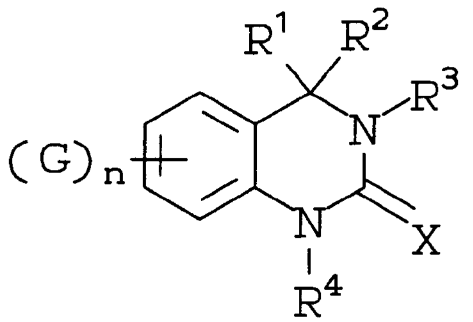

IV

wherein:

X is O, S, or NR, and R is CN, $NH_2$, OH, $C_{1-3}$alkoxy, H or $C_{1-3}$ alkyl;

G, when present, is 1-4 substituents independently selected from $C_{1-4}$ alkyl; halo; amino; nitro; cyano; carboxy; carbamoyl $C_{1-4}$ alkyl;

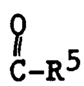

wherein $R^5$ is $CH_3$, H or $NH_2$; sulfamoyl; $C_{1-3}$ alkylsulfonamido; methanesulfonyl; $CF_3$; hydroxy; or $C_{1-4}$ alkoxy;

n is 0-4;

$R^1$ is $C_{1-8}$ alkyl; $C_{2-4}$ alkenyl; $C_{2-5}$ alkynyl; $C_{3-4}$ cycloalkyl, $C_{1-3}$ alkyl substituted one or more times with halo, carboxy, $C_{3-4}$cycloalkyl, CN, hydroxy, methylsulfinyl, methanesulfonyl, $C_{1-4}$alkoxy, $C_{2-4}$alkynyl-$C_{1-4}$alkoxy, or nitro; or aryl unsubstituted or substituted with $(G)_n$; $R^1$ and $R^3$ may be joined by a carba bridge to form a ring of 5 to 6 members;

$R^2$ is $C_{1-3}$ alkyl, $C_{2-3}$ alkenyl, $C_{3-4}$ cycloalkyl; $C_{2-5}$ alkynyl; aryl unsubstituted or substituted at the meta position with halo, methoxy or cyano;

$R^3$ is H; $C_{1-3}$ alkyl unsubstituted or substituted with one or more of A, wherein A is halo, hydroxy, amino, $C_{3-8}$ cycloalkyl, $C_{1-3}$ alkoxy, di-($C_{1-4}$ alkyl)amino, $C_{1-4}$ alkylamino, or carbamoyl; $C_{2-8}$ alkenyl unsubstituted or substituted with one or more of A; $C_{2-8}$ alkynyl unsubstituted or substituted with one or more of A; $C_{3-8}$ cycloalkyl; aryl $C_{1-4}$ alkyl wherein aryl is optionally substituted with up to three substituents selected from methyl, halo, CN, or methoxy;

$R^4$ is H; $C_{1-3}$ alkyl unsubstituted or substituted with one or more of A; $C_{2-8}$ alkenyl unsubstituted or substituted with one or more of A; $C_{2-3}$ alkynyl unsubstituted or substituted with one or more of A; $C_{1-3}$ alkanoyl; benzoyl unsubstituted or substituted with one to three of halo, methoxy, carboxy, or $C_{1-4}$ alkyl optionally substituted with carboxy, amino, $C_{1-4}$ alkylamino or di-$C_{1-4}$ alkylamino; carbamoyl; methylcarbamoyl; dimethylcarbamoyl; formyl; hydroxy; alpha-amino $C_{1-4}$ alkanoyl; or meta-cyanobenzyl;

with the proviso that when $R^1$ is $C_{1-2}$ alkyl, then $R^2$ is not $C_{1-2}$ alkyl.

or pharmaceutically acceptable salt of ester thereof.

5. A compound according to Claim 4, of the formula:

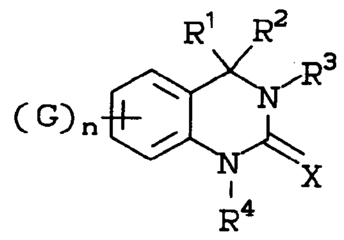

V

wherein

X is O;

G, when present, is $C_{1-4}$ alkyl; halo; amino; nitro; cyano; carboxy; carbamoyl $C_{1-4}$ alkyl; or $C_{1-3}$ alkylsulfonamido;

n is 0-4;

$R^1$ is $C_{3-4}$alkyl, $C_{3-4}$alkenyl, $C_{2-5}$alkynyl; $C_{3-4}$cycloalkyl; $C_{1-3}$alkyl substituted with $C_{3-4}$cycloalkyl, $C_{1-4}$alkoxy, or $C_{2-4}$alkynyl-$C_{1-4}$alkoxy;

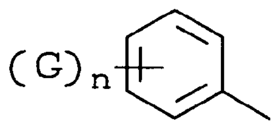 or 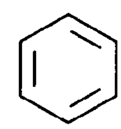 ;

$R^1$ and $R^3$ may be joined by a carba bridge to form a ring of 5 to 6 members;

$R^2$ is $C_{1-3}$ alkyl, $C_{2-3}$ alkenyl, $C_{2-3}$ alkynyl, $C_{3-4}$ cycloalkyl, or aryl;

$R^3$ is H; $C_{1-4}$ alkyl unsubstituted or substituted with $C_{3-8}$ cycloalkyl, $C_{2-4}$ alkenyl, or $C_{2-4}$ alkynyl; $C_{3-4}$ cycloalkyl,

$R^4$ is H; $C_{1-3}$ alkyl unsubstituted or substituted with one or more of A; $C_{2-8}$ alkenyl unsubstituted or substituted with one or more of A; $C_{2-3}$ alkynyl unsubstituted or substituted with one or more of A; $C_{1-3}$ alkanoyl; benzoyl unsubstituted or substituted with one to three of halo, methoxy, carboxy, or $C_{1-4}$ alkyl optionally substituted with carboxy, amino, $C_{1-4}$ alkylamino or di-$C_{1-4}$ alkylamino, carbamoyl, methylcarbamoyl, dimethylcarbamoyl, formyl, hydroxy, alpha-amino $C_{1-4}$ alkanoyl, or meta-cyanobenzyl;

with the proviso that $R^1$ and $R^2$ are not both straight-chain alkyl groups;

or pharmaceutically acceptable salts or esters thereof.

6. A compound according to Claim 5, which is 6-Chloro-3,4-dihydro-3-methyl-4-phenyl-4-ethynyl-2(1H)-quinazolinone,
6-Chloro-3,4-dihydro-3-ethyl-4-phenyl-4-ethynyl-2(1H)-quinazolinone,
6-Chloro-3,4-dihydro-3-cyclopropyl-4-phenyl-4-ethynyl-2(1H)-quinazolinone,
6-Chloro-3,4-dihydro-3-cyclopropyl-methyl-4-cyclopropyl-4-propyl-2(1H)-quinazolinone
6-Chloro-3,4-dihydro-3-methyl-4-cyclopropyl-4-propyl-2(1H)-quinazolinone,
6-Chloro-3,4-dihydro-3-ethyl-4-cyclopropyl-4-(2-ethoxymethyl)-2(1H)-quinazolinone,
6-Chloro-3,4-dihydro-3-methyl-4-cyclopropyl-4-ethynyl-2(1H)-quinazolinone,
6-Chloro-3,4-dihydro-3-methyl-4-phenyl-4-ethynyl-2(1H)-cyanoiminoquinazoline,
6-Chloro-3,4-dihydro-3-(2-propynyl)-4-cyclopropyl-4-(2-propynyloxymethyl)-2(1H)-quinazolinone,
6-fluoro-3,4-dihydro-3-methyl-4-phenyl-4-ethynyl-2(1H)-quinazolinone,
6-Chloro-3,4-dihydro-3-methyl-4-cyclopropyl-4-cyclopropylmethyl-2(1H)-quinazolinone,
6-Chloro-3,4-dihydro-4-cyclopropyl-4-ethynyl-2(1H)-quinazolinone, or
6-Chloro-3,4-dihydro-4-cyclopropyl-4-(n-butyl)-2(1H)-quinazolinone.

7. The compound (+)-6-chloro-3,4-dihydro-3-methyl-4-phenyl-4-ethynyl-2(1H)-quinazolinone.

8. The use of a compound as claimed in any of Claims 1-7 for the manufacture of a medicament for inhibiting HIV reverse transcriptase.

9. The use of a compound as claimed in any of Claims 1-7 for the manufacture of a medicament for preventing infection by HIV, or of treating infection by HIV or of treating AIDS or ARC.

**10.** A pharmaceutical composition comprising a compound as claimed in any of Claims 1-7, and a pharmaceutically acceptable carrier.

European Patent Office

# EUROPEAN SEARCH REPORT

Application Number

EP 92 30 7398

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 220 786 (ORTHO)<br>* Whole document *<br>--- | 1,3-5,8<br>-11 | C 07 D 239/80<br>C 07 D 239/82<br>C 07 D 487/04<br>C 07 D 498/04<br>C 07 D 405/04<br>A 61 K 31/505 |
| X | EP-A-0 107 398 (ORTHO)<br>* Example 7; claims *<br>--- | 1,3-5,8<br>-11 | |
| X | FR-A-2 328 700 (SUMITOMO)<br>* Pages 1-7 *<br>--- | 1,3-5,8<br>-11 | |
| X | SOVIET PATENTS ABSTRACTS, week 9105, 20th March 1991, class B02, AN=91-035113/05, Derwent Publications Ltd, London, GB; & SU-A-470 423 (TOMSK POLY) 30-05-1990<br>* Abstract *<br>--- | 1,8-11 | |
| X | PATENT ABSTRACTS OF JAPAN, vol. 6, no. 180 (C-125)(1058), 14th September 1982; & JP-A-57 95 966 (SUMITOMO) 15-06-1982<br>* Abstract *<br>--- | 1,3-5,8<br>-11 | |
| | | | **TECHNICAL FIELDS SEARCHED (Int. Cl.5)** |
| X | FR-A-2 118 932 (SUMITOMO)<br>* Whole document *<br>--- | 1,3-5,8<br>-11 | C 07 D 239/00<br>C 07 D 405/00<br>C 07 D 498/00<br>C 07 D 487/00 |
| X | FR-A-2 103 544 (SUMITOMO)<br>* Whole document *<br>--- | 1,8-11 | |
| X | FR-A-2 012 062 (SANDOZ)<br>* Claims *<br>--- -/- | 1,3-5,8<br>-11 | |

The present search report has been drawn up for all claims

| Place of search | Date of completion of the search | Examiner |
|---|---|---|
| THE HAGUE | 10-11-1992 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

Page 2

European Patent Office

# EUROPEAN SEARCH REPORT

Application Number

EP 92 30 7398

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
| --- | --- | --- | --- |
| X | FR-A-2 027 023 (SOCIETA FARMACEUTICI ITALIA)<br>* Pages 1-7 *<br>--- | 1,3-5,8-11 | |
| X | US-A-3 764 600 (H. OTT)<br>* Columns 14-22 *<br>--- | 1,3-5,8-11 | |
| A | WO-A-9 109 024 (KYORIN)<br>* Page 0, claims *<br>----- | 1,8-11 | |
| | | | TECHNICAL FIELDS SEARCHED (Int. Cl.5) |

The present search report has been drawn up for all claims

| Place of search | Date of completion of the search | Examiner |
| --- | --- | --- |
| THE HAGUE | 10-11-1992 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)